(19) Europäisches Patentamt
European Patent Office
Office européen des brevets

(11) Veröffentlichungsnummer: **0 391 179**
**A2**

(12) # EUROPÄISCHE PATENTANMELDUNG

(21) Anmeldenummer: **90105613.5**

(22) Anmeldetag: **24.03.90**

(51) Int. Cl.[5]: **C07K 5/02, C07K 5/06, C07D 233/64, A61K 37/64**

Ein Antrag gemäss Regel 88 EPÜ auf Berichtigung der Beschreibung (Seite 51) liegt vor. Über diesen Antrag wird im Laufe des Verfahrens vor der Prüfungsabteilung eine Entscheidung getroffen werden (Richtlinien für die Prüfung im EPA, A-V, 2.2).

Die Bezeichnung der Erfindung wurde geändert (Richtlinien für die Prüfung im EPA, A-III, 7.3).

(30) Priorität: **05.04.89 CH 1290/89**

(43) Veröffentlichungstag der Anmeldung:
**10.10.90 Patentblatt 90/41**

(84) Benannte Vertragsstaaten:
**AT BE CH DE DK ES FR GB GR IT LI LU NL SE**

(71) Anmelder: **F. HOFFMANN-LA ROCHE AG**
**Postfach 3255**
**CH-4002 Basel(CH)**

(72) Erfinder: **Branca, Quirico, Dr.**
**Lenzgasse 2**
**CH-4056 Basel(CH)**
Erfinder: **Märki, Hans Peter, Dr.**
**Seltisbergerstrasse 75**
**CH-4059 Basel(CH)**
Erfinder: **Neidhart, Werner, Dr.**
**Oltmannstrasse 14**
**D-7800 Freiburg im Breisgau(DE)**
Erfinder: **Ramuz, Henri, Prof. Dr.**
**Rheinparkstrasse 3**
**CH-4127 Birsfelden(CH)**
Erfinder: **Wostl, Wolfgang, Dr.**
**Im Strick 2**
**D-7889 Grenzach-Wyhlen(DE)**

(74) Vertreter: **Kellenberger, Marcus, Dr. et al**
**Grenzacherstrasse 124 Postfach 3255**
**CH-4002 Basel(CH)**

(54) **Peptidartige Aminosäurederivate mit reninhemmender Wirkung.**

(57) Die Verbindungen der Formel

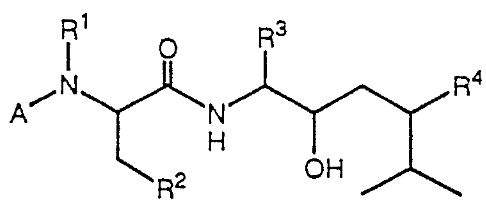

I

worin A, $R^1$, $R^2$, $R^3$ und $R^4$ die in Anspruch 1 angegebene Bedeutung besitzen, in Form von optisch reinen Diastereomeren, Diastereomerengemischen, diastereomeren Racematen oder Gemischen von diastereomeren Racematen sowie pharmazeutisch verwendbare Salze davon hemmen die Wirkung des natürlichen Enzyms Renin und können demnach in Form pharmazeutischer Präparate bei der Bekämpfung bzw. Verhütung von Bluthochdruck und Herzinsuffizienz verwendet werden. Sie können nach verschiedenen, an sich bekannten Verfahren hergestellt werden.

EP 0 391 179 A2

## Peptidartige Aminosäurederivate

Die vorliegende Erfindung betrifft Aminosäurederivate. Im speziellen betrifft sie Aminosäurederivate der allgemeinen Formel

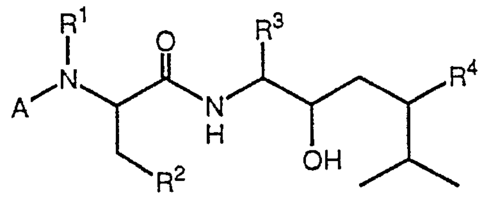

I

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, A eine der Gruppen

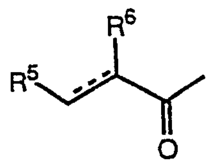

(a)

und -Y-Z (b)
und $R^4$ Alkanoyl, Arylcarbonyl, 2,2-Dialkylvinyl oder eine der Gruppen

$-CH=CH-R^7$

(c)

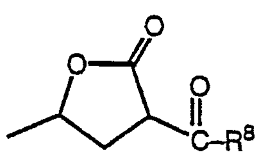

(d)

und

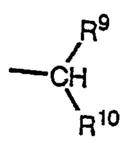

(e)

bedeuten, worin die gestrichelte Linie eine zusätzliche Bindung bedeuten kann, $R^5$ Phenyl, substituiertes Phenyl, Benzyl oder Naphthyl und $R^6$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Aminoalkylcarbonylalkyl, substituiertes Aminoalkylcarbonylalkyl, Aminoalkylsulfonylalkyl, substituiertes Aminoalkylsulfonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl, Alkylsulfonylalkyl, Arylthioalkyl, Arylsulfinylalkyl, Arylsulfonylalkyl, Arylalkylthioalkyl, Arylalkylsulfinylalkyl oder Arylalkylsulfonylalkyl bedeuten, mit der Massgabe, dass $R^6$ nicht Alkoxycarbonylamino oder Arylalkoxycarbonylamino bedeuten kann, wenn $R^5$ Phenyl, Benzyl oder α-Naphthyl bedeutet, Y den N-terminal mit Z verbundenen, bivalenten Rest von gegebenenfalls N- und/oder α-methyliertem Phenylglycin, Cyclohexylglycin, Phenylalanin, Cyclohexylalanin, 4-Fluorphenylalanin, 4-Chlorphenylalanin, Tyrosin, O-Methyltyrosin, α-Naphthylalanin oder Homophenylalanin, Z Acyl, $R^7$ Alkyl, Aryl, Heteroaryl, Alkylaminocarbonyl, Arylalkylaminocarbonyl oder Heteroarylalkylaminocarbonyl, $R^8$ Alkoxy, $R^9$ Wassersstoff oder Hydroxy und $R^{10}$ Azidomethyl, die Gruppe (c) oder eine der Gruppen

$-CH_2-OR^{11}$ (f)

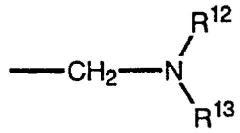 (g)

und

$-(CH_2)_n-R^7$ (h)

bedeuten, worin $R^{11}$ Wasserstoff, Alkyl, Arylalkyl, Aryl, Alkanoyl, Arylalkylcarbonyl, Heteroarylalkylcarbonyl oder Alkylcarbamoyl, $R^{12}$ und $R^{13}$ unabhängig voneinander je Wasserstoff, Alkyl, Arylalkyl, Heteroarylalkyl, Aryl, Alkanoyl, Alkoxycarbonyl, Arylalkylcarbonyl, Arylalkoxycarbonyl, Heteroarylalkylcarbonyl, Heterocycloalkylcarbonyl, Alkylcarbamoyl oder die Gruppe

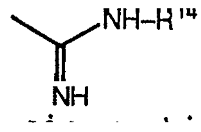

oder $R^{12}$ und $R^{13}$ zusammen mit dem sie verbindenden Stickstoffatom einen 5- oder 6-gliedrigen Heterocyclus oder gegebenenfalls substituiertes Benzimidazolonyl, n 0, 1 oder 2 und $R^{14}$ Wasserstoff oder Arylalkoxycarbonyl bedeuten,

in Form von optisch reinen Diastereomeren, Diastereomerengemischen, diastereomeren Racematen oder Gemischen von diastereomeren Racematen sowie pharmazeutisch verwendbare Salze dieser Verbindungen.

Diese Verbindungen sind neu und zeichnen sich durch wertvolle pharmakodynamische Eigenschaften aus.

Gegenstand der vorliegenden Erfindung sind die Verbindungen der Formel I und ihre pharmazeutisch verwendbaren Salze als solche und zur Anwendung als therapeutische Wirkstoffe, die Herstellung dieser Verbindungen, ferner diese enthaltende Arzneimittel und die Herstellung solcher Arzneimitteln, sowie die Verwendung von Verbindungen der Formel I und ihrer pharmazeutisch verwendbaren Salze bei der Bekämpfung bzw. Verhütung von Krankheiten, bzw. bei der Verbesserung der Gesundheit, insbesondere bei der Bekämpfung bzw. Verhütung von Bluthochdruck und Herzinsuffizienz.

Die nachstehenden Definitionen der in der vorliegenden Beschreibung verwendeten allgemeinen Ausdrücke besitzen ihre Gültigkeit unabhängig davon, ob die fraglichen Ausdrücke allein oder in Kombination aufscheinen.

Der in der vorliegenden Beschreibung verwendete Ausdruck "Alkyl" bedeutet geradkettige und verzweigte, gesättigte Kohlenwasserstoffreste mit 1-8, vorzugsweise 1-4, Kohlenstoffatomen, wie Methyl, Aethyl, n-Propyl, Isopropyl, n-Butyl, Isobutyl, sec.-Butyl, t-Butyl, Pentyl, Hexyl und dergleichen. Der Ausdruck "Alkoxy" bedeutet Alkyläthergruppen, worin der Ausdruck "Alkyl" die obige Bedeutung hat, wie Methoxy, Aethoxy, Propoxy, Isopropoxy, Butoxy, Isobutoxy, sec.-Butoxy, t-Butoxy und dergleichen. Der Ausdruck "Cycloalkyl" bedeutet gesättigte, cyclische Kohlenwasserstoffreste mit 3-8, vorzugsweise 3-6, Kohlenstoffatomen, wie Cyclopropyl, Cyclobutyl, Cyclopentyl, Cyclohexyl und dergleichen. Der Ausdruck "Heterocycloalkyl" betrifft in ähnlicher Weise gesättigte, 5-8-gliedrige, vorzugsweise 5- oder 6-gliedrige, cyclische Kohlenwasserstoffreste, in welchen eine oder zwei Methylengruppen durch ein oder zwei Sauerstoff- , Schwefel- oder gegebenenfalls durch Alkyl, Phenylalkyl, Alkanoyl oder Alkanoyloxy substituierte Stickstoffatome ersetzt sind, wie Piperidinyl, Pyrazinyl, N-Benzylpyrazinyl, Morpholinyl, N-Methylpiperidinyl, N-Benzylmorpholinyl und dergleichen. Der Ausdruck "Alkanoyl" bedeutet den Säurerest einer geradkettigen oder verzweigten Alkansäure mit 1-8, vorzugsweise 1-4, Kohlenstoffatomen, wie Formyl, Acetyl, Propionyl, Butyryl, Valeryl, Isovaleryl und dergleichen. Der Ausdruck "Aryl" bezeichnet einen gegebenenfalls durch Alkyl, Alkoxy, Alkanoyloxy, Amino, Alkylamino, Dialkylamino, Alkylcarbonylamino, Hydroxy, Halogen, Trifluormethyl oder Nitro ein- oder mehrfach substituierten mono- oder bicyclischen aromatischen Kohlenwasserstoffrest mit 6-14 Kohlenstoffatomen, wie Phenyl, α- oder β-Naphthyl, Indenyl, Anthryl oder Phenanthryl und dergleichen. Der Ausdruck "Arylalkyl" bezeichnet geradkettige oder verzweigte Alkylgruppen, worin ein oder mehrere Wasserstoffatome durch Arylgruppen ersetzt sind, wie Benzyl, Diphenylmethyl, Trityl, α- oder β-Naphthylmethyl, 2-Phenyläthyl, 3-Phenyl-2-propyl, 4-Phenyl-3-butyl, 2-(α- oder β-Naphthyl)äthyl, 3-α-Naphthyl-2-propyl, 4-α-Naphthyl-3-butyl und dergleichen, wobei der aromatische Rest jeweils wie oben angegeben ein- oder mehrfach substituiert sein kann. Der Ausdruck "substituiertes Phenyl" bezeichnet gegebenenfalls durch Alkyl, Alkoxy, Alkoxyalkoxy, Alkanoyl, Alkanoyloxy, Hydroxy, Halogen oder

Trifluormethyl ein- oder mehrfach substituiertes Phenyl, wie 4-Hydroxyphenyl, 4-Methoxyphenyl, 4-Methylphenyl, 4-Chlorphenyl, 4-Aethoxyäthoxyphenyl und dergleichen. Beispiele für gegebenenfalls substituiertes Benzimidazolonyl sind Benzimidazolonyl, 3-Methylbenzimidazolonyl, 3-Isopropylbenzimidazolonyl, 3-Butylbenzimidazolonyl, 3-Morpholinoäthylbenzimidazolonyl, 3-Benzylbenzimidazonyl und dergleichen. Der Ausdruck "5- oder 6-gliedriger Heterocyclus" betrifft gesättigte 5- oder 6-gliedrige Heterocyclen mit mindestens einem Stickstoffatom und gegebenenfalls einem zusätzlichen Sauerstoff-, Stickstoff- oder Schwefelatom als Ringglieder, die am zweiten Stickstoffatom durch Alkyl, Phenyl, Phenylalkyl oder Hydroxyalkyl oder an einem Kohlenstoffatom durch Alkylamin, Arylalkylamin, Alkyl, Phenyl oder Phenylalkyl substituiert sein kann, wie Piperidinyl, 4-Benzylaminopiperidinyl, Pyrazinyl, Piperazinyl, N-Hydroxyäthylpiperazinyl, Morpholinyl, Thiomorpholinyl, Pyrrolidinyl, Thiazolidinyl, Imidazolidinyl, Oxazolidinyl und dergleichen. Der Ausdruck "Heteroaryl" bezeichnet einen gegebenenfalls an einem Stickstoffatom durch Alkyl, Phenyl oder Phenylalkyl und/oder an einem oder mehreren Kohlenstoffatomen durch Alkyl, Phenyl, Phenylalkyl, Halogen, Hydroxy, Alkoxy, Phenylalkoxy oder Oxo substituierten und teilweise gesättigten mono- oder bicyclischen aromatischen Kohlenwasserstoffrest, in welchem ein oder mehrere Kohlenstoffatome durch ein bis zwei Stickstoffatome und/oder ein Sauerstoff- oder Schwefelatom ersetzt sind, wie Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl oder Chinoxalinyl, z.B. 2- oder 3-Pyrrolyl, Phenylpyrrolyl, z.B. 4- oder 5-Phenyl-2-pyrrolyl, 2-Furyl, 2-Thienyl, 2-Imidazolyl, 2-, 3- oder 4-Pyridyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2-indolyl, 1-Benzyl-3-indolyl, 4,5,6,7-Tetrahydro-2-indolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, 2-Benzimidazolyl und dergleichen.

Der Ausdruck "Halogen" betrifft die vier Halogene Fluor, Chlor, Brom und Jod.

Der Ausdruck "substituiertes Amino" bedeutet eine durch Alkyl, Arylalkyl, Alkanoyl, Alkoxycarbonyl oder Arylalkoxycarbonyl mono- oder di-substituierte oder eine gegebenenfalls durch ein Sauerstoff-, Schwefel- oder gegebenenfalls Alkyl-, Phenylalkyl-, Alkanoyl- oder Alkanoyloxy-substituiertes Stickstoffatom unterbrochene $C_3$-$C_6$-Alkylen disubstituierte Aminogruppe. Der Ausdruck "Acyl" betrifft die Acylgruppe einer Carbonsäure, eines Halbesters der Kohlensäure, einer gegebenenfalls N-substituierten Carbamin- oder Thiocarbaminsäure, eines gegebenenfalls N-substituierten Oxalamids, einer Sulfonsäure oder einer gegebenenfalls N-substituierten Amidosulfonsäure, insbesondere solche mit den Teilformeln $R^b$-CO-, $R^a$-O-CO-, $(R^b)(R^b)$N-CO-, $(R^b)(R^b)$N-CS-, $(R^b)(R^b)$N-CO-CO-, $R^b$-$SO_2$-, oder $(R^b)(R^b)$N-$SO_2$-, worin $R^a$ einen unsubstituierten oder substituierten, gesättigten oder ungesättigten, gegebenenfalls mit Amino, Monoalkylamino, Dialkylamino, Alkanoylamino oder Alkanoyloxyamino funktionalisierten aliphatischen, cycloaliphatischen, cycloaliphatisch-aliphatischen Kohlenwasserstoffrest mit bis zu 18, vorzugsweise 10, Kohlenstoffatomen, einen unsubstituierten oder substituierten aromatischen, heteroaromatischen, aromatisch-aliphatischen oder heteroaromatisch-aliphatischen Kohlenwasserstoffrest mit bis zu 18, vorzugsweise 10, Kohlenstoffatomen oder einen unsubstituierten oder substituierten, gesättigten 5- oder 6-gliedrigen heterocyclischen Rest bedeutet und $R^b$ Wasserstoff bedeutet oder die Bedeutung von $R^a$ besitzt. Der Ausdruck "Acyl" betrifft auch den einwertigen, über die Carboxylgruppe gebundenen Rest einer acylierten Aminosäure oder eines acylierten Dipeptids.

Ein unsubstituierter oder substituierter, gesättigter oder ungesättigter, aliphatischer, cycloaliphatischer oder cycloaliphatisch-aliphatischer Kohlenwasserstoffrest $R^a$ oder $R^b$ ist beispielsweise unsubstituiertes oder substituiertes Alkyl, Alkenyl, Alkinyl, Mono-, Bi- oder Tricycloalkyl, Monocycloalkenyl, Bicycloalkenyl, Cycloalkylalkyl, Cycloalkylalkenyl oder Cycloalkenylalkyl. "Substituiertes Alkyl" bedeutet einen Alkylrest, in welchem ein oder mehrere Wasserstoffatome durch Hydroxy, Alkoxy, Aryloxy, Alkanoyloxy, Halogen, Hydroxysulfonyloxy, Carboxy, Alkoxycarbonyl, Carbamoyl, Alkylcarbamoyl, Dialkylcarbamoyl, Cyan, Phosphono, verestertes Phosphono, Amino oder Oxo substituiert sein können, wobei die Substituenten nur dann in 1-Stellung des Alkylrestes stehen, wenn dieser in der Teilformel $R^b$-CO- an die Carbonylgruppe gebunden ist.

Beispiele von substituiertem Alkyl sind 2-Hydroxyäthyl, Methoxymethyl, 2-Methoxyäthyl, Phenoxymethyl, $\alpha$- oder $\beta$-Naphthoxymethyl, Acetoxymethyl, 2-Acetoxyäthyl, Chlormethyl, Brommethyl, 2-Chlor- oder 2-Bromäthyl, Hydroxysulfonyloxymethyl, 2-Hydroxysulfonyloxyäthyl, Carboxymethyl, 2-Carboxyäthyl, Methoxycarbonylmethyl, 2-Methoxycarbonyläthyl, Aethoxycarbonylmethyl, 2-Aethoxycarbonyläthyl, Carbamoylmethyl, 2-Carbamoyläthyl, Methylcarbamoylmethyl, Dimethylcarbamoylmethyl, Cyanomethyl, 2-Cyanoäthyl, 2-Oxopropyl, 2-Oxobutyl, Hydroxycarboxymethyl, 1-Hydroxy-2-carboxyäthyl, Hydroxyäthoxycarbonyläthyl, Hydroxymethoxycarbonyläthyl, Acetoxymethoxycarbonylmethyl, 1,2-Dihydroxy-2-carboxyäthyl, 1,2-Dihydroxy-2-äthoxycarbonyläthyl, 1,2-Dihydroxy-2-methoxycarbonyläthyl, 1,2-Diacetoxy-2-äthoxycarbonyläthyl, 1,2-Diacetoxy-2-methoxycarbonyläthyl, 1-$\alpha$-Naphthoxy-3-carboxypropyl, 1-$\alpha$-Naphthoxy-2-äthoxycar-

bonyläthyl, 1-α-Naphthoxy-3-t-butoxycarbonylpropyl, 1-α-Naphthoxy-2-benzyloxycarbonyläthyl, 1-α-Naphthoxy-3-carbamoylpropyl, α-Naphthoxycyanomethyl, 1-α-Naphthoxy-3-cyanopropyl, 1-α-Naphthoxy-4-dimethylaminobutyl oder 1-α-Naphthoxy-3-oxobutyl.

Der Ausdruck "Alkenyl" betrifft geradkettige oder verzweigte, ungesättigte Kohlenwasserstoffreste mit 2-8, vorzugsweise 2-4, Kohlenstoffatomen, wobei die Doppelbindung nur dann in 1-Stellung des Alkenylrestes steht, wenn dieser in der Teilformel $R^b$-CO- an die Carbonylgruppe gebunden ist. Beispiele solcher Alkenylreste sind Vinyl, Allyl, 2-Butenyl oder 3-Butenyl. Die Alkenylreste können durch die gleichen Substituenten substituiert sein wie die Alkylreste.

Der Ausdruck "Alkinyl" betrifft Kohlenwasserstoffreste mit 2-8, vorzugsweise 2-4, Kohlenstoffatomen, welche eine Dreifachbindung enthalten, wie Aethinyl, 1-Propinyl oder 2-Propinyl. Der Ausdruck "Bicycloalkyl" betrifft bicyclische gesättigte Kohlenwasserstoffreste mit 5-10, vorzugsweise 6-9, Kohlenstoffatomen, wie Bicyclo[3.1.O]hex-1-yl, Bicyclo[3.1.O]hex-2-yl, Bicyclo[3.1.O]hex-3-yl, Bicyclo[4.1.O]hept-1-yl, Bicyclo[4.1.O]hept-4-yl, Bicyclo[2.2.1]hept-2-yl, Bicyclo[3.2.1]oct-2-yl, Bicyclo[3.3.O]oct-3-yl, Bicyclo[3.3.1]-non-9-yl, α- oder β-Decahydronaphthyl und dergleichen.

Der Ausdruck "Tricycloalkyl" betrifft einen tricyclischen gesattigten Kohlenwasserstoffrest mit 8-10 Kohlenstoffatomen, wie 1-Adamantyl.

Der Ausdruck "Cycloalkenyl" betrifft einen ungesättigten cyclischen Kohlenwasserstoffrest mit 3-8, vorzugsweise 3-6, Kohlenstoffatomen, wie 1-Cyclohexenyl, 1,4-Cyclohexadienyl und dergleichen.

Der Ausdruck "Bicycloalkenyl" betrifft einen bicyclischen ungesättigten Kohlenwasserstoffrest mit 5-10, vorzugsweise 7-10, Kohlenstoffatomen, wie 5-Norbornen-2-yl, Bicyclo[2.2.2]octen-2-yl, Hexahydro-4,7-methanoind-1-en-6-yl und dergleichen.

Beispiele für Cycloalkylalkyl sind Cyclopropylmethyl, Cyclobutylmethyl, Cyclopentylmethyl, Cyclohexylmethyl und dergleichen. Als Beispiele für Cycloalkylalkenyl können Cyclohexylvinyl und Cyclohexylallyl und dergleichen genannt werden. Beispiele für Cycloalkenylalkyl sind 1-Cyclohexenylmethyl, 1,4-Cyclohexadienylmethyl und dergleichen.

Die genannten cycloaliphatischen und cycloaliphatisch-aliphatischen Reste können durch die gleichen Substituenten wie Alkyl substituiert sein .

Ein gegebenenfalls substituierter aromatischer oder aromatisch-aliphatischer Kohlenwasserstoffrest ist beispielsweise unsubstituiertes oder substituiertes Aryl, Arylalkyl oder Arylalkenyl. Beispiele für Arylalkenyl sind Styryl, 3-Phenylallyl, 2-(α-Naphthyl)vinyl, 2-(β-Naphthyl)vinyl und dergleichen.

In einem heteroaromatischen oder heteroaromatisch-aliphatischen Kohlenwasserstoffrest ist der Heterocyclus mono-, bi- oder tricyclisch und enthält ein bis zwei Stickstoffatome und/oder ein Sauerstoff- oder Schwefelatom und ist mit einem seiner Ring-Kohlenstoffatome mit der Gruppe -CO-, -O-CO-, >N-CO-, >N-CS-, >N-CO-CO-, $-SO_2$ oder $>N-SO_2$-verknüpft. Beispiele solcher heteroaromatischer Kohlenwasserstoffreste sind Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder ein benzanneliertes, cyclopenta-, cyclohexa- oder cyclohepta-anneliertes Derivat dieser Reste. Der heteroaromatische Rest kann an einem Stickstoffatom durch Alkyl, Phenyl oder Phenylalkyl, z.B. Benzyl und/oder an einem oder mehreren Kohlenstoffatomen durch Alkyl, Phenyl, Phenylalkyl, Halogen, Hydroxy, Alkoxy, Phenylalkoxy oder Oxo substituiert und teilweise gesättigt sein. Beispiele solcher heteroaromatischer Reste sind 2- oder 3-Pyrrolyl, Phenylpyrrolyl, z.B. 4- oder 5-Phenyl-2-pyrrolyl, 2-Furyl, 2-Thienyl, 2-Imidazolyl, 2-, 3- oder 4-Pyridyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2-indolyl, 1-Benzyl-3-indolyl, 4,5,6,7-Tetrahydro-2-indolyl, Cyclohepta[b]-5-Pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, Benz[e]indol-2-yl, β-Carbolin-3-yl und dergleichen.

Beispiele heteroaromatisch-aliphatischer Kohlenwasserstoffreste sind 2- oder 3-Pyrrolylmethyl, 2-, 3- oder 4-Pyridylmethyl, 2-(2-, 3- oder 4-Pyridyl)äthyl, 4-Imidazolylmethyl, 2-(4-Imidazolyl)äthyl, 2-Indolylmethyl, 3-Indolylmethyl, 2-(3-Indolyl)äthyl, 2-Chinolylmethyl und dergleichen.

Ein gesättigter 5- oder 6-gliedriger heterocyclischer Rest hat mindestens ein Kohlenstoffatom, 1-3 Stickstoffatome und gegebenenfalls ein Sauerstoff- oder Schwefelatom als Ringglieder und ist mit einem seiner Ringkohlenstoffatome mit der Gruppe -CO- bzw. -O-CO-, >N-CO-, >N-CS-, >N-CO-CO-, $-SO_2-$ oder $>N-SO_2-$ verknüpft. Der Heterocyclus kann an einem seiner Kohlenstoffatome oder an einem Ringstickstoffatom durch Alkyl, z.B. Methyl oder Aethyl, Phenyl oder Phenylalkyl, z.B. Benzyl, oder an einem seiner Kohlenstoffatome durch Hydroxy oder Oxo substituiert und/oder an zwei benachbarten Kohlenstoffatomen benzanneliert sein. Beispiele solcher Reste sind Pyrrolidin-3-yl, 4-Hydroxypyrrolidin-2-yl, 5-Oxopyrrolidin-2-yl, Piperidin-2-yl, Piperidin-3-yl, 1-Methylpiperidin-2-yl, 1-Methylpiperidin-3-yl, 1-Methylpiperidin-4-yl, Morpholin-2-yl, Morpholin-3-yl, Thiomorpholin-2-yl, Thiomorpholin-3-yl, 1,4-Dimethylpiperazin-2-yl, 2-Indolinyl, 3-Indolinyl, 1,2,3,4-Tetrahydrochinol-2-, -3- oder -4-yl, 1,2,3,4-Tetrahydroisochinol-1-, -3-oder -4-yl, 1-Oxo-1,2,3,4-tetrahydroisochinol-3-yl und dergleichen.

Als Rest einer über die Carboxylgruppe gebundenen Aminosäure kommen natürliche α-Aminosäuren mit der L-Konfiguration, Homologe solcher Aminosäuren, z.B. worin die Aminosäurenseitenkette um eine oder zwei Methylengruppen verlängert oder verkürzt ist und/oder eine Methylgruppe durch Wasserstoff ersetzt ist, substituierte aromatische α-Aminosäuren, z.B. substituiertes Phenylalanin oder Phenylglycin, worin der Substituent Alkyl, z.B. Methyl, Halogen, z.B. Fluor, Chlor, Brom oder Jod, Hydroxy, Alkoxy, z.B. Methoxy, Alkanoyloxy, z.B. Acetoxy, Amino, Alkylamino, z.B. Methylamino, Dialkylamino, z.B. Dimethylamino, Alkanoylamino, z.B. Acetylamino oder Pivaloylamino, Alkoxycarbonylamino, z.B. t-Butoxycarbonylamino, Arylmethoxycarbonylamino, z.B. Benzyloxycarbonylamino und/oder Nitro sein kann und ein- oder mehrfach vorkommt, benzanneliertes Phenylalanin oder Phenylglycin, wie α-Naphthylalanin, oder hydriertes Phenylalanin oder Phenylglycin, wie Cyclohexylalanin oder Cyclohexylglycin, eine 5- oder 6-gliedrige cyclische benzannelierte α-Aminosäure, z.B. Indolin-2-carbonsäure oder 1,2,3,4-Tetrahydroisochinolin-3-carbonsäure, eine natürliche oder homologe α-Aminosäure, in der eine Carboxygruppe in der Seitenkette in veresterter oder amidierter Form vorliegt, z.B. als Alkylestergruppe, wie Methoxycarbonyl oder t-Butoxycarbonyl, oder als Carbamoyl-, Alkylcarbamoyl, wie Methylcarbamoyl, oder als Dialkylcarbamoylgruppe, wie Dimethylcarbamoyl, in der eine Aminogruppe der Seitenkette in acylierter Form vorliegt, z.B. als Alkanyolamino, wie Acetylamino oder Pivaloylamino, als Alkoxycarbonylamino-, wie t-Butoxycarbonylamino, oder als Arylmethoxycarbonylaminogruppe, wie Benzylocycarbonylamino, oder in der eine Hydroxygruppe der Seitenkette in verätherter oder veresterter Form vorliegt, z.B. als Alkoxygruppe, wie Methoxy, als Arylalkoxygruppe, wie Benzyloxy, oder als nieder Alkanoyloxygruppe, wie Acetoxy, oder Epimere solcher Aminosäuren, d.h. mit der unnatürlichen D-Konfiguration. Beispiele solcher Aminosäuren sind Glycin, Alanin, Valin, Norvalin, Leucin, Isoleucin, Norleucin, Serin, Homoserin, Threonin, Methionin, Cystein, Prolin, Trans-3- und Trans-4-hydroxyprolin, Phenylalanin, Tyrosin, 4-Nitrophenylalanin, 4-Aminophenylalanin, 4-Chlorphenylalanin, β-Phenylserin, Phenylglycin, α-Naphthylalanin, Cyclohexylalanin, Cyclohexylglycin, Tryptophan, Indolin-2-carbonsäure, 1,2,3,4-Tetrahydroisochinolin-3-carbonsäure, Asparaginsäure, Asparagin, Aminomalonsäure, Aminomalonsäuremonoamid, Glutansäure, Gluta minsäuremono-t-butylester, Glutamin, N-Dimethylglutamin, Histidin, Arginin, Lysin, N-t-Butoxycarbonyllysin, δ-Hydroxylysin, Ornithin, N-Pivaloylornithin, α,γ-Diaminobuttersäure oder α,β-Diaminopropionsäure und dergleichen. Der über die Carboxylgruppe gebundene Rest der Aminosäure kann N-terminal zur Erhöhung der Stabilität der Verbindung der Formel I gegen enzymatischen Abbau durch Alkyl, z.B. Methyl oder Aethyl, substituiert sein.

Der über die Carboxylgruppe gebundene Rest eines Dipeptids besteht aus zwei der oben erwähnten Aminosäuren.

Der Ausdruck "acylierte Aminosäure" bzw. "acyliertes Dipeptid" betrifft eine der oben erwähnten Aminosäuren bzw. ein Dipeptid aus zwei der oben erwähnten Aminosäuren, welche bzw. welches N-terminal durch den Acylrest einer Carbonsäure, eines Halbesters der Kohlensäure, einer gegebenenfalls N-substituierten Carbamin- oder Thiocarbaminsäure, eines gegebenenfalls N-substituierten Oxalamids, einer Sulfonsäure oder einer gegebenenfalls N-substituierten Amidosulfonsäure substituiert ist.

Der Ausdruck "pharmazeutisch verwendbare Salze" umfasst Salze mit anorganischen oder organischen Säuren, wie Salzsäure, Bromwasserstoffsäure, Salpetersäure, Schwefelsäure, Phosphorsäure, Zitronensäure, Ameisensäure, Maleinsäure, Essigsäure, Bernsteinsäure, Weinsäure, Methansulfonsäure, p-Toluolsulfonsäure und dergleichen. Solche Salze können im Hinblick auf den Stand der Technik und unter Berücksichtigung der Natur der in ein Salz zu überführenden Verbindung durch jeden Fachmann ohne weiteres hergestellt werden.

Die Verbindungen der Formel I besitzen mindestens drei asymmetrische Kohlenstoffatome und liegen deshalb in Form von optisch reinen Diastereomeren, Diastereomerengemischen, diastereomeren Racematen oder Gemischen von diastereomeren Racematen vor. Die vorliegende Erfindung umfasst alle Formen. Diastereomerengemische, diastereomere Racemate oder Gemische von diastereomeren Racematen können nach üblichen Methoden aufgetrennt werden, z.B. durch Säulenchromatographie, Dünnschichtchromatographie, HPLC und dergleichen.

Bevorzugt sind solche Verbindungen der Formel I, worin $R^1$ Wasserstoff bedeutet. $R^2$ bedeutet vorzugsweise Imidazol-2-yl, Imidazol-4-yl oder Thiazol-4-yl, besonders bevorzugt Imidazol-4-yl. Weiter sind solche Verbindungen der Formel I bevorzugt, worin $R^3$ Cyclohexylmethyl bedeutet. $R^4$ bedeutet vorzugsweise Alkanoyl oder die Gruppe (c) oder (e), besonders bevorzugt Alkanoyl oder die Gruppe (e). Die bevorzugte Bedeutung von $R^7$ ist Heteroarylalkylaminocarbonyl, vorzugsweise Pyridylalkylaminocarbonyl. $R^{10}$ bedeutet vorzugsweise die Gruppe (f), (g) oder (h), besonders bevorzugt die Gruppe (g) oder die Gruppe (h), worin n 0 und $R^7$ Alkylaminocarbonyl bedeuten. Die bevorzugte Bedeutung von $R^{11}$ ist Alkanoyl. $R^{12}$ bedeutet vorzugsweise Wasserstoff. $R^{13}$ ist in den Bedeutungen Alkyl, Arylalkyl, Heteroarylalkyl und

Heteroarylalkylcarbonyl, insbesondere Arylalkyl und Heteroarylalkyl, bevorzugt. Die bevorzugtesten Bedeutungen von $R^{13}$ sind Phenylalkyl und Pyridylalkyl. Ebenfalls bevorzugt sind die Verbindungen der Formel I, worin A die Gruppe (b) bedeutet. Y bedeutet vorzugsweise den N-terminal mit Z verbundenen, bivalenten Rest von Phenylalanin. Z bedeutet vorzugsweise die Gruppe $R^a$-O-CO- oder den Rest einer durch diese Gruppe acylierten α-Aminosäure, vorzugsweise Prolin, worin $R^a$ einen gegebenenfalls substituierten, gesättigten aliphatischen Kohlenwasserstoffrest mit bis zu 10 Kohlenstoffatomen oder einen gegebenenfalls substituierten heteraromatischen Kohlenwasserstoffrest mit bis zu 18 Kohlenstoffatomen, besonders bevorzugt einen gesättigten, aliphatischen Kohlenwasserstoffrest mit bis zu 6 Kohlenstoffatomen, ganz besonders bevorzugt t-Butyl, bedeutet. Falls A die Gruppe (a) bedeutet, so sind diejenigen Verbindungen der Formel I bevorzugt, worin $R^5$ Phenyl oder substituiertes Phenyl, besonders bevorzugt Phenyl, bedeutet. Die bevorzugte Bedeutung von $R^6$ ist Alkylcarbonylalkyl, Aminoalkylcarbonylalkyl, substituiertes Aminoalkylcarbonylalkyl, Aminoalkylsulfonylalkyl, substituiertes Aminoalkylsulfonylalkyl oder Alkylsulfonylalkyl, vorzugsweise Alkylcarbonylalkyl oder Alkylsulfonylalkyl, besonders bevorzugt $C_1$-$C_4$-Alkylcarbonylmethyl oder $C_1$-$C_4$-Alkylsulfonylmethyl.

Aus dem obigen folgt, dass solche Verbindungen der Formel I ganz besonders bevorzugt sind, worin $R^1$ Wasserstoff, $R^2$ Imidazol-4-yl, $R^3$ Cyclohexylmethyl, $R^4$ Alkanoyl oder die Gruppe (e), $R^{10}$ die Gruppe (g) oder die Gruppe (h), worin n 0 und $R^7$ Alkylaminocarbonyl bedeuten, $R^{12}$ Wasserstoff, $R^{13}$ Arylalkyl oder Heteroarylalkyl, vorzugsweise Phenylalkyl oder Pyridylalkyl, Y den N-terminal mit Z verbundenen, bivalenten Rest von Phenylalanin und Z die Gruppe $R^a$-O-CO- oder den durch diese Gruppe acylierten Rest von Prolin bedeuten, worin $R^a$ einen gesättigten, aliphatischen Kohlenwasserstoffrest mit bis zu 6 Kohlenstoffatomen, vorzugsweise t-Butyl, bedeuten.

Ganz speziell bevorzugte Verbindungen der Formel I sind:
(S)-N-[(1S,2S,4R)-4-[(Butylcarbamoyl)methyl]-1-(cyclohexylmethyl)-2-hydroxy-5-methylhexyl]-α-[(R)-α-(3,3-dimethyl-2-oxobutyl)hydrocinnamamido]imidazol-4-propionamid,
t-Butyl [(S)-α-[[(S)-1-[[(1S,2S,4R)-4-[(butylcarbamoyl)methyl]-1-(cyclohexylmethyl)-2-hydroxy-5-methylhexyl]carbamoyl]-2-imidazol-4-yläthyl]carbamoyl]phenäthyl]carbamat,
(S)-N-[(1S,2S,4S)-1-(Cyclohexylmethyl)-2-hydroxy-4-isopropyl-5-oxodecyl]-α-[(R)-α-(3,3-dimethyl-2-oxobutyl]hydrocinnamamido]imidazol-4-propionamid,
(S)-N-[(1S,2S,4S,E)-1-(Cyclohexylmethyl)-2-hydroxy-4-isopropyl-6-[(2-pyridylmethyl)carbamoyl]-5-hexenyl]-α-[(R)-α-(3,3-dimethyl-2-oxobutyl)hydrocinnamamido]imidazol-4-propionamid,
t-Butyl [(S)-α-[[(S)-1-[[(1S,2S,4S)-1-(cyclohexylmethyl)-2-hydroxy-4-isopropyl-6-(propionyloxy)hexyl]-carbamoyl]-2-imidazol-4-yläthyl]carbamoyl]phenäthyl]carbamat,
t-Butyl (R)-2-[[(S)-α-[[(R und S)-1-[[(1S,2S,4S)-1-(cyclohexylmethyl]-2-hydroxy-4-isopropyl-6-(pivaloyloxy)-hexyl]carbamoyl]-2-imidazol-4-yläthyl]carbamoyl]phenäthyl]carbamoyl]-1-pyrrolidincarboxylat,
(2S oder R,3S,5S,6S)-6-(Boc-D-Pro-Phe-His-NH)-7-cyclohexyl-3-isopropyl-1-(phenäthylamino)-2,5-heptandiol,
(2R oder S,3S,5S,6S)-6-(Boc-D-Pro-Phe-His-NH)-7-cyclohexyl-3-isopropyl-1-(phenäthylamino)-2,5-heptandiol und,
(2RS,3S,5S,6S)-6-(Boc-D-Pro-Phe-His-NH)-7-cyclohexyl-3-isopropyl-1-[(2-pyridylmethyl)amino]-2,5-heptandiol.

Die Verbindungen der Formel I in Form von optisch reinen Diastereomeren, Diastereomerengemischen, diastereomeren Racematen oder Gemischen von diastereomeren Racematen sowie pharmazeutisch verwendbare Salze davon können hergestellt werden, indem man

a) eine Verbindung der allgemeinen Formel

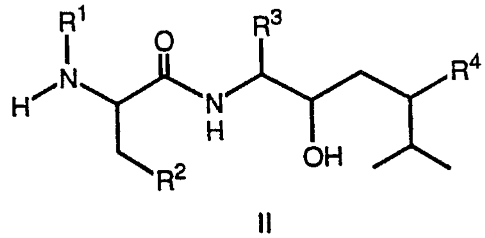

II

worin $R^1$, $R^2$, $R^3$ und $R^4$ die oben angegebene Bedeutung besitzen,
mit einem die Gruppe

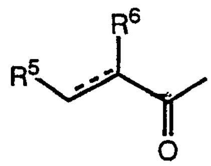

(a)

oder -Y-Z (b)
worin $R^5$, $R^6$, Y, Z und die gestrichelte Linie die oben angegebene Bedeutung besitzen, abgebenden Acylierungsmittel umsetzt, oder

b) eine Verbindung der allgemeinen Formel

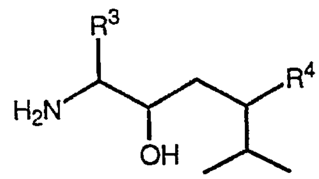

III

worin $R^3$ und $R^4$ die oben angegebene Bedeutung besitzen, mit einer Verbindung der allgemeinen Formel

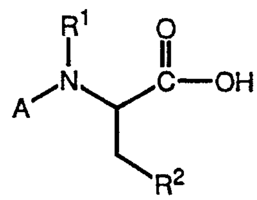

IV

worin $R^1$, $R^2$ und A die oben angegebene Bedeutung besitzen, oder einem aktivierten Derivat davon umsetzt, oder

c) eine der Formel I entsprechende Verbindung, worin Z jedoch Wasserstoff bedeutet und die übrigen Symbole die oben angegebene Bedeutung besitzen, mit einer acylierten Aminosäure oder einem acylierten Dipeptid umsetzt, oder

d) zur Herstellung einer Verbindung der Formel I, worin $R^2$ Imidazol-2-yl, Imidazol-4-yl oder Pyrazol-3-yl und/oder $R^{12}$ Wasserstoff bedeutet, aus einer Verbindung der allgemeinen Formel

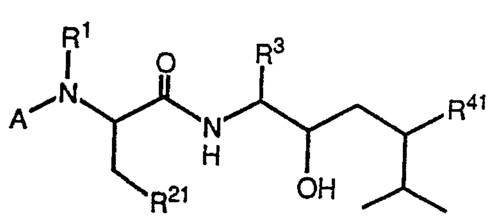

V

worin $R^{21}$ Aethyl, Propyl, Isopropyl, Thiazolyl-4-yl, Thien-2-yl, Aethoxycarbonyl, t-Butylcarbonylmethyl,

Benzyloxycarbonylmethyl, t-Butoxy oder gegebenenfalls N-geschütztes Imidazol-2-yl, Imidazol-4-yl oder Pyrazol-3-yl und $R^{41}$ Alkanoyl, Arylcarbonyl, 2,2-Dialkylvinyl oder eine der Gruppen (c), (d) und (e) bedeuten und die übrigen Symbole die oben angegebene Bedeutung besitzen, mit der Massgabe, dass $R^{21}$ N-geschütztes Imidazol-2-yl, Imidazol-4-yl oder Pyrazol-3-yl und/oder $R^{41}$ die Gruppe (e), worin $R^{10}$ die Gruppe (g) und $R^{12}$ leicht abspaltbares Arylalkyl, Alkoxycarbonyl oder Arylalkoxycarbonyl bedeuten, bedeutet, die N-Schutzgruppe und/oder den Substituenten $R^{12}$ abspaltet, und

e) erwünschtenfalls ein Gemisch diastereomerer Racemate in die diastereomeren Racemate oder optisch reinen Diastereomeren auftrennt, und/oder

f) erwünschtenfalls ein Diastereomerengemisch in die optisch reinen Diastereomeren auftrennt, und/oder

g) erwünschtenfalls eine erhaltene Verbindung in ein pharmazeutisch verwendbares Salz überführt.

Die Acylierung einer Verbindung der Formel II erfolgt nach an sich bekannten Methoden. Geeignete Acylierungsmittel sind insbesondere aktivierte Säurederivate, wie Ester, gemischte Ester, Säurehalogenide und Säureanhydride oder gemischte Säureanhydride. Die Reaktion wird in einem unter den Reaktionsbedingungen inerten organischen Lösungsmittel oder Lösungsmittelgemisch bei einer Temperatur zwischen etwa 0 °C und der Raumtemperatur durchgeführt. Als Lösungsmittel kommen insbesondere aromatische Kohlenwasserstoffe, wie Benzol, Toluol oder Xylol, chlorierte Kohlenwasserstoffe, wie Methylenchlorid oder Chloroform, Aether, wie Diäthyläther, Tetrahydrofuran oder Dioxan, und dergleichen in Frage. Falls es sich beim Acylierungsmittel um ein Peptid handelt, erfolgt die Reaktion unter in der Peptidchemie üblichen Reaktionsbedingungen, d.h. vorzugsweise in Gegenwart eines Kondensationsmittels wie HBTU (O-Benzotriazolyl-N,N,N′,N′-tetramethyluronium-hexafluorophosphat), BOP (Benzotriazol-1-yloxy-bis-(dimethylamino)phosphonium-hexafluorophosphat), BOPC (Bis(2-oxo-2-oxozolidinyl)phosphinchlorid), HOBT (N-Hydroxybenzotriazol), DBU (1,8-Diazabicyclo[5.4.O]undec-7-en), DCC (Dicyclohexylcarbodiimid), EDC (N-Aethyl-N′(3-dimethylaminopropyl)carbodiimid-hydrochlorid), Hünigbase (Aethyldiisopropylamin), und dergleichen. Die Reaktion wird zweckmässigerweise in einem unter den Reaktionsbedingungen inerten organischen Lösungsmittel oder Lösungsmittelgemisch bei einer Temperatur zwischen etwa 0° und 50 °C, vorzugsweise bei etwa Raumtemperatur durchgeführt. Als Lösungsmittel kommen insbesondere Dimethylformamid, Methylenchlorid, Acetonitril, Tetrahydrofuran, und dergleichen in Frage.

Die Umsetzung einer Verbindung der Formel III mit einer Verbindung der Formel IV erfolgt ebenfalls nach an sich in der Peptid-Chemie bekannten Methoden, d.h. unter den gleichen Bedingungen wie oben für die Umsetzung einer Verbindung der Formel II mit einem Peptid angegeben wurde. Beispiele geeigneter aktivierter Derivate einer Verbindung der Formel IV sind Säurehalogenide, Säureanhydride, gemischte Anhydride, Ester, gemischte Ester, und dergleichen.

Die Umsetzung einer Verbindung der Formel I, worin Z Wasserstoff bedeutet, mit einer acylierten Aminosäure oder einem acylierten Dipeptid gemäss Verfahrensvariante c) erfolgt ebenfalls nach an sich in der Peptid-Chemie bekannten Methoden, d.h. unter den oben für die Umsetzung einer Verbindung der Formel II mit einem Peptid angegebenen Bedingungen.

Die Abspaltung der N-Schutzgruppe(n) gemäss Verfahrensvariante d) erfolgt ebenfalls nach an sich bekannten Methoden in Abhängigkeit von der Art der abzuspaltenden N-Schutzgruppe. Die Abspaltung erfolgt jedoch zweckmässig durch saure oder basische Hydrolyse. Für die saure Hydrolyse verwendet man vorteilhafter Weise eine Lösung einer Mineralsäure, wie Chlorwasserstoffsäure, Bromwasserstoffsäure, Trifluoressigsäure, Schwefelsäure, Phosphorsäure und dergleichen, in einem inerten Lösungsmittel oder Lösungsmittelgemisch. Geeignete Lösungsmittel sind Alkohole, wie Methanol oder Aethanol, Aether, wie Tetrahydrofuran oder Dioxan, chlorierte Kohlenwasserstoffe, wie Methylenchlorid, und dergleichen. Für die basische Hydrolyse können Alkalimetallhydroxyde und -carbonate, wie Kalium- oder Natriumhydroxyd oder Kalium- oder Natriumcarbonat, organische Amine, wie Piperidin, und dergleichen verwendet werden. Inerte organische Lösungsmittel, wie sie oben für die saure Hydrolyse genannt sind, können als Lösungsvermittler zugegeben werden. Die Reaktionstemperatur kann für die saure und basische Hydrolyse in einem Bereich von etwa 0 °C bis Rückflusstemperatur variiert werden, wobei man vorzugsweise zwischen etwa 0 °C und der Raumtemperatur arbeitet. Der t-Butoxycarbonylrest wird zweckmässig mit Trifluoressigsäure oder Ameisensäure in Gegenwart oder Abwesenheit eines inerten Lösungsmittels abgespalten. Die Fmoc-Schutzgruppe wird zweckmässig mit Piperidin bei etwa Raumtemperatur abgespalten. Die Benzyloxycarbonylgruppe kann in bekannter Weise durch saure Hydrolyse wie weiter oben beschrieben oder hydrogenolytisch abgespalten werden. Die Benzylgruppe wird zweckmässigerweise hydrogenolytisch abgespalten.

Die Ausgangsstoffe der Formel II sind neu und ebenfalls Gegenstand der vorliegenden Erfindung. Diese Verbindungen können hergestellt werden, indem man eine Verbindung der Formel III mit gegebenenfalls N-methyliertem Histidin, Leucin, Norleucin, Norvalin, Thiazolylalanin, Thienylalanin, Asparaginsäureäthylester, Glutaminsäure-t-butylester, Glutaminsäurebenzylester oder t-Butoxyserin umsetzt. Diese Umsetzung erfolgt

ebenfalls nach in der Peptid-Chemie bekannten Methoden, d.h. unter den Reaktionsbedingungen, wie sie weiter oben für die Umsetzung einer Verbindung der Formel II mit einem Dipeptid beschrieben sind.

Die Ausgangsstoffe der Formel III sind ebenfalls neu und Gegenstand der vorliegenden Erfindung. Sie können beispielsweise hergestellt werden, indem man in einer Verbindung der allgemeinen Formeln

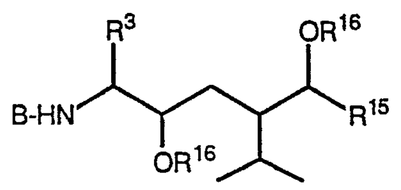

und

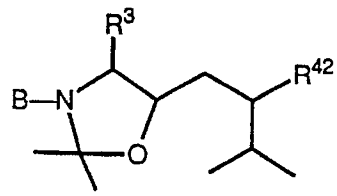

VI VII

worin B eine Aminoschutzgruppe, vorzugsweise t-Butoxycarbonyl oder Benzyloxycarbonyl, $R^{15}$ die Gruppe (f) oder Alkylaminocarbonyläthyl, Arylalkylaminocarbonyläthyl oder Heteroarylalkylaminocarbonyläthyl und $R^{16}$ eine O-Schutzgruppe, vorzugsweise Tetrahydropyranyl oder 1-Aethoxyäthyl, oder $R^{15}$ Alkylaminocarbonyl, Arylalkylaminocarbonyl oder Heteroarylalkylaminocarbonyl und $R^{16}$ Wasserstoff und $R^{42}$ Alkanoyl, Arylcarbonyl, 2,2-Dialkylvinyl oder eine der Gruppen (c), (d) und (e) bedeuten, mit der Massgabe, dass in der Gruppe (e) $R^9$ Wasserstoff bedeutet, wenn $R^{10}$ die Gruppe (f) oder die Gruppe (h) bedeutet, worin n 0 oder 2 und $R^7$ Alkylaminoacarbonyl, Arylalkylaminocarbonyl oder Heteroarylalkylaminocarbonyl bedeuten und $R^3$ die oben angegebene Bedeutung besitzt,
die Aminoschutzgruppe und gegebenenfalls gleichzeitig auch die O-Schutzgruppe(n) abspaltet.

Die Abspaltung der N-Schutzgruppe und gegebenenfalls O-Schutzgruppe(n) erfolgt ebenfalls nach an sich bekannten Methoden, beispielsweise in einem unter den Reaktionsbedingungen inerten organischen Lösungsmittel oder Lösungsmittelgemisch bei einer Temperatur zwischen etwa 0 °C und der Raumtemperatur mit einer Säure, wie Chlorwasserstoffsäure, Trifluoressigsäure, und dergleichen. Geeignete Lösungsmittel sind Aether, wie Tetrahydrofuran oder Dioxan, Alkohole, wie Methanol, oder chlorierte Kohlenwasserstoffe, wie Methylenchlorid, und dergleichen. Unter diesen Reaktionsbedingungen wird in einer Verbindung der Formel VI, worin $R^{16}$ eine O-Schutzgruppe bedeutet, bzw. in einer Verbindung der Formel VII - wie bereits erwähnt - gleichzeitig die O-Schutzgruppen abgespalten bzw. der Oxazolidinring aufgespalten.

Die Ausgangsstoffe der Formel IV sind bekannt oder können in Analogie zur Herstellung der bekannten Verbindungen erhalten werden.

Die Verbindungen der Formeln VI und VII sind ebenfalls neu und Gegenstand vorliegender Erfindung. Sie können nach verschiedenen, an sich bekannten Methoden ausgehend von Verbindungen der Formel VIII hergestellt werden. Diese Herstellungsverfahren sind in den nachfolgenden Schemata I-IV zusammengefasst. In diesen Schemata bedeuten $R^{71}$ Alkyl, Aryl oder Heteroaryl, $R^{72}$ Alkylaminocarbonyl, Arylalkylaminocarbonyl oder Heteroarylalkylaminocarbonyl, $R^{73}$ Alkyl oder Aryl, $R^{121}$ und $R^{131}$ unabhängig voneinander je Wasserstoff, Alkyl, Arylalkyl, Heteroarylalkyl oder Aryl oder $R^{121}$ und $R^{131}$ zusammen mit dem sie verbindenden Stickstoffatom einen 5- oder 6-gliedrigen Heterocyclus oder gegebenenfalls substituiertes Benzimidazolonyl, $R^{161}$ eine O-Schutzgruppe, Et Aethyl und X p-Tolylsulfonyl, Methylsulfonyl, Trifluormethylsulfonyl oder p-Brombenzolsulfonyl und besitzen B, $R^3$, $R^{12}$ und $R^{13}$ die oben angegebene Bedeutung. Bezüglich der genauen Reaktionsbedingungen wird auf den Beispielteil verwiesen.

Schema I

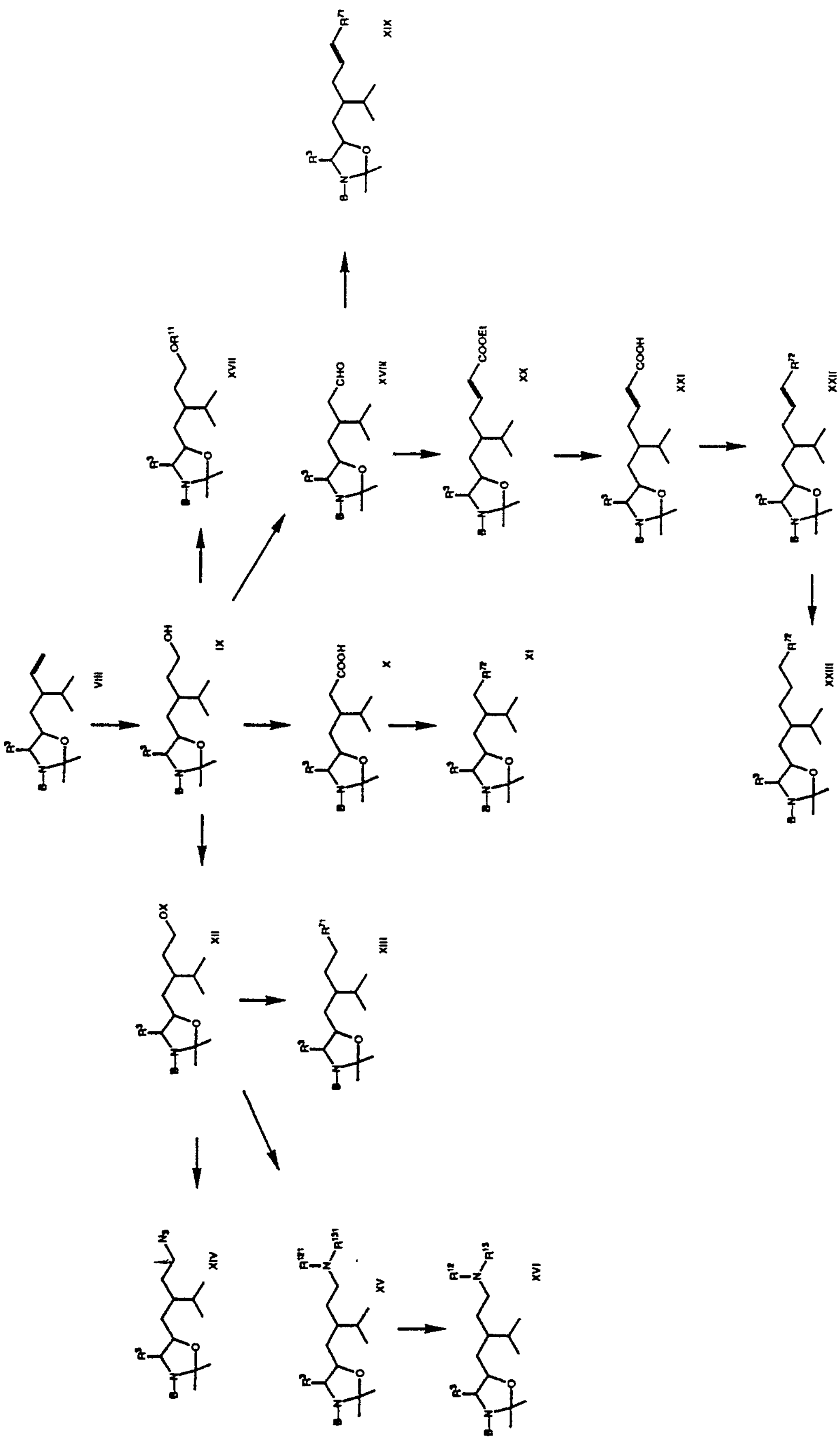

VIII IX X XI XII XIII XIV XV XVI XVII XVIII XIX XX XXI XXII XXIII

Schema II

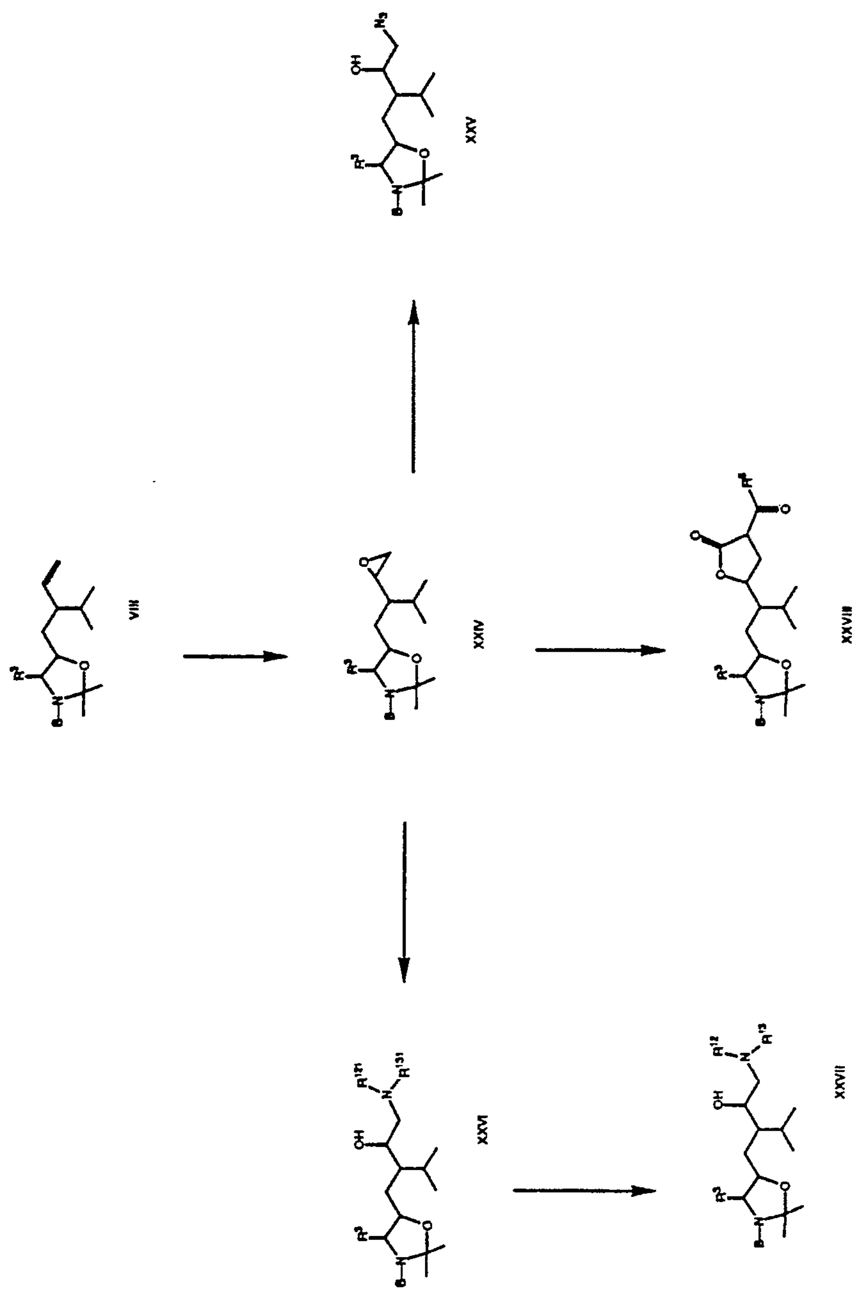

VIII

XXIV

XXV

XXVIII

XXVI

XXVII

Schema III

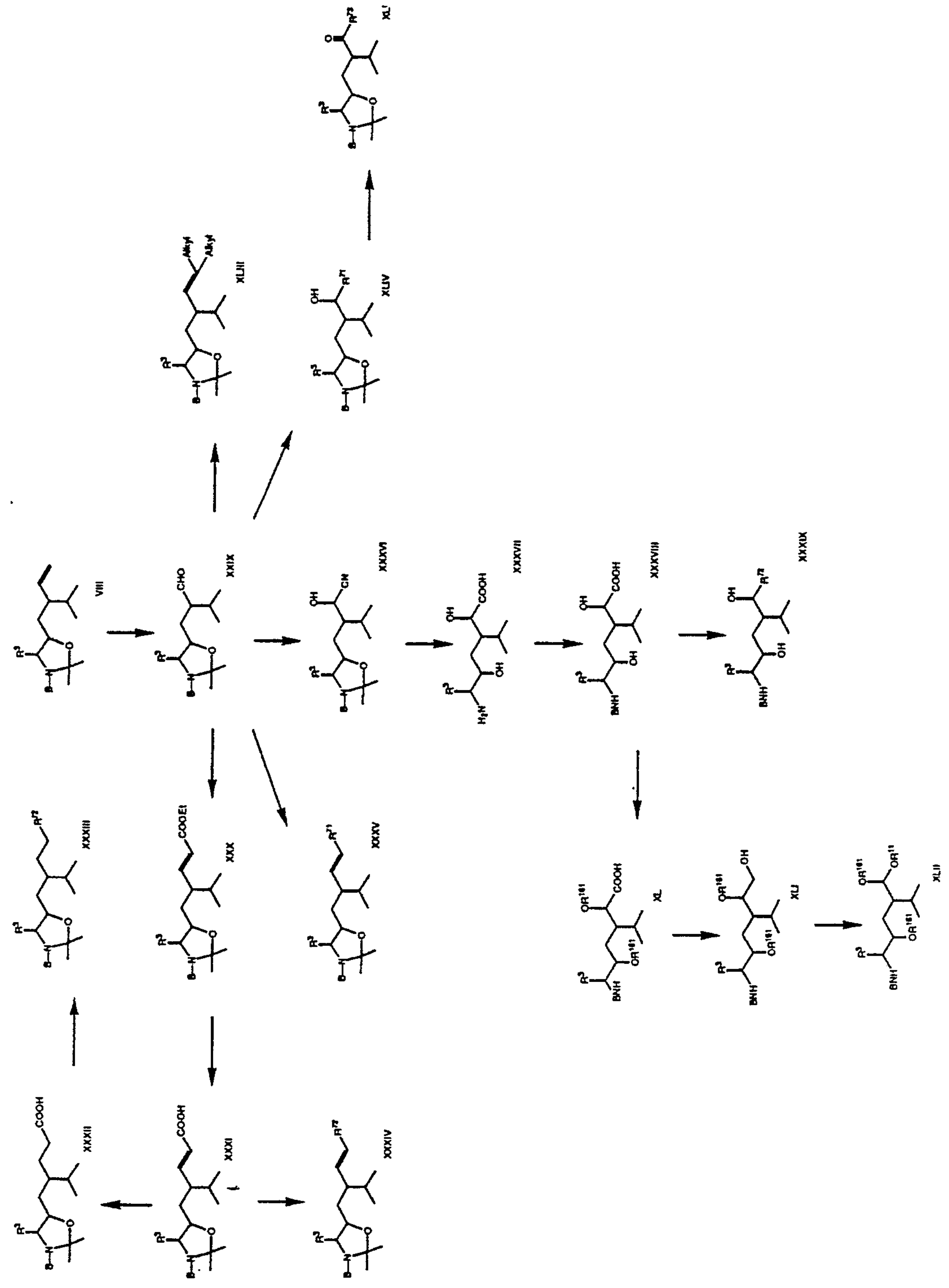

VIII XXIX XXXVI XXXVII XXXVIII XXXIX XLIII XLIV XLV XXXIII XXX XXXV XL XLI XLII XXXII XXXI XXXIV

SCHEMA IV

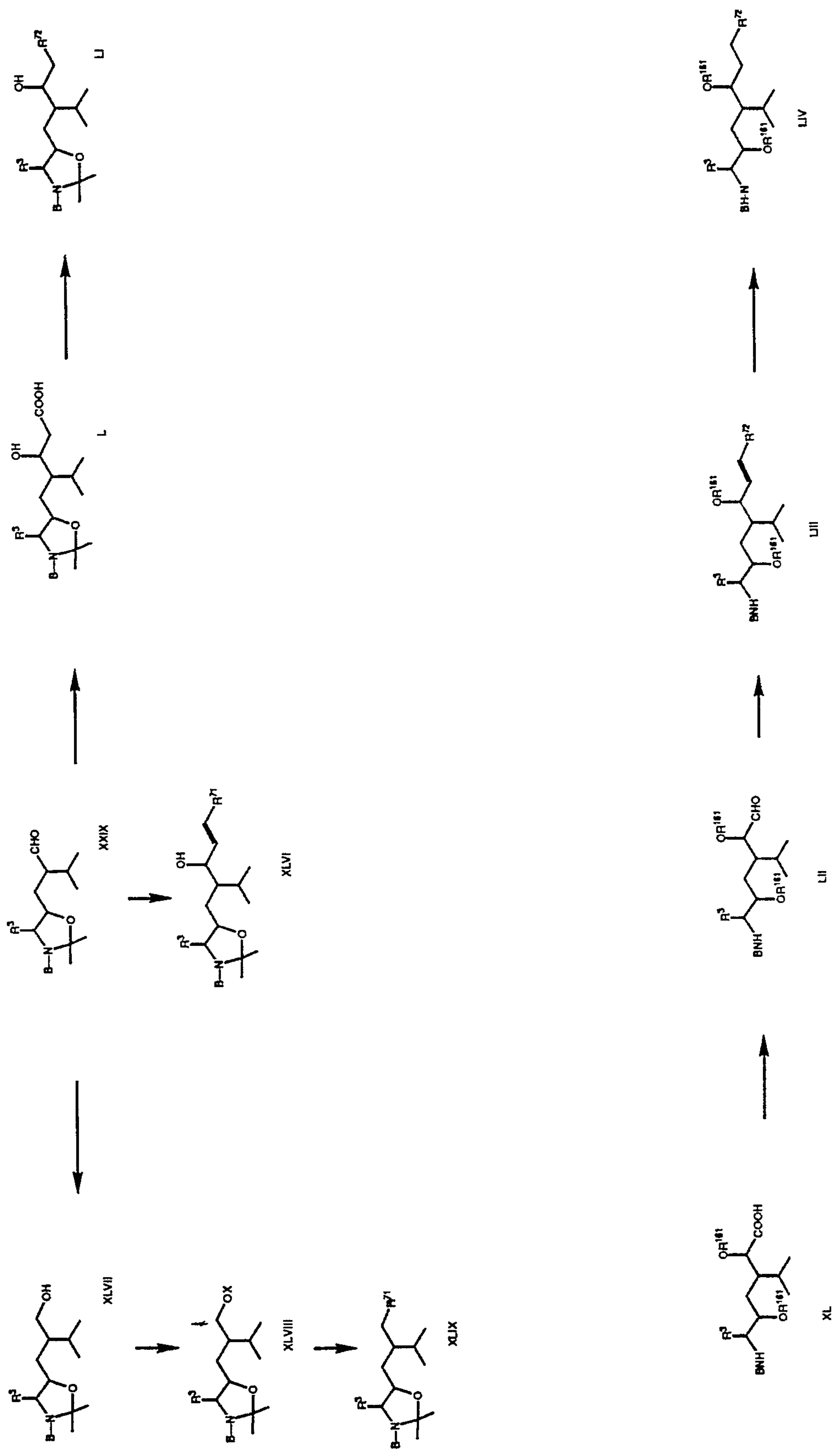

LI, L, XXIX, XLVI, XLVII, XLVIII, XLIX, LIV, LIII, LII, XL

Es versteht sich von selbst, dass es sich bei den Verbindungen den Formeln XXXIX, XLII und LIV um solche handelt, die unter die obige Formel VI fallen, während die Verbindungen der Formeln XI, XIII-XVII, XIX, XXII, XXIII, XXV-XXVIII, XXXIII-XXXV, XLIII-XLVII, XLIX und LI unter die obige Formel VII fallen.

Alle in den Schemata I-IV aufscheinenden Verbindungen mit Ausnahme derjenigen der Formeln VIII und

XXIX sind neu und ebenfalls Gegenstand der vorliegenden Erfindung.

Die Verbindungen der Formel VIII sind ebenfalls neu und können beispielsweise hergestellt werden, indem man einen Aldehyd der allgemeinen Formel

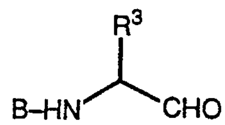

LV

worin B und $R^3$ die oben angegebene Bedeutung besitzen,
mit einer Verbindung der allgemeinen Formel

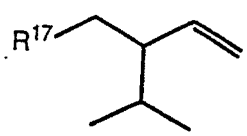

LVI

worin $R^{17}$ Chlor, Brom oder Jod, vorzugsweise Brom, bedeutet,
in einer Grignard-Reaktion umsetzt. Diese Umsetzung erfolgt ebenfalls nach an sich bekannten Methoden, beispielsweise in einem unter den Reaktionsbedingungen inerten Lösungsmittel, wie einem Aether, bei einer Temperatur zwischen etwa 0° und 50° C, vorzugsweise bei Raumtemperatur. Anschliessend wird die erhaltene Verbindung der allgemeinen Formel

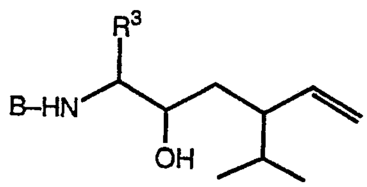

LVII

worin B und $R^3$ die oben angegebene Bedeutung besitzen,
mit 2,2-Dimethoxypropan in Gegenwart von p-Toluolsulfonsäure zur erwünschten Verbindung der Formel VIII umgesetzt.

Die Ausgangsstoffe der Formel V, worin $R^{21}$, N-geschütztes Imidazol-2-yl, Imidazol-4-yl oder Pyrazol-3-yl bedeutet, sind ebenfalls neu und Gegenstand der vorliegenden Erfindung. Sie können beispielsweise hergestellt werden, indem man eine Verbindung der Formel III mit einer der Formel IV entsprechenden Verbindung, worin $R^2$ jedoch N-geschütztes Imidazol-2-yl, Imidazol-4-yl oder Pyrazol-3-yl bedeutet, oder einem aktivierten Derivat davon umsetzt. Die Umsetzung erfolgt nach an sich in der Peptid-Chemie bekannten Methoden, d.h. unter den gleichen Bedingungen wie oben für die Umsetzung einer Verbindung der Formel III mit einer Verbindung der Formel IV angegeben wurde.

Die übrigen Verbindungen der Formel V fallen unter die Formel I und können nach den für die Herstellung der Verbindungen der Formel I angegebenen Verfahren hergestellt werden.

Die Verbindungen der Formel I und ihre pharmazeutisch verwendbaren Salze weisen hemmende Wirkung des natürlichen Enzyms Renin auf. Letzteres gelangt aus den Nieren in das Blut und bewirkt dort die Spaltung von Angiotensinogen unter Bildung des Dekapeptids Angiotensin I, das dann in der Lunge, den Nieren und anderen Organen zum Octapeptid Angiotensin II gespalten wird. Angiotensin II erhöht den Blutdruck sowohl direkt durch arterielle Konstriktion, als auch indirekt durch die Freisetzung des Natriumionen zurückhal tenden Hormons Aldosteron aus den Nebennieren, womit ein Anstieg des extrazellulären Flüssigkeitsvolumens verbunden ist. Dieser Anstieg ist auf die Wirkung von Angiotensin II selber oder des

daraus als Spaltprodukt gebildeten Heptapeptids Angiotensin III zurückzuführen. Hemmer der enzymatischen Aktivität von Renin bewirken eine Verringerung der Bildung von Angiotensin I und als Folge davon die Bildung einer geringeren Menge von Angiotensin II. Die verminderte Konzentration dieses aktiven Peptid-Hormons ist die unmittelbare Ursache für die blutdrucksenkende Wirkung von Renin-Hemmern.

Die Wirkung von Renin-Hemmern kann experimentell mittels des nachstehend beschriebenen In vitro-Tests gezeigt werden:

In vitro-Test mit reinem Human-Renin

Der Test wird in Eppendorf Röhrchen durchgeführt. Die Inkubationsmischung besteht aus (1) 100 µl Human-Renin in Puffer A (0,1M Natriumphosphatlösung, pH 7,4, enthaltend 0,1% Rinderserumalbumin, 0,1 % Natriumazid und 1 mM Aethylendiamintetraessigsäure), genügend für eine Renin-Aktivität von 2-3 ng Angiotensin I/ ml/Std.; (2) 145 µl Puffer A; (3) 30 µl von 10 µM humanem Tetradekapeptid-Reninsubstrat (hTD) in 10 mM Salzsäure; (4) 15 µl Dimethylsulfoxid mit bzw. ohne Hemmer und (5) 10 µl einer 0,03 molaren Lösung von Hydroxychinolinsulfat in Wasser.

Die Proben werden drei Stunden bei 37° C bzw. 4° C in Triplikaten inkubiert. 2 x 100 µl Proben pro Versuchsröhrchen werden dann verwendet, um die Produktion von Angiotensin I via RIA (standard radioimmunoassay; Clinical Assay solid phase kit) zu messen. Kreuzreaktivitäten der verwendeten Antikörper im RIA sind: Angiotensin I 100 %; Angiotensin II 0,0013 %; hTD (Angiotensin I-Val-Ile-His-Ser-OH) 0,09 %. Die Produktion von Angiotensin I wird durch die Differenz zwischen dem Versuch bei 37° C und demjenigen bei 4° C bestimmt.

Folgende Kontrollen werden mitgeführt:

(a) Inkubation von hTD-Proben ohne Renin und ohne Hemmer bei 37° C und 4° C. Die Differenz zwischen diesen beiden Werten ergibt den Grundwert der Angiotensin I-Produktion.

(b) Inkubation von hTD-Proben mit Renin, jedoch ohne Hemmer bei 37° C und 4° C. Die Differenz zwischen diesen Werten ergibt den Maximalwert der Angiotensin I-Produktion.

In jeder Probe wird von der ermittelten Angiotensin I-Produktion der Grundwert der Angiotensin I-Produktion abgezogen. Die Differenz zwischen dem Maximalwert und dem Grundwert ergibt den Wert der maximalen Substrathydrolyse (=100%) durch Renin.

Die Resultate werden als $IC_{50}$-Werte angegeben, welche diejenige Konzentration des Hemmers bezeichnen, bei welcher die enzymatische Aktivität um 50% gehemmt wird. Die $IC_{50}$-Werte werden aus einer linearen Regressionskurve aus einem logit-log plot ermittelt.

Die in diesem Test erhaltenen Resultate sind in der folgenden Tabelle zusammengefasst:

Tabelle

| Verbindung | $IC_{50}$-Werte in µMol/lt. |
|---|---|
| A | 0,003 |
| B | 0,009 |
| C | 0,007 |
| D | 0,013 |
| E | 0,009 |
| F | 0,010 |
| G | 0,011 |
| H | 0,004 |
| I | 0,008 |

A = (S)-N-[(1S,2S,4R)-4-[(Butylcarbamoyl)methyl]-1-(cyclohexylmethyl)-2-hydroxy-5-methylhexyl]-α-[-(R)-α-(3,3-dimethyl-2-oxobutyl)hydrocinnamamido]imidazol-4-propionamid,

B = t-Butyl [(S)-α-[[(S)-1-[[(1S,2S,4R)-4-[(butylcarbamoyl)methyl]-1-(cyclohexylmethyl)-2-hydroxy-5-methylhexyl]carbamoyl]-2-imidazol-4-yläthyl]carbamoyl]phenäthyl]carbamat,

C = (S)-N-[(1S,2S,4S)-1-(Cyclohexylmethyl)-2-hydroxy-4-isopropyl-5-oxodecyl]-α-[(R)-α-(3,3-dimethyl-2-oxobutyl]hydrocinnamamido]imidazol-4-propionamid,

D = (S)-N-[(1S,2S,4S,E)-1-(Cyclohexylmethyl)-2-hydroxy-4-isopropyl-6-[(2-pyridylmethyl)carbamoyl]-5-hexenyl]-α-[(R)-α-(3,3-dimethyl-2-oxobutyl)hydrocinnamamido]imidazol-4-propionamid,

E = t-Butyl [(S)-α-[[(S)-1-[[(1S,2S,4S)-1-(cyclohexylmethyl)-2-hydroxy-4-isopropyl-6-(propionyloxy)-hexyl]carbamoyl]-2-imidazol-4-yläthyl]carbamoyl]phenäthyl]carbamat,

F = t-Butyl (R)-2-[[(S)-α-[[(R und S)-1-[[(1S,2S,4S)-1-(cyclohexylmethyl]-2-hydroxy-4-isopropyl-6-(pivaloyloxy) hexyl]carbamoyl]-2-imidazol-4-yläthyl]carbamoyl]phenäthyl]carbamoyl]-1-pyrrolidincarboxylat,

G = (2S oder R,3S,5S,6S)-6-(Boc-D-Pro-Phe-His-NH)-7-cyclohexyl-3-isopropyl-1-(phenäthylamino)-2,5-heptandiol,

H = (2R oder S,3S,5S,6S)-6-(Boc-D-Pro-Phe-His-NH)-7-cyclohexyl-3-isopropyl-1-(phenäthylamino)-2,5-heptandiol und

I = (2RS,3S,5S,6S)-6-(Boc-D-Pro-Phe-His-NH)-7-cyclohexyl-3-isopropyl-1-[(2-pyridylmethyl)amino]-2,5-heptandiol.

Die Verbindungen der Formel I sowie deren pharmazeutisch verwendbare Salze können als Heilmittel, z.B. in Form pharmazeutischer Präparate, Verwendung finden. Die pharmazeutischen Präparate können enteral, wie oral, z.B. in Form von Tabletten, Lacktabletten, Dragees, Hart- und Weichgelatinekapseln, Lösungen, Emulsionen oder Suspensionen, nasal, z.B. in Form von Nasensprays, oder rektal, z.B. in Form von Suppositorien, verabreicht werden. Die Verabreichung kann aber auch parenteral, wie intramuskulär oder intravenös, z.B. in Form von Injektionslösungen, erfolgen.

Zur Herstellung von Tabletten, Lacktabletten, Dragees und Hartgelatinekapseln können die Verbindungen der Formel I sowie ihre pharmazeutisch verwendbaren Salze mit pharmazeutisch inerten, anorganischen oder organischen Excipientien verarbeitet werden. Als solche Excipientien kann man z.B. für Tabletten, Dragees und Hartgelatinekapseln, Laktose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Sacharose, Invertzucker, Glukose etc.

Für Injektionslösungen eignen sich als Excipientien z.B. Wasser, Alkohole, Polyole, Glycerin, vegetabile Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, viskositätserhöhende Stoffe, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der allgemeinen Formel I sowie deren pharmazeutisch verwendbare Salze bei der Bekämpfung bzw. Verhütung von Bluthochdruck und Herzinsuffizienz verwenden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 3 mg bis etwa 3 g, vorzugsweise etwa 10 mg bis etwa 1 g, z.B. ungefähr 300 mg pro Person, verteilt auf vorzugsweise 1-3 Einzeldosen, die z.B. gleich gross sein können, angemessen sein, wobei aber die soeben angegebene obere Grenze auch überschritten werden kann, wenn sich dies als angezeigt erweisen sollte. Ueblicherweise erhalten Kinder die halbe Dosis von Erwachsenen.

Die folgenden Beispiele sollen die vorliegende Erfindung erläutern, sie jedoch in keiner Weise einschränken. Alle Temperaturen sind in Celsiusgraden angegeben. Es werden die folgenden Abkürzungen bzw. Kombinationen davon verwendet:

Boc = t-Butoxycarbonyl
Fmoc = 9-Fluorenylmethoxycarbonyl
H-His-OH = L-Histidin
H-Phe-OH = L-Phenylalanin
H-D-Pro-OH = D-Prolin
H-Phe-His-OH = N-[(S)-2-Amino-3-phenylpropyl]-L-histidin
(Phe-His-NH) = L-Phenylalaninyl-L-histidinamido
$(Fmoc)_2$His-OH = N-α-N-im-Di-Fmoc-L-histidin
Boc-Phe-His(Boc)-OH = Boc-L-phenylalaninyl-N-im-Boc-L-histidin

## Beispiel 1

Ein Gemisch von 290 mg (0.36 mMol) Benzyl [(2RS,3S,5S,6S)-7-cyclohexyl-2,5-dihydroxy-3-isopropyl-6-[[N-(3-phenyl-L-alanyl)-L-histidyl]amino]heptyl]-(2-pyridylmethyl)carbamat, 86 mg (0.40 mMol) Boc-D-Pro-OH, 0.092 ml (0.73 mMol) 4-Aethylmorpholin, 108 mg (0.80 mMol) HOBT, 84 mg (0.44 mMol) EDC und 10 ml Dimethylformamid wird 2 Tage bei Raumtemperatur gerührt. Danach wird das Reaktionsgemisch auf 70 ml eiskalte 2N Natriumbicarbonatlösung gegossen und dreimal mit je 150 ml Essigester extrahiert. Die organischen Extrakte werden nacheinander mit 70 ml Natriumbicarbonatlösung und 70 ml gesättigter Kochsalzlösung gewaschen, vereinigt, getrocknet und eingedampft. Chromatographie des Rückstands an 25 g Kieselgel mit einem 14:1:0.1-Gemisch von Methylenchlorid, Methanol und Ammoniak liefert nach Umkristallisation aus Methylenchlorid/Aether/Hexan 280 mg Benzyl [(2RS,3S,5S,6S)-6-(Boc-D-Pro-Phe-His-NH)-7-cyclohexyl-2,5-dihydroxy-3-isopropylheptyl]-(2-pyridylmethyl)carbamat als weissen Festkörper, Schmelzpunkt 108°.

Das als Ausgangstoff eingesetzte Benzyl [(2RS,3S,5S,6S)-7-cyclohexyl-2,5-dihydroxy-3-isopropyl-6-[[N-(3-phenyl-L-alanyl)-L-histidyl]amino]heptyl]-(2-pyridylmethyl)carbamat wurde wie folgt hergestellt:

100 mg (0.27 mMol) t-Butyl [(1S,2S,4S,5RS)-1-(cyclohexylmethyl)-5,6-epoxy-2-hydroxy-4-isopropylhexyl]carbamat und 0.136 ml (1.35 mMol) 2-Aminommethylpyridin in 5 ml Methanol werden 24 Stunden unter Argon zum Rückfluss erhitzt. Danach wird das Reaktionsgemisch zur Trockene eingedampft, und der Rückstand an 20 g Kieselgel mit einem 14:1:0.1-Gemisch von Methylenchlorid, Methanol und Ammoniak als Eluierungsmittel chromatographiert, wobei 100 mg t-Butyl [(1S,2S,4S,5RS)-1-(cyclohexylmethyl)-2,5-dihydro-4-isopropyl-6-(2-pyridylmethyl)hexyl]carbamat als Oel erhalten werden, MS: 477 $(M + H)^+$.

Ein Gemisch von 770 mg (1.49 mMol) t-Butyl [(1S,2S,4S,5RS)-1-(cyclohexylmethyl)-2,5-dihydro-4-isopropyl-6-(2-pyridylmethyl)hexyl]carbamat , 0.414 ml (2,97 mMol) Triäthylamin, 4.44 mg (1.784 mMol) N-(Benzyloxycarbonyloxy)succinimid und 20 ml Methylenchlorid wird 1,5 Stunden bei Raumtemperatur gerührt. Danach wird das Reaktionsgemisch unter vermindertem Druck zur Trockene eingedampft, und der Rückstand in 250 ml Aether gelöst. Die Aetherlösung wird nacheinander mit 80 ml 2N Natriumbicarbonatlösung und Kochsalzlösung gewaschen, getrocknet und unter vermindertem Druck eingedampft. Der Rückstand (1,2 g) wird über Nacht in 9 ml 3N methanolischer Salzsäure stehen gelassen und danach unter vermindertem Druck zur Trockene eingedampft. Chromatographie des Rückstands an 100 g Kieselgel mit einem 150:10:1-Gemisch von Methylenchlorid, Methanol und Ammoniak liefert 640 mg (2RS,3S,5S,6S)-6-Amino-7-cyclohexyl-3-isopropyl-1-[(2-pyridylmethyl)amino]-2,5-heptandiol als Oel, MS: 377 $(M + H)^+$.

Ein Gemisch von 350 mg (0.68 mMol) (2RS,3S,5S,6S)-6-Amino-7-cyclohexyl-3-isopropyl-1-[(2-pyridylmethyl)amino]-2,5-heptandiol, 450 mg (0.75 mMol) $(Fmoc)_2$His-OH, 0.17 ml (1.37 mMol) 4-Aethylmorpholin, 203 mg (1.5 mMol) HOBT, 157 mg (0.82 mMol) EDC und 10 ml Dimethylformamid wird über Nacht bei Raumtemperatur gerührt, anschliessend auf 70 ml eiskalte 2N Natriumbicarbonatlösung gegossen und dreimal mit je 150 ml Essigester extrahiert. Die organischen Extrakte werden nacheinander mit 70 ml Natriumbicarbonatlösung und 70 ml gesättigter Kochsalzlösung gewaschen, vereinigt, getrocknet und eingedampft. Der Rückstand (910 mg) wird in 30 ml Methylenchlorid und 1 ml Piperidin 2,5 Stunden bei Raumtemperatur gerührt. Danach wird das Reaktionsgemisch eingedampft, und der Rückstand mit Hexan angerieben. Abfiltrieren des ausgefallenen Niederschlags liefert 620 mg eines Festkörpers, der an 50 g Kieselgel mit einem 14:1:0.1-Gemisch von Methylenchlorid, Methanol und Ammoniak chromatographiert wird. Auf diese Weise werden 360 mg Benzyl [(2RS,3S,5S,6S)-6-[(S)-α-amino-4-imidazolpropionamido]-7-cyclohexyl-2,5-dihydroxy-3-isopropylheptyl]-(2-pyridylmethyl)carbamat als Schaum erhalten, MS: 649 $(M + H)^+$.

Ein Gemisch von 340 mg (0.52 mMol) Benzyl [(2RS,3S,5S,6S)-6-[(S)-α-amino-4-imidazolpropionamido]-7-cyclohexyl-2,5-dihydroxy-3-isopropylheptyl]-(2-pyridylmethyl)carbamat, 225 mg (0.58 mMol) Fmoc-Phe-OH, 0.132 ml (1.05 mMol) 4-Aethylmorpholin, 155 mg (1.15 mMol) HOBT, 121 mg (0.63 mMol) EDC und 10 ml Dimethylformamid wird über Nacht bei Raumtemperatur gerührt. Danach wird das Reaktionsgemisch auf 70 ml eiskalte 2N Natriumbicarbonatlösung gegossen und dreimal mit je 150 ml Essigester extrahiert. Die organischen Extrakte werden nacheinander mit 70 ml Natriumbicarbonatlösung und 70 ml gesättigter Kochsalzlösung gewaschen, vereinigt, getrocknet und eingedampft. Der Rückstand (640 mg) wird in 30 ml Methylenchlorid und 1 ml Piperidin 2,5 Stunden bei Raumtemperatur gerührt. Dann wird das Reaktionsgemisch eingedampft, und der Rückstand (500 mg) an 50 mg Kieselgel mit einem 14:1:0.1-Gemisch von Methylenchlorid, Methanol und Ammoniak als Eluierungsmittel chromatographiert. Umkristallisation des erhaltenen Rohprodukts (320 mg) aus Methylenchlorid/Aether/Hexan liefert 280 mg Benzyl [(2RS,3S,5S,6S)-7-cyclohexyl-2,5-dihydroxy-3-isopropyl-6-[[N-(3-phenyl-L-alanyl)-L-histidyl]amino]heptyl]-(2-pyridylmethyl)-carbamat als weissen Festkörper, Schmelzpunkt 94°.

## Beispiel 2

100 mg (0,1 mMol) Benzyl [(2RS,3S,5S,6S)-6-(Boc-D-Pro-Phe-His-NH)-7-cyclohexyl-2,5-dihydroxy-3-isopropylheptyl]-2-(pyridylmethyl)carbamat werden in 15 ml 1N methanolischem Ammoniak in Gegenwart von 40 mg 5%igem Palladium auf Kohle 4 Stunden bei Raumtemperatur und Atmosphärendruck hydriert. Nach beendeter Wasserstoffaufnahme wird der Katalysator abfiltriert, das Filtrat unter vermindertem Druck zur Trockene eingedampft, und der Rückstand aus Methylenchlorid/Aether umkristallisiert. Auf diese Weise erhält man 90 mg (2RS,3S,5S,6S)-6-(Boc-D-Pro-Phe-His-NH)-7-cyclohexyl-3-isopropyl-1-[(2-pyridylmethyl)-amino]-2,5-heptandiol als weissen Festkörper, Schmelzpunkt 103°.

## Beispiel 3

In analoger Weise wie in Beispiel 1 beschrieben, wurden ausgehend von t-Butyl [(1S,2S,4S,5RS)-1-(cyclohexylmethyl)-5,6-epoxy-2-hydroxy-4-isopropylhexyl]carbamat durch Umsetzen mit einem Amin, Einführen der Carbobenzoxy-Schutzgruppe und Abspalten der Boc-Schutzgruppe, Umsetzen mit $(Fmoc)_2$His-OH und Abspalten der Fmoc-Schutzgruppe mit Piperidin, Umsetzen mit Fmoc-Phe-OH und Abspalten der Fmoc-Schutzgruppe mit Piperidin und Umsetzen mit Boc-D-Pro-OH die folgenden Verbindungen hergestellt:
- Unter Verwendung von Butylamin das Benzyl [(2RS,3S,5S,6S)-6-(Boc-D-Pro-Phe-His-NH)-7-cyclohexyl-2,5-dihydroxy-3-isopropylheptyl]butylcarbamat als weisser Festkörper, Schmelzpunkt 111° (aus Methylenchlorid/Aether/Hexan), MS: 959 $(M + H)^+$ und
- unter Verwendung von Phenäthylamin das Benzyl (2S oder R,3S,5S,6S)-6-[(Boc-D-Pro-Phe-His-NH)-7-cyclohexyl-2,5-dihydroxy-3-isopropylheptyl]phenäthylcarbamat als weisser Festkörper, Schmelzpunkt 98° (aus Methylenchlorid/Aether/Hexan), MS: 1007 $(M + H)^+$.

## Beispiel 4

In analoger Weise wie in Beispiel 1 beschrieben, wurden die beiden folgenden, epimeren Verbindungen hergestellt:
- Aus Benzyl [(2S oder R,3S,5S,6S)-7-cyclohexyl-2,5-dihydroxy-6-(His-NH)-3-isopropylheptyl]-phenäthylcarbamat und Boc-Phe-OH das Benzyl [(2S oder R,3S,5S,6S)-6-[[N-[N-(t-butoxycarbonyl)-3-phenyl-L-alanyl]-L-histidyl]amino]-7-cyclohexyl-2,5-dihydroxy-3-isopropylheptyl]phenäthylcarbamat als weisser Festkörper, MS: 910 $(M + H)^+$ und
- aus Benzyl [(2R oder S,3S,5S,6S)-7-cyclohexyl-2,5-dihydroxy-6-(His-NH)-3-isopropylheptyl]-phenäthylcarbamat und Boc-Phe-OH das Benzyl [(2R oder S,3S,5S,6S)-6-[[N-[N-(t-butoxycarbonyl)-3-phenyl-L-alanyl]-L-histidyl]amino]-7-cyclohexyl-2,5-dihydroxy-3-isopropylheptyl]phenäthylcarbamat als weisser Festkörper, Schmelzpunkt 80° (Zers., aus Methylenchlorid/Aether/Methanol/Hexan), MS: 910 $(M + H)^+$.

Die als Ausgangsstoffe eingesetzten epimeren Verbindungen Benzyl [(2S oder R,3S,5S,6S)-7-cyclohexyl-2,5-dihydroxy-6-(His-NH)-3-isopropylheptyl]phenäthylcarbamat und Benzyl [(2R oder S,3S,5S,6S)-7-cyclohexyl-2,5-dihydroxy-6-(His-NH)-3-isopropylheptyl]phenäthylcarbamat wurden, in analoger Weise wie in Beispiel 1 beschrieben, durch Umsetzen von t-Butyl [(1S,2S,4S,5RS)-1-(cyclohexylmethyl)-5,6-epoxy-2-hydroxy-4-isopropylhexyl]carbamat mit Phenäthylamin, Einführen der Carbobenzoxy-Schutzgruppe und Abspalten der Boc-Schutzgruppe mit Salzsäure in Methanol, Umsetzen der erhaltenen Verbindung mit $(Fmoc)_2$His-OH und Abspalten der Fmoc-Schutzgruppe mit Piperidin und anschliessender säulenchromatographischer Auftrennung der beiden epimeren Verbindungen jeweils als weisser Schaum hergestellt, MS: 662 $(M + H)^+$.

## Beispiel 5

In analoger Weise wie in Beispiel 2 beschrieben, wurden durch hydrogenolytische Abspaltung der Carbobenzoxy-Schutzgruppe die folgenden Verbindungen hergestellt:
- Aus Benzyl [(2RS,3S,5S,6S)-6-(Boc-D-Pro-Phe-His-NH)-7-cyclohexyl-2,5-dihydroxy-3-isopropylheptyl]-

butylcarbamat das (2RS,3S,5S,6S)-6-(Boc-D-Pro-Phe-His-NH)-1-(butylamino)-7-cyclohexyl-3-isopropyl-2,5-heptandiol als weisser Festkörper, Schmelzpunkt 110° (aus Methylenchlorid/Aether), MS: 825 $(M + H)^+$;

- aus Benzyl (2S oder R,3S,5S,6S)-6-[(Boc-D-Pro-Phe-His-NH)-7-cyclohexyl-2,5-dihydroxy-3-isopropylheptyl]phenäthylcarbamat das (2R oder S,3S,5S,6S)-6-(Boc-D-Pro-Phe-His-NH)-7-cyclohexyl-3-isopropyl-1-(phenäthylamino)-2,5-heptandiol und das epimere (2S oder R,3S,5S,6S)-6-(Boc-D-Pro-Phe-His-NH)-7-cyclohexyl-3-isopropyl-1-(phenäthylamino)-2,5-heptandiol jeweils als Schaum, MS (jeweils): 873 $(M + H)^+$, welche säulenchromatographisch aufgetrennt wurden;
- aus Benzyl [(2S oder R,3S,5S,6S)-6-[[N-[N-(t-butoxycarbonyl)-3-phenyl-L-alanyl]-L-histidyl]amino]-7-cyclohexyl-2,5-dihydroxy-3-isopropylheptyl]phenäthylcarbamat das t-Butyl [(S)-α-[[(S)-1-[[(1S,2S,4S,5S oder R)-1-cyclohexyl-2,5-dihydroxy-4-isopropyl-6-(phenäthylamino)hexyl]carbamoyl]-2-imidazol-4-yläthyl]-carbamoyl]phenäthyl]carbamat als Schaum, MS: 776 $(M + H)^+$ und
- aus Benzyl [(2R oder S,3S,5S,6S)-6-[[N-[N-(t-butoxycarbonyl)-3-phenyl-L-alanyl]-L-histidyl]amino]-7-cyclohexyl-2,5-dihydroxy-3-isopropylheptyl]phenäthylcarbamat das t-Butyl [(S)-α-[[(S)-1-[[(1S,2S,4S,5R oder S)-1-cyclohexyl-2,5-dihydroxy-4-isopropyl-6-(phenäthylamino)hexyl]carbamoyl]-2-imidazol-4-yläthyl]-carbamoyl]phenäthyl]carbamat als Schaum, MS: 776 $(M + H)^+$.

## Beispiel 6

490 mg (0.565 mMol) (S)-N-[(1S,2S,4S,5S oder R)-1-(Cyclohexylmethyl)-2,5-dihydroxy-6-[4-(2-hydroxyäthyl)-1-piperazinyl]-4-isopropylhexyl]-α-[(R)-α-(3,3-dimethyl-2-oxobutyl)hydrocinnamamido]-3-t-butoxycarbonylimidazol-4-propionamid werden in 5.5 ml Methanol gelöst, mit 23 mg Kaliumcarbonat versetzt und 5 Stunden bei Raumtemperatur gerührt. Danach wird das Reaktionsgemisch eingedampft, und der Rückstand an 35 g Kieselgel mit einem 110:10:1-Gemisch von Methylenchlorid, Methanol und Ammoniak als Eluierungsmittel chromatographiert, wobei man 347 mg (S)-N-[(1S,2S,4S,5S oder R)-1-(Cyclohexylmethyl)-2,5-dihydroxy-6-[4-(2-hydroxyäthyl)-1-piperazinyl]-4-isopropylhexyl]-α-[(R)-α-(3,3-dimethyl-2-oxobutyl)hydrocinnamamido]imidazol-4-propionamid als weissen Schaum erhält, MS: 768 $(M + H)^+$.

Das als Ausgangsstoff eingesetzte (S)-N-[(1S,2S,4S,5S oder R)-1-(Cyclohexylmethyl)-2,5-dihydroxy-6-[4-(2-hydroxyäthyl)-1-piperazinyl]-4-isopropylhexyl]-α-[(R)-α-(3,3-di methyl-2-oxobutyl)hydrocinnamamido]-3-t-butoxycarbonylimidazol-4-propionamid wurde wie folgt hergestellt:

315.3 g (1.28 mMol) t-Butoxycarbonyl-L-phenylalaninmethylester werden nach der von J. Boger et al. in J.Med.Chem., <u>28</u>, 1779 (1985) beschriebenen Methode in Gegenwart von 5%-igem Rhodium auf Aluminiumoxyd bei Raumtemperatur und 440 kPa hydriert, wobei man 315.3 g 2-t-Butoxycarbonylamino-3-cyclohexylpropionsäuremethylester als Oel erhält, das direkt in die nächste Stufe eingesetzt wird.

Zu 85.61 g (300 mMol) 2-t-Butoxycarbonylamino-3-cyclohexylpropionsäuremethylester in 1.2 l Toluol werden bei -75° 750 ml (750 mMol) einer 20%igen Lösung von Diisobutylaluminiumhydrid in Hexan im Verlauf von 90 Minuten getropft. Nach beendeter Zugabe wird das Reaktionsgemisch noch 10 Minuten bei -75° gerührt. Anschliessend werden je innerhalb von 25 Minuten bei einer Temperatur von -75° bis -70° zuerst 70 ml Methanol und dann 840 ml gesättigte Kalium-Natrium-Tartrat-Lösung zugetropft. Die sich langsam auf Raumtemperatur erwärmte milchige Reaktionslösung wird danach mit Aether extrahiert, und die Extrakte getrocknet und eingedampft, wobei man 82 g 2-t-Butoxycarbonylamino-3-cyclohexylpropylaldehyd als Oel erhält, welches ohne weitere Reinigung in die nächste Stufe eingesetzt wird.

Zu 24.65 g Magnesiumspänen in 300 ml Aether wird in einer Argonatmosphäre und bei einer Temperatur zwischen 30° und der Rückflusstemperatur des Lösungsmittels innerhalb von 2.5 Stunden eine Lösung von 179.7 g (1.01 mMol) 4-Brom-3-isopropyl-1-buten (welches nach der von R.G. Helmquist in Tetrahendron Letters, 2533 (1982) beschriebenen Methode hergestellt wurde) in 900 ml Aether getropft. Nach beendeter Zugabe wird das Reaktionsgemisch 3.5 Stunden zum Rückfluss erhitzt. Zum auf -60° abgekühlten Reaktionsgemisch wird innerhalb 75 Minuten eine Lösung von 82 g 2-t-Butoxycarbonylamino-3-cyclohexylpropylaldehyd in 900 ml Aether getropft, wobei die Temperatur -60° bis -70° beträgt. Nach beendeter Zugabe wird das Kühlbad entfernt, und das Reaktionsgemisch 21 Stunden bei Raumtemperatur unter Argon gerührt. Dann kühlt man auf 5° ab und gibt unter Rühren vorsichtig 225 ml einer gesättigten Ammoniumchloridlösung zu, wobei die Temperatur auf 20° ansteigt. Die beiden Phasen werden getrennt, und die organische Phase über Natriumsulfat getrocknet und eingedampft, wobei man 127.5 g eines gelben Oels erhält. Chromatographische Auftrennung dieses Oels an 2.5 kg Kieselgel mit Methylenchlorid, welches 2.5 % Aether enthält, als Eluierungsmittel liefert 33.9 g (αS,βS)-β-t-Butoxycarbonylamino-α-[(S)-2-isopropyl-3-butenyl]cyclohexylpropanol, 13.62 g (αS,βS)-β-t-Butoxycarbonylamino-α-[(R)-2-isopropyl-3-butenyl]-

cyclohexylpropanol sowie 35.5 g eines Gemisches der beiden oben genannten Diastereomeren in Form von Oelen, MS: 354 $(M + H)^+$.

1.0 g (2.83 mMol) (αS,βS)-β-t-Butoxycarbonylamino-α-[(S)-2-isopropyl-3-butenyl]cyclohexylpropanol in 15 ml 2,2-Dimethoxypropan und 30 mg p-Toluolsulfonsäure wird über Nacht bei Raumtemperatur gerührt. Danach wird das Reaktionsgemisch auf 2N Natriumbicarbonatlösung gegossen und dreimal mit je 150 ml Aether extrahiert. Die Aetherextrakte werden mit Wasser gewaschen, vereinigt, getrocknet und eingedampft, wobei man 1,2 g t-Butyl (4S,5S)-4-(cyclohexylmethyl)-5-[(S)-2-isopropyl-3-butenyl]-3-oxazolidincarboxylat als Oel erhält, MS: 393 $(M + H)^+$.

1,2 g (ca. 3mMol) t-Butyl (4S,5S)-4-(cyclohexylmethyl)-5-[(S)-2-isopropyl-3-butenyl]-3-oxazolidincarboxylat in 50 ml Methylenchlorid werden nacheinander mit 1,2 g Natriumbicarbonat und 1,27 g m-Chlorperbenzoesäure (85%ig, 6mMol) versetzt. Danach wird das Reaktionsgemisch bei Raumtemperatur über Nacht gerührt, anschliessend auf 2N Natriumbicarbonatlösung gegossen und zweimal mit 200 ml Methylenchlorid extrahiert. Die organischen Extrakte werden mit 2N Natriumbicarbonatlösung und mit Wasser gewaschen, vereinigt, getrocknet und eingedampft. Chromatographie des Rückstands (1,2 g) an 90 g Kieselgel mit einem 98:2-Gemisch von Toluol und Essigester als Eluierungsmittel liefert 535 mg t-Butyl (4S,5S)-4-(cyclohexylmethyl)-5-[(2S,3R oder S)-3,4-epoxy-2-isopropylbutyl]-2,2-dimethyl-3-oxazolidincarboxylat als polarere Komponente und 465 mg der epimeren Verbindung t-Butyl (4S,5S)-4-(cyclohexylmethyl)-5-[(2S,3S oder R)-3,4-epoxy-2-isopropylbutyl]-2,2-dimethyl-3-oxazolidincarboxylat als weniger polare Komponente, beide als Oel, MS (jeweils): 409 $(M + H)^+$.

In analoger Weise wie in Beispiel 1 beschrieben, wurde t-Butyl (4S,5S)-4-(cyclohexylmethyl)-5-[(2S,3R oder S)-3,4-epoxy-2-isopropylbutyl]-2,2-dimethyl-3-oxazolidincarboxylat in das (S)-N-[(1S,2S,4S,5S oder R)-1-(Cyclohexylmethyl)-2,5-dihydroxy-6-[4-(2-hydroxyäthyl)-1-piperazinyl]-4-isopropylhexyl]-α-[(R)-α-(3,3-dimethyl-2-oxobutyl)hydrocinnamamido]-3-t-butoxycarbonylimidazol-4-propionamid überge führt, und zwar durch Umsetzen mit N-Hydroxyäthylpiperazin unter Bildung von t-Butyl (4S,5S)-4-(cyclohexylmethyl)-5-[-(2S,3R oder S)-3-hydroxy-4-[4-(2-hydroxyäthyl)-1-piperazinyl]-2-isopropylbutyl]-2,2-dimethyl-3-oxazolidincarboxylat (Oel, MS: 539 $(M + H)^+$), Abspalten der Boc-Schutzgruppe mit Salzsäure in Methanol unter gleichzeitiger Oeffnung des Oxazolidinrings und Umsetzen mit 1-(t-Butoxycarbonyl)-N-[(R)-α-(3,3-dimethyl-2-oxobutyl)hydrocinnamoyl]-L-histidin.

Das ebenfalls als Ausgangstoff eingesetzte 1-(t-Butoxycarbonyl)-N-[(R)-α-(3,3-dimethyl-2-oxobutyl)-hydrocinnamoyl]-L-histidin wurde wie folgt hergestellt:

Eine Suspension von 3,0 g (12 mMol) (R)-α-(Pivaloylmethyl)hydrozimtsäure (vgl. EPA 0.184.550) und 2,66 g (11 mMol) L-Histidinmethylester-dihydrochlorid in 340 ml Dimethylformamid wird bei Raumtemperatur unter einer Stickstoffatmosphäre mit 3,45 g (34 mMol) Triäthylamin und 4,58 g (12 mMol) HBTU versetzt. Das Reaktionsgemisch wird 5 Stunden bei Raumtemperatur gerührt und anschliessend im Hochvakuum eingedampft. Der Rückstand wird in 500 ml Essigester gelöst und nacheinander mit 100 ml Wasser, dreimal je 100 ml gesättigter Natriumbicarbonatlösung und 100 ml gesättigter Natriumchloridlösung gewaschen. Die organische Phase wird über Natriumsulfat getrocknet, unter vermindertem Druck eingedampft, und das erhaltene gelbliche Rohprodukt an Kieselgel mit einem 95:5-Gemisch von Methylenchlorid und Methanol, welches 0,1% Ammoniak enthält, chromatographiert. Man erhält auf diese Weise 3,6 g N-[(R)-α-(3,3-Dimethyl-2-oxobutyl)hydrocinnamoyl]-L-histidinmethylester als farblosen Schaum, MS: 399 $(M + H)^+$.

Eine Lösung von 3,56 g (8,9 mMol) N-[(R)-α-(3,3-Dimethyl-2-oxobutyl)hydrocinnamoyl]-L-histidinmethylester und 9,36 ml 1N Natronlauge in 50 ml Methanol wird 15 Stunden bei Raumtemperatur gerührt und danach in der Kälte unter vermindertem Druck eingedampft. Man löst den Rückstand in 70 ml Dioxan und 30 ml Wasser, tropft bei Raumtemperatur eine Lösung von 2,95 g (13,5 mMol) Di-t-butyldicarbonat in 100 ml Dioxan zu und rührt danach 15 Stunden bei Raumtemperatur. Zur Aufarbeitung wird die Reaktionslösung unter vermindertem Druck auf ca. 1/3 ihres Volumens eingeengt und dann mit 200 ml Essigester verdünnt. Nach Zugabe von 50 ml Eiswasser wird das Reaktionsgemisch auf pH 2,5 gestellt, und die wässrige Phase mit festem Natriumchlorid gesättigt. Die wässrige Phase wird noch zweimal mit Essigester extrahiert, und die vereinigten Essigesterphasen über Natriumsulfat getrocknet und eingedampft. Das erhaltene Rohpodukt wird an Kieselgel mit einem 95:5-Gemisch von Methylenchlorid und Methanol, welches 0,1% Essigsäure enthält, chromatographiert, wobei man 3,5 g 1-(t-Butoxycarbonyl)-N-[(R)-α-(3,3-dimethyl-2-oxobutyl)-hydrocinnamoyl]-L-histidin als farbloses Pulver erhält, MS: 486 $(M+H)^+$.

## Beispiel 7

In analoger Weise wie in Beispiel 6 beschrieben, wurden die folgenden Verbindungen hergestellt:

- Aus (S)-N-[(1S,2S,4S,5R oder S)-6-(Dibenzylamino)-1-(cyclohexylmethyl)-2,5-dihydroxy-4-isopropylhexyl]-α-[(R)-α-(3,3-dimethyl-2-oxobutyl)hydrocinnamamido]-3-t-butylcarbonylimidazol-4-propionamid das (S)-N-[(1S,2S,4S,5R oder S)-6-(Dibenzylamino)-1-(cyclohexylmethyl)-2,5-dihydroxy-4-isopropylhexyl]-α-[(R)-α-(3,3-dimethyl-2-oxobutyl)hydrocinnamamido]imidazol-4-propionamid als Schaum, MS: 835 $(M + H)^+$;
- aus (S)-N-[(1S,2S,4S,5R oder S)-6-[4-(Benzylamino)piperidino)-1-(cyclohexylmethyl)-2,5-dihydroxy-4-isopropylhexyl]-α-[(R)-α-(3,3-dimethyl-2-oxobutyl)hydrocinnamamido]-3-t-butoxycarbonylimidazol-4-propionamid das (S)-N-[(1S,2S,4S,5R oder S)-6-[4-(Benzylamino)piperidino]-1-(cyclohexylmethyl)-2,5-dihydroxy-4-isopropylhexyl]-α-[(R)-α-(3,3-dimethyl-2-oxobutyl)hydro cinnamamido]imidazol-4-propionamid als weisser Festkörper, Schmelzpunkt 81° (Zers., aus Methylenchlorid/Aether/Hexan), MS: 828 $(M + H)^+$ und
- aus (S)-N-[(1S,2S,4S,5R oder S)-6-Azido-1-(cyclohexylmethyl)-2,5-dihydroxy-4-isopropylhexyl]-α-[(R)-α-(3,3-dimethyl-2-oxobutyl)hydrocinnamamido]-3-t-butoxycarbonylimidazol-4-propionamid das (S)-N-[(1S,2S,4S,5R oder S)-6-Azido-1-(cyclohexylmethyl)-2,5-dihydroxy-4-isopropylhexyl]-α-[(R)-α-(3,3-dimethyl-2-oxobutyl)hydrocinnamamido]imidazol-4-propionamid als weisser Schaum, MS: 680 $(M + H)^+$.

Die als Ausgangsstoffe eingesetzten Propionamide wurden, in analoger Weise wie in Beispiel 6 beschrieben, wie folgt hergestellt:

<u>(S)-N-[(1S,2S,4S,5R oder S)-6-(Dibenzylamino)-1-(cyclohexylmethyl)-2,5-dihydroxy-4-isopropylhexyl]-α-[(R)-α-(3,3-dimethyl-2-oxobutyl)hydrocinnamamido]- 3-t-butylcarbonylimidazol-4-propionamid</u>

Durch Umsetzen von t-Butyl (4S,5S)-4-(cyclohexylmethyl)-5-[(2S,3R oder S)-3,4-epoxy-2-isopropylbutyl]-2,2-dimethyl-3-oxazolidincarboxylat mit N,N-Dibenzylamin wurde das t-Butyl (4S,5S)-4-(cyclohexylmethyl)-5-[(2S,3R oder S)-4-(dibenzylamino)-3-hydroxy-2-isopropylbutyl]-2,2-dimethyl-3-oxazolidincarboxylat als Oel erhalten, MS: 606 $(M + H)^+$, aus welchem durch Abspalten der Boc-Schutzgruppe unter gleichzeitiger Oeffnung des Oxazolidinrings mit Salzsäure in Methanol und anschliessendem Umsetzen mit 1-(t-Butoxycarbonyl)-N-[(R)-α-(3,3-dimethyl-2-oxobutyl)hydrocinnamoyl]-L-histidin das obige Propionamid hergestellt wurde, das ohne weitere Reinigung in die nächste Stufe eingesetzt wurde.

<u>(S)-N-[(1S,2S,4S,5R oder S)-6-[4-(Benzylamino)piperidino)-1-(cyclohexylmethyl)-2,5-dihydroxy-4-isopropylhexyl]-α-[(R)-α-(3,3-dimethyl-2-oxobutyl)hydrocinnamamido]-3-t-butoxycarbonylimidazol-4-propionamid</u>

Durch Umsetzen von t-Butyl (4S,5S)-4-(cyclohexylmethyl)-5-[(2S,3R oder S)-3,4-epoxy-2-isopropylbutyl]-2,2-dimethyl-3-oxazolidincarboxylat mit 4-Benzylaminopiperidin wurde das t-Butyl (4S,5S)-5-[(2S,3R oder S)-4-[(benzylamino)piperidino]-3-hydroxy-2-isopropylbutyl]-4-(cyclohexylmethyl)-2,2-dimethyl-3-oxazolidincarboxylat als Oel erhalten, MS 599 $(M + H)^+$, aus welchem durch Abspalten der Boc-Schutzgruppe unter gleichzeitiger Oeffnung des Oxazolidinrings mit Salzsäure in Methanol und anschliessendem Umsetzen mit 1-(t-Butoxycarbonyl)-N-[(R)-α-(3,3-dimethyl-2-oxobutyl)hydrocinnamoyl]-L-histidin das obige Propionamid hergestellt wurde, das ohne weitere Reinigung direkt in die nächste Stufe eingesetzt wurde.

<u>(S)-N-[(1S,2S,4S,5R oder S)-6-Azido-1-(cyclohexylmethyl)-2,5-dihydroxy-4-isopropylhexyl]-α-[(R)-α-(3,3-dimethyl-2-oxobutyl)hydrocinnamamido]-3-t-butoxycarbonylimidazol-4-propionamid</u>

Durch Umsetzen von t-Butyl (4S,5S)-4-(cyclohexylmethyl)-5-[(2S,3R oder S)-3,4-epoxy-2-isopropylbutyl]-2,2-dimethyl-3-oxazolidincarboxylat mit Ammoniumazid wurde das t-Butyl (4S,5S)-5-[(2S,3R oder S)-4-azido-3-hydroxy-2-isopropylbutyl)-4-(cyclohexylmethyl)-2,2-dimethyl-3-oxazolidincarboxylat als weisser Schaum erhalten, MS: 453 $(M + H)^+$, aus welchem durch Abspalten der Boc-Schutzgruppe unter gleichzeitiger Oeffnung des Oxazolidinrings mit Salzsäure in Methanol und anschliessendem Umsetzen mit 1-(t-Butoxycarbonyl)-N-[(R)-α-(3,3-dimethyl-2-oxobutyl)hydrocinnamoyl]-L-histidin das obige Propionamid hergestellt wurde, welches direkt in die nächste Stufe eingesetzt wurde.

<u>Beispiel 8</u>

Ein Gemisch von 410 mg (1 mMol) t-Butyl [(1S,2S,4S,5RS)-1-(cyclohexylmethyl)-5,6-epoxy-2-hydroxy-4-isopropylhexyl]carbamat, 0.38 ml (2.5 mMol) Malonsäurediäthylester, 3.02 ml (3.5 mMol) 1.16 N äthanolisches Natriumäthylat und 4 ml Aethanol wird unter Argon 8 Stunden zum Rückfluss erhitzt. Danach wird das Reaktionsgemisch dreimal mit je 150 ml Essigester extrahiert, und die organischen Extrakte zweimal mit je 70 ml Wasser gewaschen, vereinigt, über Magnesiumsulfat getrocknet und eingedampft. Das zurückbleibende Oel (460 mg) wird an 30 g Kieselgel mit einem 95:5-Gemisch von Toluol und Essigester als Eluierungsmittel chromatographiert, wobei 160 mg eines Oels erhalten werden. Dieses Oel wird, in analoger Weise wie in Beispiel 6 beschrieben, zur Abspaltung der Boc-Schutzgruppe unter gleichzeitiger Oeffnung des Oxazolidinrings mit methanolischer Salzsäure behandelt und anschliessend mit Boc-Phe-His-(Boc)-OH (vgl. US Patentschrift 4.599.198) umgesetzt. Durch Abspalten der Boc-Schutzgruppe am Imidazolring mit Kaliumcarbonat in Methanol werden 62 mg (27 %) t-Butyl [(S)-α-[[(S)-1-[[(1S,2S,4S)-1-(cyclohexylmethyl)-2-hydroxy-5-methyl-4-[(2RS,4RS)-tetrahydro-4-(methoxycarbonyl)-5-oxo-2-furyl]hexyl]-carbamoyl]-2-imidazol-4-yläthyl]carbamoyl]phenäthyl]carbamat als weisser Festkörper erhalten, Schmelzpunkt 95° (aus Methylenchlorid/Aether/Hexan), MS: 754 $(M + H)^+$.

## Beispiel 9

Man löst 174 mg (0.5 mMol) (2S,3S,5S)-2-Amino-1-cyclohexyl-5-isopropyl-7-phenoxy-3-heptanol in 8 ml Acetonitril, versetzt die Lösung mit 0.102 ml (0.5 mMol) Hünigbase, 220 mg (0.5 mMol) BOP und 204 mg (0.5 mMol) Boc-Phe-His-OH und rührt das Reaktionsgemisch 3,5 Stunden bei Raumtemperatur. Danach wird das Gemisch in Essigester aufgenommen, und die organische Phase je einmal mit 1N Salzsäure und 2N Natrium carbonatlösung gewaschen, über Natriumsulfat getrocknet und unter vermindertem Druck eingedampft. Chromatographie des Rückstands an Kieselgel mit einem 15:1-Gemisch von Methylenchlorid und Methanol als Eluierungsmittel liefert 226 mg (60%) t-Butyl [(S)-α-[[1-[[(1S,2S,4S)-1-(cyclohexylmethyl)-2-hydroxy-4-isopropyl-6-phenoxyhexyl]carbamoyl]-2-imidazol-4-yläthyl]carbamoyl]phenäthyl]carbamat als Harz, MS: 732 $(M + H)^+$.

Das als Ausgangsstoff eingesetzte (2S,3S,5S)-2-Amino-1-cyclohexyl-5-isopropyl-7-phenoxy-3-heptanol wurde wie folgt hergestellt:

8.0 g (22.6 mMol) (αS,βS)-β-t-Butoxycarbonylamino-α-[(S)-2-isopropyl-3-butenyl]cyclohexylpropanol werden in 50 ml 2.2-Dimethoxypropan gelöst, mit 0.8 g p-Toluolsulfonsäure-hydrat versetzt und über Nacht bei Raumtemperatur gerührt. Das nach üblicher Aufarbeitung erhaltene Rohprodukt (5.34 g; 13.56 mMol) wird in 200 ml Hexan gelöst, unter Eiskühlung rasch mit 0.5 ml Boran-Dimethylsulfid-Komplex (5 mMol bezogen auf Borhydrid) tropfenweise versetzt und danach 1 Stunde zum Rückfluss erhitzt. Dann lässt man die Reaktionslösung auf Raumtemperatur abkühlen, gibt nacheinander 30 ml Aethanol und 1.67 ml 3N Natronlauge zu und tropft schliesslich unter Eiskühlung 1.67 ml 30%iges Wasserstoffperoxid zu. Nach beendeter Zugabe wird die Reaktionslösung 1 Stunde zum Rückfluss erhitzt. Danach giesst man das Reaktionsgemisch auf 200 ml Wasser, extrahiert es mit Essigester, trocknet die Extrakte über Natriumsulfat und dampft diese unter vermindertem Druck zur Trockene ein. Flash-Chromatographie des Rückstands an Kieselgel mit einem 1:1-Gemisch von Aether und Petroläther als Eluierungsmittel liefert 4 g (71%) t-Butyl 4-(cyclohexylmethyl)-5-(4-hydroxy-2-isopropylbutyl)-2,2-dimethyl-3-oxazolidincarboxylat als hellgelben Festkörper, MS: 396 $(M-CH_3)^+$.

Eine Lösung von 1.0 g (2.43 mMol) t-Butyl 4-(cyclohexylmethyl)-5-(4-hydroxy-2-isopropylbutyl)-2,2-dimethyl-3-oxazolidincarboxylat in 70 ml Tetrahydrofuran wird nacheinander mit 0.637 g (2.56 mMol) Triphenylphosphin und 228 mg (2.43 mMol) Phenol versetzt. Dazu tropft man 0.418 g (2.42 mMol) Azodicarbonsäurediäthylester in 10 ml Tetrahydrofuran innerhalb von 5 Minuten zu. Danach wird das Reaktionsgemisch 20 Stunden bei Raumtemperatur gerührt, anschliessend unter vermindertem Druck eingedampft und schliesslich mit einem Gemisch von Aether und Petroläther versetzt. Danach wird der ausgefallene Festkörper abfiltriert, und das Filtrat unter vermindertem Druck eingedampft. Flash-Chromatograpie des Rückstands an Kieselgel mit einem 2:3-Gemisch von Aether und Petroläther als Eluierungsmittel liefert 459 mg (39%) t-Butyl (4S,5S)-4-(cyclohexylmethyl)-5-[(S)-2-isopropyl-4-phenoxybutyl)-2,2-dimethylbutyl-3-oxazolidincarboxylat als hellgelbes Harz, MS: 486 $(M-CH_3)^+$.

Eluierungsmittel liefert 182 mg (58%) (2S,3S,5S)-2-Amino-1-cyclohexyl-5-isopropyl-7-phenoxy-3-heptanol als Harz, MS: 348 $(M + H)^+$.

## Beispiel 10

In analoger Weise wie in Beispiel 9 beschrieben, wurden die folgenden Verbindungen hergestellt:
- Aus 100 mg (0.37 mMol) (3S,5S,6S)-6-Amino-7-cyclohexyl-5-hydroxy-3-isopropyl-1-heptanol durch Umsetzen mit 160 mg (0.4 mMol) Boc-Phe-His-OH 56 mg (27%) t-Butyl [(S)-α-[[(S)-1-[[(1S,2S,4S)-1-(cyclohexylmethyl)-2,6-dihydroxy-4-isopropylhexyl]carbamoyl]-2-imidazol-4-yläthyl]carbamoyl]phenäthyl]-carbamat als amorpher Festkörper, MS: 656 $(M + H)^+$;
- aus 109 mg (0.4 mMol) (3S,5S,6S)-6-Amino-7-cyclohexyl-5-hydroxy-3-isopropylheptylpropionat durch Umsetzen mit 200 mg (0.5 mMol) Boc-Phe-His-OH 94 mg (33%) t-Butyl [(S)-α-[[(S)-1-[[(1S,2S,4S)-1-(cyclohexylmethyl)-2-hydroxy-4-isopropyl-6-(propionyloxy)hexyl]carbamoyl]-2-imidazol-4-yläthyl]carbamoyl]-phenäthyl]carbamat als Harz, MS: 712 $(M + H)^+$ und
- aus 90 mg (0.248 mMol) (2S,3S,5S)-2-Amino-7-(benzyloxy)-1-cyclohexyl-5-isopropyl-3-heptanol durch Umsetzen mit 102 mg (0.248 mMol) Boc-Phe-His-OH 50 mg (27%) t-Butyl [(S)-α-[[(RS)-1-[[(1S,2S,4S)-6-(benzyloxy)-1-(cyclohexylmethyl)-2-hydroxy-4-isopropylhexyl]carbamoyl]-2-imidazol-4-yläthyl]carbamoyl]-phenäthyl]carbamat als Harz, MS: 746 $(M + H)^+$.

Die als Ausgangsstoffe eingesetzten Amine wurden wie folgt hergestellt:

### (3S,5S,6S)-6-Amino-7-cyclohexyl-5-hydroxy-3-isopropyl-1-heptanol

1.1 g (2.67 mMol) t-Butyl 4-(cyclohexylmethyl)-5-(4-hydroxy-2-isopropylbutyl)-2,2-dimethyl-3-oxazolidincarboxylat werden in 1.85 N Salzsäure in Dioxan gelöst und 2 Stunden bei Raumtemperatur gerührt. Danach wird das Reaktionsgemisch auf 2N Kaliumbicarbonatlösung gegossen und mit Essigester extrahiert. Das nach dem Trocknen und Eindampfen der organischen Extrakte erhaltene Rohprodukt wird mit einem 4:1-Gemisch von Methylenchlorid und Methanol als Eluierungsmittel an Kieselgel chromatographiert, wobei 268 mg (37%) (3S,5S,6S)-6-Amino-7-cyclohexyl-5-hydroxy-3-isopropyl-1-heptanol als Harz erhalten werden, MS: 272 $(M + H)^+$.

### (3S,5S,6S)-6-Amino-7-cyclohexyl-5-hydroxy-3-isopropylheptylpropionat

1.0 g (2.43 mMol) t-Butyl 4-(cyclohexylmethyl)-5-(4-hydroxy-2-isopropylbutyl)-2,2-dimethyl-3-oxazolidincarboxylat in 50 ml Methylenchlorid wird mit 0.38 ml (2.7 mMol) Triäthylamin und 0.24 ml (2.7 mMol) Propionsäurechlorid versetzt und 12 Stunden bei Raumtemperatur gerührt. Nach üblicher Aufarbeitung wird das Rohprodukt durch Flash-Chromatographie mit einem 1:5-Gemisch von Aether und Petroläther an Kieselgel gereinigt, wobei 1.0 g (88%) t-Butyl (4S,5S)-4-(cyclohexylmethyl)-5-[(S)-2-isopropyl-4-(propionyloxy)butyl]-2,2-dimethyl-3-oxazolidincarboxylat als Oel erhalten wird, MS: 452 $(M-CH_3)^+$.

Aus 950 mg (2.03 mMol) t-Butyl (4S,5S)-4-(cyclohexylmethyl)-5-[(S)-2-isopropyl-4-(propionyloxy)butyl]-2,2-dimethyl-3-oxazolidincarboxylat werden durch Abspalten der Boc-Schutzgruppe mit Trifluoressigsäure in analoger Weise wie in Beispiel 9 beschrieben 392 mg (59%) (3S,5S,6S)-6-Amino-7-cyclohexyl-5-hydroxy-3-isopropylheptylpropionat als Harz erhalten, MS: 328 $(M + H)^+$.

### (2S,3S,5S)-2-Amino-7-(benzyloxy)-1-cyclohexyl-5-isopropyl-3-heptanol

Ein Gemisch von 500 mg (1.2 mMol) t-Butyl 4-(cyclohexylmethyl)-5-(4-hydroxy-2-isopropylbutyl)-2,2-dimethyl-3-oxa zolidincarboxylat in 3 ml Toluol, 191 mg (0.5 mMol) Tetrabutylammoniumjodid, 5.9 g (150 mMol) Natriumhydroxid in Wasser (8.8 ml) und 1.16 ml (9.8 mMol) Benzylbromid wird 24 Stunden bei Raumtemperatur gerührt. Danach gibt man Wasser zu, extrahiert mit Aether, trocknet die Aetherextrakte über Natriumsulfat und dampft unter vermindertem Druck zur Trockene ein. Chromatographie des Rückstands an Kieselgel mit einem 1:8-Gemisch von Aether und Petroläther liefert 457 mg (78.5%) t-Butyl (4S,5S)-5-[(S)-2-(benzyloxy)-2-isopropylbutyl]-4-(cyclohexylmethyl)-2,2-dimethyl-3-oxazolidincarboxylat als hellgelbes Harz, MS: 486 $(M-CH_3)^+$.

Aus 450 mg (0.9 mMol) t-Butyl (4S,5S)-5-[(S)-2-(benzyloxy)-2-isopropylbutyl]-4-(cyclohexylmethyl)-2,2-dimethyl-3-oxazolidincarboxylat werden durch Abspalten der Boc-Schutzgruppe mit Trifluoressigsäure in analoger Weise wie in Beispiel 9 beschrieben 100 mg (29%) (2S,3S,5S)-2-Amino-7-(benzyloxy)-1-cyclohexyl-5-isopropyl-3-heptanol als Harz erhalten, MS: 264 $(M\text{-}Cyclohexylmethyl)^+$.

## Beispiel 11

In analoger Weise wie in Beispiel 9 beschrieben, werden 635 mg (1.78 mMol) t-Butyl [(4S,5S)-4-(cyclohexylmethyl)-5-(S)-2-isopropyl-4-(pivaloyloxy)butyl]-2,2-dimethyl-3-oxazolidincarboxylat zur Abspaltung der Boc-Schutzgruppe unter gleichzeitiger Oeffnung des Oxazolidinrings mit Trifluoressigsäure/Wasser/Methylenchlorid behandelt, und die erhaltenen 347 mg (76%) (3S,5S,6S)-6-Amino-7-cyclohexyl-5-hydroxy-3-isopropylheptylpivaloat ohne weitere Reinigung mit Boc-Phe-His-OH umgesetzt, wobei 280 mg (37%) t-Butyl [(S)-α-[[(RS)-1-[[(1S,2S,4S)-1-(cyclohexylmethyl)-2-hydroxy-4-isopropyl-6-(pivaloyloxy)hexyl]carbamoyl]-2-imidazol-4-yläthyl]carbamoyl]phenäthyl]carbamat als amorpher Festkörper erhalten werden, MS: 740 $(M + H)^+$.

In analoger Weise wie oben beschrieben, wurden aus 270 mg (0.53 mMol) (S)-4-[(4S,5S)-3-(t-Butoxycarbonyl)-4-(cyclohexylmethyl)-2,2-dimethyl-5-oxazolidinyl]-[3-isopropylbutyl]butylcarbamat 80 mg (25%) [-(3S,5S,6S)-6-[[N-[N-(t-Butoxycarbonyl)-3-phenyl-L-alanyl]-DL-histidyl]amino]-7-cyclohexyl-5-hydroxy-3-isopropylheptyl]butylcarbamat als Harz erhalten, MS: 755 $(M + H)^+$.

Das als Ausgangsstoff eingesetzte t-Butyl [(4S,5S)-4-(cyclohexylmethyl)-5-(S)-2-isopropyl-4-(pivaloyloxy)butyl]-2,2-dimethyl-3-oxazolidincarboxylat wurde wie folgt hergestellt:

630 mg (1.53 mMol) t-Butyl 4-(cyclohexylmethyl)-5-(4-hydroxy-2-isopropylbutyl)-2,2-dimethyl-3-oxazolidincarboxylat in 20 ml Methylenchlorid werden mit 0.234 ml (1.7 mMol) Triäthylamin und 0.206 ml (1.7 mMol) Pivaloylchlorid versetzt und anschliessend 12 Stunden bei Raumtemperatur gerührt. Das nach üblicher Aufarbeitung erhaltene Rohprodukt wird durch Flash-Chromatographie an Kieselgel mit einem 1:5-Gemisch von Aether und Petroläther als Eluierungsmittel gereinigt, wobei man 635 mg (83%) t-Butyl [-(4S,5S)-4-(cyclohexylmethyl)-5-(S)-2-isopropyl-4-(pivaloyloxy)butyl]-2,2-dimethyl-3-oxazolidincarboxylat als Harz erhält, MS: 480 $(M-CH_3)^+$.

Das als Ausgangsstoff eingesetzte (S)-4-[(4S,5S)-3-(t-Butoxycarbonyl)-4-(cyclohexylmethyl)-2,2-dimethyl-5-oxazolidinyl]-[3-isopropylbutyl]butylcarbamat wurde wie folgt hergestellt:

411 mg (1.0 mMol) t-Butyl 4-(cyclohexylmethyl)-5-(4-hydroxy-2-isopropylbutyl)-2,2-dimethyl-3-oxazolidincarboxylat in 5 ml Aether werden mit einer katalytischen Menge Triäthylamin (ca. 0.1 mMol) und 0.22 ml (2.0 mMol) Butylisocyanat in 5 ml Tetrahydrofuran versetzt und anschliessend 72 Stunden zum Rückfluss erhitzt. Danach wird die Reaktionslösung unter vermindertem Druck eingedampft, und der Rückstand zur Reinigung an Kieselgel mit einem 1:1-Gemisch von Aether und Petroläther flash-chromatographiert, wobei 275 mg (54%) (S)-4-[(4S,5S)-3-(t-Butoxycarbonyl)-4-(cyclohexylmethyl)-2,2-dimethyl-5-oxazolidinyl]-[3-isopropylbutyl]butylcarbamat als Harz erhalten werden, MS: 495 $(M-CH_3)^+$.

## Beispiel 12

Aus 180 mg (0.245 mMol) t-Butyl [(S)-α-[[1-[[(1S,2S,4S)-1-(cyclohexylmethyl)-2-hydroxy-4-isopropyl-6-phenoxyhexyl]carbamoyl]-2-imidazol-4-yläthyl]carbamoyl]phenäthyl]carbamat wird in Analogie zu Beispiel 10 mit 1.8 N Salzsäure in Dioxan die Boc-Schutzgruppe abgespalten. Das so erhaltene freie Amin wird in 5 ml Acetonitril gelöst und nacheinander mit 41 µl (0.2 mMol) Hünigbase, 88 mg (0.2 mMol) BOP und 43 mg (0.2 mMol) Boc-D-Pro-OH versetzt und anschliessend 20 Stunden bei Raumtemperatur gerührt. Das nach üblicher Aufarbeitung erhaltene Rohprodukt wird mit einem 10:1-Gemisch von Methylenchlorid und Methanol als Eluierungsmittel an Kieselgel chromatographiert, wobei 107 mg (64%) t-Butyl (R)-[[(S)-1-[[1-[[(1S,2S,4S)-1-(cyclohexylmethyl)-2-hydroxy-4-isopropyl-6-phenoxyhexyl]carbamoyl]-2-imidazol-4-yläthyl]-carbamoyl]phenäthyl]carbamoyl]-1-pyrrolidincarboxylat als amorpher Festkörper erhalten werden, MS: 829 $(M + H)^+$.

In analoger Weise wie oben beschrieben, wurden aus 240 mg (0.324 mMol) t-Butyl [(S)-α-[[(RS)-1-[[(1S,2S,4S)-1-(cyclohexylmethyl)-2-hydroxy-4-isopropyl-6-(pivaloyloxy)hexyl]carbamoyl]-2-imidazol-4-yläthyl]carbamoyl]phenäthyl]carbamat 124 mg (50%) t-Butyl (R)-2-[[(S)-α-[[(R und S)-1-[[(1S,2S,4S)-1-(cyclohexylmethyl]-2-hydroxy-4-isopropyl-6-(pivaloyloxy)hexyl]carbamoyl]-2-imidazol-4-yläthyl]carbamoyl]-phenäthyl]carbamoyl]-1-pyrrolidincarboxylat als amorpher Festkörper erhalten, MS: 740 $(M + H)^+$.

## Beispiel 13

In analoger Weise wie in Beispiel 6 beschrieben, wurden durch Abspalten der Boc-Schutzgruppe am Imidazolring mit Kaliumcarbonat in Methanol die folgenden Verbindungen hergestellt:

- Aus (S)-N-[(1S,2S,4S,E)-6-(Butylcarbamoyl)-1-(cyclohexylmethyl)-2-hydroxy-4-isopropyl-5-hexenyl]-α-[(R)-α-(3,3-dimethyl-2-oxobutyl)hydrocinnamamido]-3-t-butoxycarbonylimidazol-4-propionamid das (S)-N-[(1S,2S,4S,E)-6-(Butylcarbamoyl)-1-(cyclohexylmethyl)-2-hydroxy-4-isopropyl-5-hexenyl]-α-[(R)-α-(3,3-dimethyl-2-oxobutyl)hydrocinnamamido]imidazol-4-propionamid als gelblicher amorpher Festkörper, MS: 720 $(M + H)^+$;
- aus (S)-N-[(1S,2S,4S,E)-1-(Cyclohexylmethyl)-2-hydroxy-4-isopropyl-6-[(2-pyridylmethyl)carbamoyl]-5-hexenyl]-α-[(R)-α-(3,3-dimethyl-2-oxobutyl)hydrocinnamamido]-3-t-butoxycarbonylimidazol-4-propionamid das (S)-N-[(1S,2S,4S,E)-1-(Cyclohexylmethyl)-2-hydroxy-4-isopropyl-6-[(2-pyridylmethyl)carbamoyl]-5-hexenyl]-α-[(R)-α-(3,3-dimethyl-2-oxobutyl)hydrocinnamamido]imidazol-4-propionamid als weisser amorpher Festkörper, MS: 755 $(M + H)^+$;
- aus (S)-N-[(1S,2S,4S)-1-(Cyclohexylmethyl)-2-hydroxy-4-isopropyl-6-methyl-5-heptenyl]-α-[(R)-α-(3,3-dimethyl-2-oxobutyl)hydrocinnamamido]-3-t-butoxycarbonylimidazol-4-propionamid das (S)-N-(1S,2S,4S)-1-(Cyclohexylmethyl)-2-hydroxy-4-isopropyl-6-methyl-5-heptenyl]-α-[(R)-α-(3,3-dimethyl-2-oxobutyl)-hydrocinnamamido]imidazol-4-propionamid als amorpher Festkörper, MS: 649 $(M + H)^+$;
- aus (S)-N-[(1S,2S,4S,E)-1-(Cyclohexylmethyl)-2-hydroxy-4-isopropyl-6-(2-pyridyl)-5-hexenyl]-α-[(R)-α-(3,3-dimethyl-2-oxobutyl)hydrocinnamamido]-3-t-butoxycarbonylimidazol-4-propionamid das (S)-N-[(1S,2S,4S,E)-1-(Cyclohexylmethyl)-2-hydroxy-4-isopropyl-6-(2-pyridyl)-5-hexenyl]-α-[(R)-α-3,3-dimethyl-2-oxobutyl]-hydrocinnamamido]imidazol-4-propionamid als weisser amorpher Festkörper, MS: 698 $(M + H)^+$ und
- aus (S)-N-[(1S,2S,4S,E)-1-(Cyclohexylmethyl)-2-hydroxy-4-isopropyl-6-phenyl-5-hexenyl]-α-[(R)-α-(3,3-dimethyl-2-oxobutyl)hydrocinnamamido]-3-t-butoxycarbonylimidazol-4-propionamid das (S)-N-[(1S,2S,4S,E)-1-(Cyclohexylmethyl)-2-hydroxy-4-isopropyl-6-phenyl-5-hexenyl]-α-[(R)-α-(3,3-dimethyl-2-oxobutyl]-hydrocinnamamido]imidazol-4-propionamid als weisser amorpher Festkörper, MS: 697 $(M + H)^+$.

Die als Ausgangsstoffe eingesetzten Propionamide wurden wie folgt hergestellt:

(S)-N-[(1S,2S,4S,E)-6-(Butylcarbamoyl)-1-(cyclohexylmethyl)-2-hydroxy-4-isopropyl-5-hexenyl]-α-[(R)-α-(3,3-dimethyl-2-oxobutyl)hydrocinnamamido]-3-t-butoxycarbonylimidazol-4-propionamid

0.5 g (2 mMol) Osmiumtetroxyd werden mit 25 ml destilliertem Wasser überschichtet und danach mit 9.6 g (82 mMol) N-Methylmorpholin-N-oxid versetzt. Dann werden zu diesem Gemisch 25 ml Wasser und 50 ml Aceton gegeben, und das Reaktionsgemisch auf 5° abgekühlt. Unter Rühren und Kühlen werden dann 20.0 g (50.8 mMol) t-Butyl (4S,5S)-4-(cyclohexylmethyl)-5-[(S)-2-isopropyl-3-butenyl]-3-oxazolidincarboxylat in 70 ml Aceton innerhalb von 40 Minuten zugetropft. Nach beendeter Zugabe wird das Kühlbad entfernt, und das Reaktionsgemisch 20 Stunden weiter gerührt. Danach wird eine Suspension von 2.5 g (13 mMol) Natriumpyrosulfit und 16.6 g Florisil in 80 ml Wasser zugegeben, und das Gemisch weitere 2 Stunden bei Raumtemperatur intensiv gerührt. Anschliessend wird das Reaktionsgemisch durch Dicalit filtriert, und das Filtrat mit wässriger Kaliumbisulfatlösung auf pH 7 neutralisiert. Nach dem Abdampfen des organischen Lösungsmittels wird die wässrige Phase mit Essigester extrahiert, und die Essigesterextrakte über Natriumsulfat getrocknet und eingedampft. Der verbleibende Rückstand wird an 300 g Kieselgel mit einem Gemisch von Methylenchlorid und Isopropanol als Eluierungsmittel chromatographiert, wobei man 19.5 g t-Butyl (4S,5S)-4-(cyclohexylmethyl)-5-[(2S,3RS)-3,4-dihydroxy-2-isopropylbutyl]-2,2-dimethyl-3-oxazolidincarboxylat als Oel erhält, welches ohne weitere Reinigung in die nächste Stufe eingesetzt wird.

20.0 g (46.8 mMol) t-Butyl (4S,5S)-4-(cyclohexylmethyl)-5-[(2S,3RS)-3,4-dihydroxy-2-isopropylbutyl]-2,2-dimethyl-3-oxazolidincarboxylat werden in 250 ml Benzol gelöst, auf 5° abgekühlt und innerhalb von 10 Minuten portionsweise mit 20.8 g (47 mMol) Bleitetraacetat versetzt. Nach 4-stündigem Rühren bei 5° werden 500 ml Aether zugegeben, und das Reaktionsgemisch weitere 15 Minuten gerührt. Danach wird das Reaktionsgemisch filtriert, und das Filtrat unter vermindertem Druck eingedampft. Chromatographie des Rückstands an 300 g Kieselgel mit Methylenchlorid als Eluierungsmittel liefert 15.9 g t-Butyl (4S,5S)-4-(cyclohexylmethyl)-5-[(S)-2-formyl-3-methylbutyl]-2,2-dimethyl-3-oxazolidincarboxylat als Oel, MS: 380 $(M-CH_3)^+$.

330 mg Natriumhydrid-Suspension (55% in Mineralöl) werden mit Hexan ölfrei gewaschen und anschliessend mit 15 ml Tetrahydrofuran überschichtet. Die Suspension wird danach auf 0° abgekühlt und tropfenweise mit einer Lösung von 2.25 ml (11 mMol) Phosphonoessigsäuretriäthylester in 5 ml Tetrahydrofuran versetzt. Nach beendeter Zugabe wird das Reaktionsgemisch 30 Minuten bei Raumtemperatur gerührt, dann erneut auf 0° abgekühlt und mit 1.50 g (3.8 mMol) t-Butyl (4S,5S)-4-(cyclohexylmethyl)-5-[-(S)-2-formyl-3-methylbutyl]-2,2-dimethyl-3-oxazolidincarboxylat in 10 ml Tetrahydrofuran innerhalb von 10 Minuten tropfenweise versetzt. Nach beendeter Zugabe wird das Reaktionsgemisch 0,5 Stunden bei 0° und anschliessend 2 Stunden bei Raumtemperatur gerührt. Danach wird das Gemisch auf Eiswasser gegossen und mit Essigester extrahiert. Die Essigesterextrakte werden über Magnesiumsulfat getrocknet und unter vermindertem Druck eingedampft. Chromatographie des Rückstands an 30 g Kieselgel mit einem Gemisch von Methylenchlorid und Isopropanol als Eluierungsmittel liefert 1.7 g (S)-5-[(4S,5S)-3-(t-Butoxycarbonyl)-4-(cyclohexylmethyl)-2,2-dimethyl-5-oxazolidinyl]-4-isopropyl-2-pentensäureäthylester, welcher ohne weitere Reinigung in die nächste Stufe eingesetzt wird.

1.7 g (3.7 mMol) (S)-5-[(4S,5S)-3-(t-Butoxycarbonyl)-4-(cyclohexylmethyl)-2,2-dimethyl-5-oxazolidinyl]-4-isopropyl-2-pentensäureäthylester werden in 10 ml Methanol gelöst, mit 2 ml (18 mMol) 28%iger Natronlauge versetzt und 16 Stunden bei Raumtemperatur gerührt. Danach wird das Reaktionsgemisch auf Eiswasser gegossen, der pH-Wert mit Essigsäure auf 5 eingestellt, und das Gemisch mit Essigester extrahiert. Die Essigesterextrakte werden über Magnesiumsulfat getrocknet und unter vermindertem Druck eingedampft. Chromatographie des Rückstands an 20 g Kieselgel mit einem Gemisch von Methylenchlorid und Isopropanol als Eluierungsmittel liefert 1.2 g (S)-5-[(4S,5S)-3-(t-Butoxycarbonyl)-4-(cyclohexylmethyl)-2,2-dimethyl-5-oxazolidinyl]-4-isopropyl-2-pentensäure als Oel, MS: 422 $(M-CH_3)^+$.

Ein Gemisch von 0.55 g (1.26 mMol) (S)-5-[(4S,5S)-3-(t-Butoxycarbonyl)-4-(cyclohexylmethyl)-2,2-dimethyl-5-oxazolidinyl]-4-isopropyl-2-pentensäure, 0.22 g (1.6 mMol) HOBT, 0.14 ml (1.4 mMol) Butylamin und 8 ml Methylenchlorid wird auf 0° gekühlt und tropfenweise mit einer Lösung von 0.29 g (1.5 mMol) EDC in 2 ml Dimethylformamid versetzt. Danach wird das Reaktionsgemisch 1 Stunde bei 0° und anschliessend 16 Stunden bei Raumtemperatur gerührt und dann auf Eiswasser gegossen und mit Methylenchlorid extrahiert. Die Methylenchloridextrakte werden über Kaliumcarbonat getrocknet und unter vermindertem Druck eingedampft. Chromatographie des erhaltenen Rückstands an 10 g Kieselgel mit einem Gemisch von Methylenchlorid und Isopropanol als Eluierungsmittel liefert 0.53 g t-Butyl (4S,5S)-5-[-(S,E)-4-(butylcarbamoyl)-2-isopropyl-3-butenyl]-4-(cyclohexylmethyl)-2,2-dimethyl-3-oxazolidincarboxylat als hellgelbes Oel, welches ohne weitere Reinigung direkt in die nächste Stufe eingesetzt wird.

0.53 g (1.07 mMol) t-Butyl (4S,5S)-5-[(S,E)-4-(butylcarbamoyl)-2-isopropyl-3-butenyl]-4-(cyclohexylmethyl)-2,2-dimethyl-3-oxazolidincarboxylat wird in 18.2 ml Methanol gelöst und bei 10° mit 6.4 ml 3.88 M Salzsäure in Methanol versetzt. Das Reaktionsgemisch wird anschliessend 5 Stunden bei Raumtemperatur gerührt und danach eingedampft. Der Rückstand wird in Methylenchlorid gelöst, und die Methylenchloridlösung mit 2N Natriumbicarbonatlösung gewaschen, über Kaliumcarbonat getrocknet und unter vermindertem Druck eingedampft. Auf diese Weise erhält man 0.314 g (E,4S,6S,7S)-7-Amino-N-butyl-8-cyclohexyl-6-hydroxy-4-isopropyl-2-octenamid als Oel, welches ohne weitere Reinigung in die nächste Stufe eingesetzt wird.

In analoger Weise wie in Beispiel 6 beschrieben, wird durch Umsetzen von (E,4S,6S,7S)-7-Amino-N-butyl-8-cyclohexyl-6-hydroxy-4-isopropyl-2-octenamid mit 1-(t-Butoxycarbonyl)-N-[(R)-α-(3,3-dimethyl-2-oxobutyl)hydrocinnamoyl]-L-histidin das (S)-N-[(1S,2S,4S,E)-6-(Butylcarbamoyl)-1-(cyclohexylmethyl)-2-hydroxy-4-isopropyl-5-hexenyl]-α-[(R)-α-(3,3-dimethyl-2-oxobutyl)hydrocinnamamido]-3-t-butoxycarbonylimidazol-4-propionamid erhalten, welches ohne weitere Reinigung direkt in die nächste Stufe eingesetzt wird.

(S)-N-[(1S,2S,4S,E)-1-(Cyclohexylmethyl)-2-hydroxy-4-isopropyl-6-[(2-pyridylmethyl)carbamoyl]-5-hexenyl]-α-[(R)-α-(3,3-dimethyl-2-oxobutyl)hydrocinnamamido]-3-t-butoxycarbonylimidazol-4-propionamid

Diese Verbindung wurde in analoger Weise wie oben beschrieben durch Umsetzen von (S)-5-[(4S,5S)-3-(t-Butoxycarbonyl)-4-(cyclohexylmethyl)-2,2-dimethyl-5-oxazolidinyl]-4-isopropyl-2-pentensäure mit 2-Pyridylmethylamin, anschliessendem Abspalten der Boc-Schutzgruppe unter gleichzeitiger Oeffnung des Oxazolidinrings mit Salzsäure in Methanol und Umsetzen mit 1-(t-Butoxycarbonyl)-N-[(R)-α-(3,3-dimethyl-2-oxobutyl)hydrocinnamoyl]-L-histidin hergestellt.

(S)-N-[(1S,2S,4S)-1-(Cyclohexylmethyl)-2-hydroxy-4-isopropyl-6-methyl-5-heptenyl]-α-[(R)-α-(3,3-dimethyl-2-oxobutyl)hydrocinnamamido]-3-t-butoxycarbonylimidazol-4-propionamid

2.60 g (6 mMol) Isopropyltriphenylphosphoniumjodid werden in 20 ml Tetrahydrofuran suspendiert, auf -10° abgekühlt und unter Rühren mit 3.5 ml (5.6 mMol) 1.6 M Butyllithiumlösung in Hexan versetzt. Danach wird das Reaktionsgemisch 1 Stunde bei Raumtemperatur gerührt, dann auf -65° abgekühlt und mit einer Lösung von 1.58 g (4.0 mMol) t-Butyl (4S,5S)-4-(cyclohexylmethyl)-5-[(S)-2-formyl-3-methylbutyl]-2,2-dimethyl-3-oxazolidincarboxylat in 10 ml Tetrahydrofuran tropfenweise versetzt. Danach wird das Reaktionsgemisch ohne Kühlung 2 Stunden gerührt und anschliessend auf Eiswasser gegossen und mit Methylenchlorid extrahiert. Die Methylenchloridextrakte werden über Magnesiumsulfat getrocknet und eingedampft. Chromatographie des Rückstands an 20 g Kieselgel mit Methylenchlorid als Eluierungsmittel liefert 1.02 g t-Butyl (4S,5S,)-4-(cyclohexylmethyl)-5-[(S)-2-isopropyl-4-methyl-3-pentenyl]-2,2-dimethyl-3-oxazolidincarboxylat als Oel, MS: 421 $(M + H)^+$.

1.0 g (2.37 mMol) t-Butyl (4S,5S,)-4-(cyclohexylmethyl)-5-[(S)-2-isopropyl-4-methyl-3-pentenyl]-2,2-dimethyl-3-oxazolidincarboxylat wird in 40 ml Methanol gelöst und mit 12.9 ml (50 mMol) 3.9M Salzsäure in Methanol versetzt. Danach wird das Reaktionsgemisch 3,5 Stunden bei Raumtemperatur gerührt und anschliessend unter vermindertem Druck eingedampft. Der Rückstand wird in Methylenchlorid gelöst, und die Methylenchloridlösung mit 2N Natriumbicarbonatlösung gewaschen, über Kaliumcarbonat getrocknet und unter vermindertem Druck eingedampft. Chromatographie des Rückstands an 10 g Kieselgel mit einem Gemisch von Methylenchlorid und Isopropanol als Eluierungsmittel liefert 0.377 g (αS,βS)-β-Amino-α-[(S)-2-isopropyl-4-methyl-3-pentenyl]cyclohexylpropanol als Oel, MS: 282 $(M + H)^+$.

In analoger Weise wie in Beispiel 6 beschrieben, wird (αS,βS)-β-Amino-α-[(S)-2-isopropyl-4-methyl-3-pentenyl]cyclohexylpropanol mit 1-(t-Butoxycarbonyl)-N-[(R)-α-(3,3-dimethyl-2-oxobutyl)hydrocinnamoyl]-L-histidin zu (S)-N-[(1S,2S,4S)-1-(Cyclohexylmethyl)-2-hydroxy-4-isopropyl-6-methyl-5-heptenyl]-α-[(R)-α-(3,3-dimethyl-2-oxobutyl)hydrocinnamamido]-3-t-butoxycarbonylimidazol-4-propionamid umgesetzt, welches direkt in die nächste Stufe eingesetzt wird.

(S)-N-[(1S,2S,4S,E)-1-(Cyclohexylmethyl)-2-hydroxy-4-isopropyl-6-(2-pyridyl)-5-hexenyl]-α-[(R)-α-(3,3-dimethyl-2-oxobutyl)hydrocinnamamido]-3-t-butoxycarbonylimidazol-4-propionamid

Diese Verbindung wurde in analoger Weise wie oben beschrieben durch Umsetzen von t-Butyl (4S,5S)-4-(cyclohexylmethyl)-5-[(S)-2-formyl-3-methylbutyl]-2,2-dimethyl-3-oxazolidincarboxylat mit 2-Pyridylmethyltriphenylphosphoniumjodid, Abspalten der Boc-Schutzgruppe unter gleichzeitiger Oeffnung des Oxazolidinrings mit Salzsäure in Methanol und Umsetzen des erhaltenen (2S,3S,5S)-2-Amino-1-cyclohexyl-5-isopropyl-7-(2-pyridyl)-6-hepten-3-ols mit 1-(t-Butoxycarbonyl)-N-[(R)-α-(3,3-dimethyl-2-oxobutyl)-hydrocinnamoyl]-L-histidin hergestellt.

(S)-N-[(1S,2S,4S,E)-1-(Cyclohexylmethyl)-2-hydroxy-4-isopropyl-6-phenyl-5-hexenyl]-α-[(R)-α-(3,3-dimethyl-2-oxobutyl)hydrocinnamamido]-3-t-butoxycarbonylimidazol-4-propionamid

Auch diese Verbindung wurde, in analoger Weise wie oben beschrieben, durch Umsetzen von t-Butyl (4S,5S)-4-(cyclohexylmethyl)-5-[(S)-2-formyl-3-methylbutyl]-2,2-dimethyl-3-oxazolidincarboxylat mit Benzyltriphenylphosphoniumjodid, Abspalten der Boc-Schutzgruppe unter gleichzeitiger Oeffnung des Oxazolidinrings mit Salzsäure in Methanol und Umsetzen des erhaltenen (αS,βS)-β-Amino-α-[(S,E)-2-isopropyl-4-phenyl-3-butenyl]cyclohexylpropanols mit 1-(t-Butoxycarbonyl)-N-[(R)-α-(3,3-dimethyl-2-oxobutyl)-hydrocinnamoyl]-L-histidin hergestellt.

Beispiel 14

150 mg (0.22 mMol) (S)-N-[(1S,2S,4S,E)-1-(Cyclohexylmethyl)-2-hydroxy-4-isopropyl-6-(2-pyridyl)-5-hexenyl]-α-[(R)-α-3,3-dimethyl-2-oxobutyl]hydrocinnamamido]imidazol-4-propionamid werden in 5 ml Methanol gelöst, mit 0.15 ml (0.6 mMol) 3.9M Salzsäure in Methanol und 80 mg Palladium auf Kohle (10%ig) versetzt und 6 Stunden bei Raumtemperatur und Atmosphärendruck hydriert. Nach beendeter Wasserstoffaufnahme wird der Katalysator abfiltriert, und das Filtrat unter vermindertem Druck eingedampft. Dabei werden 150 mg (S)-N-[(1S,2S,4S)-1-(Cyclohexylmethyl)-2-hydroxy-4-isopropyl-6-(2-pyridyl)hexyl]-α-[(R)-α-(3,3-dimethyl-2-oxobutyl)hydrocinnamamido]imidazol-4-propionamid-dihydrochlorid als amorpher Festkörper erhalten, MS: 700 $(M + H)^+$.

In analoger Weise wie oben beschrieben wurden die folgenden Verbindungen hergestellt:

- Aus (S)-N-[(1S,2S,4S,E)-1-(Cyclohexylmethyl)-2-hydroxy-4-isopropyl-6-phenyl-5-hexenyl]-α-[(R)-α-(3,3-dimethyl-2-oxobutyl]hydrocinnamamido]imidazol-4-propionamid das (S)-N-[(1S,2S,4S)-1-(Cyclohexylmethyl)-2-hydroxy-4-isopropyl-6-phenylhexyl]-α-[(R)-α-(3,3-dimethyl-2-oxobutyl)hydrocinnamamido]imidazol-4-propionamid-hydrochlorid als amorpher Festkörper, MS: 699 $(M + H)^+$;
- aus (S)-N-[(1S,2S,4S,E)-6-(Butylcarbamoyl)-1-(cyclohexylmethyl)-2-hydroxy-4-isopropyl-5-hexenyl]-α-[(R)-α-(3,3-dimethyl-2-oxobutyl)hydrocinnamamido]imidazol-4-propionamid das (S)-N-[(1S,2S,4S)-6-(Butylcarbamoyl)-1-(cyclohexylmethyl)-2-hydroxy-4-isopropylhexyl]-α-[(R)-α-(3,3-dimethyl-2-oxobutyl)-hydrocinnamamido]imidazol-4-propionamid-hydrochlorid als amorpher Festkörper, MS: 722 $(M + H)^+$ und
- aus (S)-N-[(1S,2S,4S,E)-1-(Cyclohexylmethyl)-2-hydroxy-4-isopropyl-6-[(2-pyridylmethyl)carbamoyl]-5-hexenyl]-α-[(R)-α-(3,3-dimethyl-2-oxobutyl)hydrocinnamamido]imidazol-4-propionamid das (S)-N-[-(1S,2S,4S)-1-(Cyclohexylmethyl)-2-hydroxy-4-isopropyl-6-[(2-pyridyl)carbamoyl]hexyl]-α-[(R)-α-(3,3-dimethyl-2-oxobutyl)hydrocinnamamido]imidazol-4-propionamid-dihydrochlorid als amorpher Festkörper, MS: 757 $(M + H)^+$.

## Beispiel 15

In analoger Weise wie in Beispiel 6 beschrieben, wurden durch Abspaltung der Boc-Schutzgruppe am Imidazolring mit Kaliumcarbonat in Methanol die folgenden Verbindungen hergestellt:

- Aus t-Butyl [(S)-α-[[(S)-1-[[(1S,2S,4S)-6-[1-(benzyloxy)formamido]-1-(cyclohexylmethyl)-2-hydroxy-4-isopropylhexyl]carbamoyl]-2-(3-t-butoxycarbonyl)imidazol-4-yläthyl]carbamoyl]phenäthyl]carbamat das t-Butyl [(S)-α-[[(S)-1-[[(1S,2S,4S)-6-[1-(benzyloxy)formamido]-1-(cyclohexylmethyl)-2-hydroxy-4-isopropylhexyl]-carbamoyl]-2-imidazol-4-yläthyl]carbamoyl]phenäthyl]carbamat als amorpher Festkörper, MS: 789 $(M + H)^+$ und
- aus t-Butyl [(S)-α-[[(S)-1-[[(1S,2S,4S)-6-(3-benzyl-2-oxo-1-imidazolinyl)hexyl]carbamoyl]-2-(3-t-butoxycarbonyl)imidazol-4-yläthyl]carbamoyl]phenäthyl]carbamat das t-Butyl [(S)-α-[[(S)-1-[[(1S,2S,4S)-6-(3-benzyl-2-oxo-1-imidazolinyl)hexyl]carbamoyl]-2-imidazol-4-yläthyl]carbamoyl]phenäthyl]carbamat als amorpher Festkörper, MS:862 $(M + H)^+$.

Die als Ausgangsstoffe eingesetzten Carbamate wurden wie folgt hergestellt:

### t-Butyl [(S)-α-[[(S)-1-[[(1S,2S,4S)-6-[1-(benzyloxy)formamido]-1-(cyclohexylmethyl)-2-hydroxy-4-isopropylhexyl]carbamoyl]-2-(3-t-butoxycarbonyl)imidazol-4-yläthyl]carbamoyl]phenäthyl]carbamat

5.0 g (12.15 mMol) t-Butyl 4-(cyclohexylmethyl)-5-(4-hydroxy-2-isopropylbutyl)-2,2-dimethyl-3-oxazolidincarboxylat, 4.68 g (17.8 mMol) Triphenylphosphin und 2.48 g (17 mMol) Phthalimid werden in 70 ml Tetrahydrofuran gelöst und auf 10° abgekühlt. Dann wird innerhalb von 10 Minuten eine Lösung von 2.8 ml (17.8 mMol) Azodicarbonsäurediäthylester in 15 ml Tetrahydrofuran zugetropft, und das Reaktionsgemisch 4 Stunden bei Raumtemperatur gerührt. Dann wird das Reaktionsgemisch unter vermindertem Druck eingedampft, und der Rückstand an 160 g Kieselgel mit einem Gemisch von Methylenchlorid und Hexan als Eluierungsmittel chromatographiert, wobei man 5.4 g t-Butyl (4S,5S)-4-(cyclohexylmethyl)-5-[(S)-2-isopropyl-4-phthalimidobutyl]-2,2-dimethyl-3-oxazolidincarboxylat als Oel erhält, MS: 525 $(M-CH_3)^+$.

5.4 g (9.99 mMol) t-Butyl (4S,5S)-4-(cyclohexylmethyl)-5-[(S)-2-isopropyl-4-phthalimidobutyl]-2,2-dimethyl-3-oxazolidincarboxylat werden in 200 ml Aethanol gelöst, mit 2.0 ml (41 mMol) Hydrazinhydrat versetzt und 2,5 Stunden zum Rückfluss erhitzt. Anschliessend wird das Reaktionsgemisch unter vermindertem Druck eingedampft, und der Rückstand in Aether aufgeschlämmt und filtriert. Eindampfen des Filtrates

liefert 3.82 g t-Butyl (4S,5S)-5-[(S)-4-amino-2-isopropyl]-4-(cyclohexylmethyl)-2,2-dimethyl-3-oxazolidincarboxylat als Oel, MS: 395 $(M-CH_3)^+$.

3.72 g (9.06 mMol) t-Butyl (4S,5S)-5-[(S)-4-amino-2-isopropyl]-4-(cyclohexylmethyl)-2,2-dimethyl-3-oxazolidincarboxylat werden in 20 ml Methylenchlorid gelöst, mit 1.5 ml (10.7 mMol) Triäthylamin versetzt und auf 0° abgekühlt. Danach werden bei dieser Temperatur 2.49 g (10.7 mMol) N-(Benzyloxycarbonyloxy)-succinimid in Portionen innerhalb von 5 Minuten zugegeben. Das Reaktionsgemisch wird anschliessend 2 Stunden bei Raumtemperatur gerührt, danach unter vermindertem Druck eingedampft, mit 70 ml Aether und 70 ml 1N Natronlauge versetzt und 1 Stunde bei Raumtemperatur gerührt. Anschliessend wird das Reaktionsgemisch mit Aether extrahiert, und die Aetherextrakte über Magnesiumsulfat getrocknet und eingedampft. Chromatographie des erhaltenen Rückstands an 50 g Kieselgel mit einem Gemisch von Methylenchlorid und Isopropanol als Eluierungsmittel liefert 4.23 g t-Butyl (4S,5S)-5-[(S)-4-[1-(benzyloxy)-formamido]-2-isopropylbutyl]-4-(cyclohexylmethyl)-2,2-dimethyl-3-oxazolidincarboxylat als Oel, MS: 529 $(M-CH_3)^+$.

Abspalten der Boc-Schutzgruppe unter gleichzeitiger Oeffnung des Oxazolidinrings mit Salzsäure in Methanol und Umsetzen mit Boc-Phe-His(Boc)-OH liefert t-Butyl [(S)-α-[[(S)-1-[[(1S,2S,4S)-6-[1-(benzyloxy)-formamido]-1-(cyclohexylmethyl)-2-hydroxy-4-isopropylhexyl]carbamoyl]-2-(3-t-butoxycarbonyl)imidazol-4-yläthyl]carbamoyl]phenäthyl]carbamat, das ohne weitere Reinigung in die nächste Stufe eingesetzt wird.

t-Butyl <u>[(S)-α-[[(S)-1-[[(1S,2S,4S)-6-(3-benzyl-2-oxo-1-imidazolidinyl)hexyl]carbamoyl]-2-(3-t-butoxycarbonyl)-imidazol-4-yläthyl]carbamoyl]phenäthyl]carbamat</u>

1.0 g (2.4 mMol) t-Butyl 4-(cyclohexylmethyl)-5-(4-hydroxy-2-isopropylbutyl)-2,2-dimethyl-3-oxazolidincarboxylat, 0.74 g (3.3 mMol) 1-Benzyl-2-benzimidazolidinon und 0.92 g (3.5 mMol) Triphenylphosphin werden in 17 ml Tetrahydrofuran gelöst, auf 0° abgekühlt und mit einer Lösung von 0.81 g (3.5 mMol) Azodicarbonsäuredi-t-butylester in 3 ml Tetrahydrofuran versetzt. Das Reaktionsgemisch wird anschliessend 2,5 Stunden ohne Kühlung gerührt und danach unter vermindertem Druck eingedampft. Chromatographie des Rückstands an 36 g Kieselgel mit einem Gemisch von Methylenchlorid und Isopropanol als Eluierungsmittel liefert 1.76 g eines Rohprodukts, das in 30 ml Methanol gelöst und unter Eiskühlung mit 11 ml (41.8 mMol) 3.8M Salzsäure in Methanol versetzt wird. Das so erhaltene Reaktionsgemisch wird 4 Stunden bei Raumtemperatur gerührt und anschliessend unter vermindertem Druck eingedampft, und der Rückstand auf 2N Natriumcarbonatlösung gegossen und mit Methylenchlorid extrahiert. Die Methylenchloridextrakte werden über Magnesiumsulfat getrocknet und eingedampft, und der Rückstand an 20 g Kieselgel mit einem Gemisch von Methylenchlorid, Isopropanol und konzentriertem wässrigem Ammoniak als Eluierungsmittel chromatographiert. Auf diese Weise erhält man 0.83 g 1-[(3S,5S,6S)-6-Amino-7-cyclohexyl-5-hydroxy-3-isopropylheptyl]-3-benzyl-2-benzimidazolinon als Oel, MS: 478 $(M + H)^+$.

Umsetzen von 1-[(3S,5S,6S)-6-Amino-7-cyclohexyl-5-hydroxy-3-isopropylheptyl]-3-benzyl-2-benzimidazolinon mit Boc-Phe-His(Boc)-OH liefert t-Butyl [(S)-α-[[(S)-1-[[(1S,2S,4S)-6-(3-benzyl-2-oxo-1-imidazolinyl)-hexyl]carbamoyl]-2-(3-t-butoxycarbonyl)imidazol-4-yläthyl]carbamoyl]phenäthyl]carbamat, das ohne weitere Reinigung direkt in die nächste Stufe eingesetzt wird.

## <u>Beispiel 16</u>

1.0 g (1.27 mMol) t-Butyl [(S)-α-[[(S)-1-[[(1S,2S,4S)-6-[1-(benzyloxy)formamido]-1-(cyclohexylmethyl)-2-hydroxy-4-isopropylhexyl]carbamoyl]-2-imidazol-4-yläthyl]carbamoyl]phenäthyl]carbamat und 0.146 g (1.27 mMol) Pyridinhydrochlorid werden in 15 ml Methanol gelöst und nach Zusatz von 0.10 g Palladium auf Kohle (5%ig) 2,5 Stunden bei Raumtemperatur und Atmosphärendruck hydriert. Nach beendeter Wasserstoffaufnahme wird der Katalysator abfiltriert, und das Filtrat unter vermindertem Druck eingedampft, wobei 0.87 g t-Butyl [(S)-α-[[(S)-1-[[(1S,2S,4S)-6-amino-1-(cyclohexylmethyl)-2-hydroxy-4-isopropylhexyl]-carbamoyl]-2-imidazol-4-yläthyl]carbamoyl]phenäthyl]carbamat-hydrochlorid als amorpher Festkörper erhalten wird, MS: 655 $(M + H)^+$.

## <u>Beispiel 17</u>

32 mg (0.2 mMol) Dihydrozimtsäure, 45 mg (0.2 mMol) EDC und 37 mg (0.2 mMol) HOBT werden in 5 ml Methylenchlorid gelöst, und das Reaktionsgemisch mit 0.09 ml (0.7 mMol) N-Aethylmorpholin versetzt und anschliessend auf -10° abgekühlt. Anschliessend werden bei dieser Temperatur 135 mg (0.195 mMol) t-Butyl [(S)-α-[[(S)-1-[[(1S,2S,4S)-6-amino-1-(cyclohexylmethyl)-2-hydroxy-4-isopropylhexyl]carbamoyl]-2-imidazol-4-yläthyl]carbamoyl]phenäthyl]carbamat-hydrochlorid in 2 ml Dimethylformamid zugetropft, und das Reaktionsgemisch 1 Stunde bei -10° und 24 Stunden bei Raumtemperatur gerührt. Dann wird das Reaktionsgemisch auf 2N Natriumcarbonatlösung gegossen und mit Methylenchlorid extrahiert. Die Methylenchloridextrakte werden über Magnesiumsulfat getrocknet und unter vermindertem Druck eingedampft. Chromatographie des so erhaltenen Rückstands an 5 g Kieselgel mit einem Gemisch von Methylenchlorid und Isopropanol als Eluierungsmittel liefert 93 mg t-Butyl [(S)-α-[[(S)-1-[[(1S,2S,4S)-1-(cyclohexylmethyl)-6-hydrocinnamamido-2-hydroxy-4-isopropylhexyl]carbamoyl]-2-imidazol-4-yläthyl]carbamoyl]phenäthyl]-carbamat als amorphen Festkörper, MS: 787 $(M + H)^+$.

## Beispiel 18

In analoger Weise wie in Beispiel 17 beschrieben, wurden die folgenden Verbindungen hergestellt:

- Aus t-Butyl [(S)-α-[[(S)-1-[[(1S,2S,4S)-6-amino-1-(cyclohexylmethyl)-2-hydroxy-4-isopropylhexyl]-carbamoyl]-2-imidazol-4-yläthyl]carbamoyl]phenäthyl]carbamat-hydrochlorid und 3-(4-Pyridyl)propionsäure das t-Butyl [(S)-α-[[(S)-1-[[(1S,2S,4S)-1-(cyclohexylmethyl)-2-hydroxy-4-isopropyl-6-[3-(4-pyridyl)-propionamido]hexyl]carbamoyl]-2-imidazol-4-yläthyl]carbamoyl]phenäthyl]carbamat als amorpher Festkörper, MS: 788 $(M + H)^+$;
- aus t-Butyl [(S)-α-[[(S)-1-[[(1S,2S,4S)-6-amino-1-(cyclohexylmethyl)-2-hydroxy-4-isopropylhexyl]-carbamoyl]-2-imidazol-4-yläthyl]carbamoyl]phenäthyl]carbamat-hydrochlorid und 1-Benzyl-4-piperidincarbonsäure das t-Butyl [(S)-α-[[(S)-1-[[(1S,2S,4S)-6-(1-benzyl-4-piperidincarboxamido)-1-(cyclohexylmethyl)-2-hydroxy-4-isopropylhexyl]carbamoyl]-2-imidazol-4-yläthyl]carbamoyl]phenäthyl]carbamat als amorpher Festkörper, MS: 856 $(M + H)^+$;
- aus t-Butyl [(S)-α-[[(S)-1-[[(1S,2S,4S)-6-amino-1-(cyclohexylmethyl)-2-hydroxy-4-isopropylhexyl]-carbamoyl]-2-imidazol-4-yläthyl]carbamoyl]phenäthyl]carbamat-hydrochlorid und 2-[7-Chlor-5-(2-fluorphenyl)-1,3-dihydro-2-oxo-2H-1,4-benzodiazepin-1-yl]essigsäure das t-Butyl [(S)-α-[[(S)-1-[[(1S,2S,4S)-6-[2-[7-chlor-5-(2-fluorphenyl)-2,3-dihydro-2-oxo-1H-[1,4]benzodiazepin-1-yl]acetamido]-1-(cyclohexylmethyl)-2-hydroxy-4-isopropylhexyl]carbamoyl]-2-imidazol-4-yläthyl]carbamoyl]phenäthyl]carbamat als amorpher Festkörper, MS: 983 $(M + H)^+$;
- aus t-Butyl [(S)-α-[[(S)-1-[[(1S,2S,4S)-6-amino-1-(cyclohexylmethyl)-2-hydroxy-4-isopropylhexyl]-carbamoyl]-2-imidazol-4-yläthyl]carbamoyl]phenäthyl]carbamat-hydrochlorid und 3-(4-Imidazolyl)-propionsäure das t-Butyl [(S)-α-[[(S)-1-[[(1S,2S,4S)-1-(cyclohexylmethyl)-2-hydroxy-6-[imidazol-4-propionamido]-4-isopropylhexyl]carbamoyl]-2-imidazol-4-yläthyl]carbamoyl]phenäthyl]carbamat als amorpher Festkörper, MS: 777 $(M + H)^+$ und
- aus t-Butyl [(S)-α-[[(S)-1-[[(1S,2S,4S)-6-amino-1-(cyclohexylmethyl)-2-hydroxy-4-isopropylhexyl]-carbamoyl]-2-imidazol-4-yläthyl]carbamoyl]phenäthyl)carbamat-hydrochlorid und 2-Benzimidazolylpentansäure das t-Butyl [(S)-α-[[(S)-1-[[(1S,2S,4S)-6-(2-benzimidazolvaleramido)-1-(cyclohexylmethyl)-2-hydroxy-4-isopropylhexyl]carbamoyl]-2-imidazol-4-yläthyl]carbamoyl]phenäthyl]carbamat als amorpher Festkörper, MS: 855 $(M + H)^+$.

Die als Ausgangsstoffe eingesetzten Säuren sind bekannt oder können in Analogie zur Herstellung bekannter Verbindungen hergestellt werden.

## Beispiel 19

0.100 g (0.145 mMol) t-Butyl [(S)-α-[[(S)-1-[[(1S,2S,4S)-6-amino-1-(cyclohexylmethyl)-2-hydroxy-4-isopropylhexyl]carbamoyl]-2-imidazol-4-yläthyl]carbamoyl]phenäthyl]carbamat-hydrochlorid in 1 ml Aethanol wird mit 0.05 ml (0.25 mMol) Hünigbase versetzt, auf 0° abgekühlt und mit 0.018 ml (0.16 mMol) Butylisocyanat in 1 ml Aethanol versetzt. Dann wird das Reaktionsgemisch 2 Stunden bei Raumtemperatur gerührt und anschliessend unter vermindertem Druck eingedampft. Chromatographie des Rückstands an 5 g Kieselgel mit einem Gemisch von Methylenchlorid und Isopropanol als Eluierungsmittel liefert 0.063 g t-Butyl [(S)-α-[[(S)-1-[[(1S,2S,4S)-6-(3-butylureido)-1-(cyclohexylmethyl)-2-hydroxy-4-isopropylhexyl]-

carbamoyl]-2-imidazol-4-yläthyl]carbamoyl]phenäthyl]carbamat als amorphen Festkörper, MS: 754 (M + H)$^+$.

Beispiel 20

0.136 g (0.197 mMol) t-Butyl [(S)-α-[[(S)-1-[[(1S,2S,4S)-6-amino-1-(cyclohexylmethyl)-2-hydroxy-4-isopropylhexyl]carbamoyl]-2-imidazol-4-yläthyl]carbamoyl]phenäthyl]carbamat-hydrochlorid, 0.051 g (0.2 mMol) Benzyl [(methylthio)formimidoyl]carbamat und 0.07 ml (0.34 mMol) Hünigbase in 1 ml absolutem Aethanol werden unter Argon 3 Stunden bei 70° gerührt. Danach wird das Reaktionsgemisch abgekühlt, auf 2N Kaliumcarbonatlösung gegossen und mit Methylen chlorid extrahiert. Die Methylenchloridextrakte werden über Magnesiumsulfat getrocknet und unter vermindertem Druck eingedampft. Chromatographie des erhaltenen Rückstands an 2 g Kieselgel mit einem Gemisch von Methylenchlorid und Isopropanol als Eluierungsmittel liefert 0.131 g Benzyl [N-[(3S,5S,6S)-6-[[N-[N-(t-butoxycarbonyl)-3-phenyl-L-alanyl]-L-histidyl]amino]-7-cyclohexyl-5-hydroxy-3-isopropylheptyl]amidino]carbamat als farbloses Oel, MS: 831 (M + H)$^+$.

Beispiel 21

0.110 g (0.132 mMol) Benzyl [N-[(3S,5S,6S)-6-[[N-[N-(t-butoxycarbonyl)-3-phenyl-L-alanyl]-L-histidyl]-amino]-7-cyclohexyl-5-hydroxy-3-isopropylheptyl]amidino]carbamat und 0.015 mg (0.132 mMol) Pyridin-hydrochlorid werden in 2.4 ml Methanol gelöst und in Gegenwart von 20 mg Palladium auf Kohle (5%) 3 Stunden bei Raumtemperatur und Atmosphärendruck hydriert. Nach beendeter Wasserstoffaufnahme wird der Katalysator abfiltriert, und das Filtrat unter vermindertem Druck eingedampft. Auf diese Weise erhält man 0.074 g t-Butyl [(S)-α-[[(S)-1-[[(1S,2S,4S)-1-(cyclohexylmethyl)-6-guanidino-2-hydroxy-4-isopropylhexyl]carbamoyl]-2-imidazol-4-yläthyl]carbamoyl]phenäthyl]carbamat-hydrochlorid als amorphen Festkörper, MS: 697 (M + H)$^+$.

Beispiel 22

In analoger Weise wie in Beispiel 6 beschrieben, wurden durch Abspalten der Boc-Schutzgruppe am Imidazolring die folgenden Verbindungen hergestellt:

- Aus (S)-N-[(1S,2S,4S,5S oder R)-1-(Cyclohexylmethyl)-2,5-dihydroxy-4-isopropyldecyl]-α-[(R)-α-(3,3-dimethyl-2-oxobutyl)hydrocinnamamido]-3-t-butoxycarbonylimidazol-4-propionamid das (S)-N-[(1S,2S,4S,5S oder R)-1-(Cyclohexylmethyl)-2,5-dihydroxy-4-isopro pyldecyl]-α-[(R)-α-(3,3-dimethyl-2-oxobutyl)-hydrocinnamamido]imidazol-4-propionamid als amorpher Festkörper, MS: 695 (M + H)$^+$;
- aus (S)-N-[(1S,2S,4S,5R oder S)-1-(Cyclohexylmethyl)-2,5-dihydroxy-4-isopropyldecyl]-α-[(R)-α-(3,3-dimethyl-2-oxobutyl)hydrocinnamamido]-3-t-butoxycarbonylimidazol-4-propionamid das (S)-N-[(1S,2S,4S,5R oder S)-1-(Cyclohexylmethyl)-2,5-dihydroxy-4-isopropyldecyl]-α-[(R)-α-(3,3-dimethyl-2-oxobutyl)-hydrocinnamamido]imidazol-4-propionamid als amorpher Festkörper, MS: 695 (M + H)$^+$ und
- aus (S)-N-[(1S,2S,4S)-1-(Cyclohexylmethyl)-2-hydroxy-4-isopropyl-5-oxodecyl]-α-[(R)-α-(3,3-dimethyl-2-oxobutyl)hydrocinnamamido]-3-t-butoxycarbonylimidazol-4-propionamid das (S)-N-[(1S,2S,4S)-1-(Cyclohexylmethyl)-2-hydroxy-4-isoproyl-5-oxodecyl]-α-[(R)-α-(3,3-dimethyl-2-oxobutyl)hydrocinnamamido]-imidazol-4-propionamid als amorpher Festkörper, MS: 675 ((M-$H_2O$) + H)$^+$.

Die als Ausgangsstoffe eingesetzten Propionamide wurden wie folgt hergestellt:

(S)-N-[(1S,2S,4S,5S oder R)-1-(Cyclohexylmethyl)-2,5-dihydroxy-4-isopropyldecyl]-α-[(R)-α-(3,3-dimethyl-2-oxobutyl)hydrocinnamamido]-3-t-butoxycarbonylimidazol-4-propionamid

0.32 g (0.013 Grammatom) Magnesiumspäne werden mit 10 ml absolutem Aether überschichtet und danach mit einer Lösung von 1.66 ml (13 mMol) Pentylbromid in 35 ml absolutem Aether tropfenweise so versetzt, dass das Reaktionsgemisch leicht siedet. Nach beendeter Zugabe wird das Gemisch noch 1

Stunde zum Rückfluss erhitzt und anschliessend auf -50° abgekühlt und bei dieser Temperatur tropfenweise mit einer Lösung von 3.5 g (8.8 mMol) t-Butyl (4S,5S)-4-(cyclohexyl methyl)-5-[(S)-2-formyl-3-methylbutyl]-2,2-dimethyl-3-oxazolidincarboxylat in 45 ml absolutem Aether versetzt. Nach 1-stündigem Rühren ohne Kühlung wird das Reaktionsgemisch auf Eiswasser gegossen und mit Aether extrahiert. Die Aetherextrakte werden über Magnesiumsulfat getrocknet und unter vermindertem Druck eingedampft. Chromatographie des Rückstands an 50 g Kieselgel mit einem Gemisch von Methylenchlorid und Isopropanol als Eluierungsmittel liefert 1.73 g t-Butyl (4S,5S)-4-(cyclohexylmethyl)-5-[(2S,3S oder R)-3-hydroxy-2-isopropyloctyl]-2,2-dimethyl-3-oxazolidincarboxylat und 1.41 g des epimeren t-Butyl (4S,5S)-4-(cyclohexylmethyl)-5-[(2S,3R oder S)-3-hydroxy-2-isopropyloctyl)-2,2-dimethyl-3-oxazolydincarboxylats sowie 0.65 g eines Gemisches der beiden Epimeren jeweils als Oele, MS (jeweils): 452 $(M-CH_3)^+$.

Abspalten der Boc-Schutzgruppe unter gleichzeitiger Oeffnung des Oxazolidinrings mit Salzsäure in Methanol aus t-Butyl (4S,5S)-4-(cyclohexylmethyl)-5-[(2S,3S oder R)-3-hydroxy-2-isopropyloctyl]-2,2-dimethyl-3-oxazolidincarboxylat und Umsetzen mit 1-(t-Butoxycarbonyl)-N-[(R)-α-(3,3-dimethyl-2-oxobutyl)-hydrocinnamoyl]-L-histidin liefert das (S)-N-[(1S,2S,4S,5S oder R)-1-(Cyclohexylmethyl)-2,5-dihydroxy-4-isopropyldecyl]-α-[(R)-α-(3,3-dimethyl-2-oxobutyl)hydrocinnamamido]-3-t-butoxycarbonylimidazol-4-propionamid, das ohne weitere Reinigung direkt in die nächste Stufe eingesetzt wird.

(S)-N-[(1S,2S,4S,5R oder S)-1-(Cyclohexylmethyl)-2,5-dihydroxy-4-isopropyldecyl]-α-[(R)-α-(3,3-dimethyl-2-oxobutyl)hydrocinnamamido]-3-t-butoxycarbonylimidazol-4-propionamid

Durch Abspalten der Boc-Schutzgruppe unter gleichzeitiger Oeffnung des Oxazolidinrings mit Salzsäure in Methanol und Umsetzen mit 1-(t-Butoxycarbonyl)-N-[(R)-α-(3,3-dimethyl-2-oxobutyl)hydrocinnamoyl]-L-histidin wird aus t-Butyl (4S,5S)-4-(cyclohexylmethyl)-5-[(2S,3R oder S)-3-hydroxy-2-isopropyloctyl]-2,2-dimethyl-3-oxazolidincarboxylat das (S)-N-[(1S,2S,4S,5R oder S)-1-(Cyclohexylmethyl)-2,5-dihydroxy-4-isopropyldecyl]-α-[(R)-α-(3,3-dimethyl-2-oxobutyl)hydrocinnamamido]-3-t-butoxycarbonylimidazol-4-propionamid erhalten, welches ohne weitere Reinigung direkt in die nächste Stufe eingesetzt wird.

(S)-N-[(1S,2S,4S)-1-(Cyclohexylmethyl)-2-hydroxy-4-isopropyl-5-oxodecyl]-α-[(R)-α-(3,3-dimethyl-2-oxobutyl)hydrocinnamamido]-3-t-butoxycarbonylimidazol-4-propionamid

235 mg (0.5 mMol) des Epimerengemisches von t-Butyl (4S,5S)-4-(cyclohexylmethyl)-5-[(2S,3S oder R)-3-hydroxy-2-isopropyloctyl]-2,2-dimethyl-3-oxazolidincarboxylat und t-Butyl (4S,5S)-4-(cyclohexylmethyl)-5-[(2S,3R oder S)-3-hydroxy-2-isopropyloctyl]-2,2-dimethyl-3-oxazolidincarboxylat werden in 5 ml Methylenchlorid gelöst und mit 400 mg Molekularsieb (3Å), 0.05 ml (0.87 mMol) Essigsäure sowie 280 mg (0.7 mMol) Pyridiniumdichromat versetzt und 1 Stunde bei Raumtemperatur gerührt. Danach wird das Reaktionsgemisch durch Dicalit filtriert, und das Filtrat unter vermindertem Druck eingedampft. Chromatographie des Rückstands an 5 g Kieselgel mit Methylenchlorid als Eluierungsmittel liefert 197 mg t-Butyl (4S,5S)-4-(cyclohexylmethyl)-5-[(S)-2-isopropyl-3-oxooctyl]-2,2-dimethyl-3-oxazolidincarboxylat als Oel, MS: 450 $(M-CH_3)^+$.

Abspalten der Boc-Schutzgruppe unter gleichzeitiger Oeffnung des Oxazolidinrings mit Salzsäure in Methanol und Umsetzen mit 1-(t-Butoxycarbonyl)-N-[(R)-α-(3,3-dimethyl-2-oxobutyl)hydrocinnamoyl]-L-histidin liefert das (S)-N-[(1S,2S,4S)-1-(Cyclohexylmethyl)-2-hydroxy-4-isopropyl-5-oxodecyl]-α-[(R)-α-(3,3-dimethyl-2-oxobutyl)hydrocinnamamido]-3-t-butoxycarbonylimidazol-4-propionamid, welches ohne weitere Reinigung direkt in die nächste Stufe eingesetzt wird.

## Beispiel 23

0.4 g (0.5 mMol) (S)-N-[(1S,2S,4S)-4-Benzoyl-1-(cyclohexylmethyl)-2-hydroxy-5-methylhexyl]-α-[(R)-α-(3,3-dimethyl-2-oxobutyl)hydrocinnamamido]-3-t-butoxycarbonylimidazol-4-propionamid wird in 5 ml Methylenchlorid gelöst und mit 2 ml 90%iger Trifluoressigsäure versetzt und 4 Stunden bei Raumtemperatur gerührt. Danach wird das Reaktionsgemisch auf 2N Natriumbicarbonatlösung gegossen und mit Methylenchlorid extrahiert. Die Methylenchloridextrakte werden über Kaliumcarbonat getrocknet und unter vermindertem Druck eingedampft. Chromatographie des Rückstands an 8 g Kieselgel mit einem Gemisch von Methylenchlorid und Isopropanol als Eluierungsmittel liefert 0.087 g (S)-N-[(1S,2S,4S)-4-Benzoyl-1-(cyclohexylmethyl)-2-hydroxy-5-methylhexyl]-α-[(R)-α-(3,3-dimethyl-2-oxobutyl)hydrocinnamamido]imidazol-4-propionamid als amorphen Festkörper, MS: 699 $(M+H)^+$.

Das als Ausgangsstoff eingesetzte (S)-N-[(1S,2S,4S)-4-Benzoyl-1-(cyclohexylmethyl)-2-hydroxy-5-methylhexyl]-α-[(R)-α-(3,3-dimethyl-2-oxobutyl)hydrocinnamamido]-3-t-butoxycarbonylimidazol-4-propionamid wurde wie folgt hergestellt:

0.237 g (0.01 Grammatom) Magnesiumspäne werden mit 5 ml absolutem Aether überschichtet und tropfenweise mit einer Lösung von 1.03 ml (10 mMol) Brombenzol in 30 ml absolutem Aether so versetzt, dass das Reaktionsgemisch leicht siedet. Anschliessend wird das Gemisch 1 Stunde zum Rückfluss erhitzt und dann auf -30° abgekühlt und mit einer Lösung von 2.60 g (6.5 mMol) t-Butyl (4S,5S)-4-(cyclohexylmethyl)-5-[(S)-2-formyl-3-methylbutyl]-2,2-dimethyl-3-oxazolidincarboxylat in 35 ml absolutem Aether bei -15° tropfenweise versetzt. Nach 1-stündigem Rühren ohne Kühlung wird das Reaktionsgemisch auf Eiswasser gegossen und mit Aether extrahiert. Die Aetherextrakte werden über Magnesiumsulfat getrocknet und unter vermindertem Druck eingedampft. Chromatographie des Rückstands an 40 g Kieselgel mit einem Gemisch von Methylenchlorid und Isopropanol als Eluierungsmittel liefert 3.1 g t-Butyl (4S,5S)-4-(cyclohexylmethyl)-5-[(S)-2-[(R oder S)-α-hydroxybenzyl]-3-methylbutyl]-2,2-dimethyl-3-oxazolidincarboxylat als Oel, MS: 458 $(M-CH_3)^+$.

2.2 g (4.65 mMol) t-Butyl (4S,5S)-4-(cyclohexylmethyl)-5-[(S)-2-[(R oder S)-α-hydroxybenzyl]-3-methylbutyl]-2,2-dimethyl-3-oxazolidincarboxylat werden in 100 ml Methylenchlorid gelöst, mit 22 g (253 mMol) Braunstein versetzt und 1 Stunde bei Raumtemperatur gerührt. Nach jeweils 1 weiteren Stunde werden noch zweimal je 11 g (126 mMol) Braunstein zugegeben. Das Reaktionsgemisch wird anschliessend noch 2,5 Stunden weitergerührt und dann filtriert. Das Filtrat wird unter vermindertem Druck eingedampft, und der Rückstand an 20 g Kieselgel mit Methylenchlorid als Eluierungsmittel chromatographiert, wobei man 1.69 g t-Butyl (4S,5S)-5-[(S)-2-benzoyl-3-methylbutyl]-4-(cyclohexylmethyl)-2,2-dimethyl-3-oxazolidincarboxylat als Oel erhält, MS: 471 $(M+H)^+$.

400 mg (0.85 mMol) t-Butyl (4S,5S)-5-[(S)-2-benzoyl-3-methylbutyl]-4-(cyclohexylmethyl)-2,2-dimethyl-3-oxazolidincarboxylat werden in 15 ml Methanol gelöst und mit 4.7 ml (18 mMol) 3.9M Salzsäure in Methanol versetzt. Das Reaktionsgemisch wird danach 20 Stunden bei Raumtemperatur gerührt und anschliessend unter vermindertem Druck eingedampft. Der Rückstand wird in 5 ml Methanol gelöst, und die Lösung wird mit 0.2 ml (1.8 mMol) Trimethylorthoformiat und einigen Kristallen p-Toluolsulfonsäure versetzt und 2 Stunden bei Raumtemperatur und 2 Stunden bei 50° gerührt. Danach wird das Reaktionsgemisch auf 2N Natriumbicarbonatlösung gegossen und mit Methylenchlorid extrahiert. Die Methylenchloridextrakte werden über Magnesiumsulfat getrocknet und unter vermindertem Druck eingedampft, wobei man 0.27 g (αS,2S,4S)-α-(Cyclohexylmethyl)tetrahydro-4-isopropyl-5-methoxy-5-phenyl-2-furanmethanamin als Oel erhält, MS: 313 $(M-CH_3OH)^+$.

250 mg (0.72 mMol) (αS,2S,4S)-α-(Cyclohexylmethyl)tetrahydro-4-isopropyl-5-methoxy-5-phenyl-2-furanmethanamin und 390 mg (0.80 mMol) 1-(t-Butoxycarbonyl)-N-[(R)-α-(3,3-dimethyl-2-oxobutyl)-hydrocinnamoyl]-L-histidin werden in 5 ml Methylenchlorid gelöst und bei -20° mit zunächst 0.15 ml (0.8 mMol) Hünigbase und anschliessend 0.13 ml (0.8 mMol) Diäthylcyanphosphonat tropfenweise versetzt. Danach wird das Reaktionsgemisch 45 Stunden bei Raumtemperatur gerührt und anschliessend auf Wasser gegossen und mit Methylenchlorid extrahiert. Die Methylenchloridextrakte werden über Kaliumcarbonat getrocknet und unter vermindertem Druck eingedampft. Chromatographie des erhaltenen Rückstands an 10 g Kieselgel mit einem Gemisch von Methylenchlorid und Isopropanol als Eluierungsmittel liefert 400 mg (S)-N-[(1S,2S,4S)-4-Benzoyl-1-(cyclohexylmethyl)-2-hydroxy-5-methylhexyl]-α-[(R)-α-(3,3-dimethyl-2-oxobutyl)-hydrocinnamamido]-3-t-butoxycarbonylimidazol-4-propionamid, welches ohne weitere Reinigung direkt in die nächste Stufe eingesetzt wird.

## Beispiel 24

In analoger Weise wie in Beispiel 6 beschrieben, wurden durch Abspaltung der Boc-Schutzgruppe am Imidazolring die folgenden Verbindungen hergestellt:

- Aus t-Butyl [(S)-[[(S)-1-[[(1S,2S,4S,E)-1-(cyclohexylmethyl)-2-hydroxy-4-isopropyl-7-(phenäthylcarbamoyl)-6-heptenyl]carbamoyl)-2-(3-t-butoxycarbonyl)imidazol-4-yläthyl]carbamoyl]phenäthyl]carbamat das t-Butyl [-(S)-[[(S)-1-[[(1S,2S,4S,E)-1-(cyclohexylmethyl)-2-hydroxy-4-isopropyl-7-phenäthylcarbamoyl)-6-heptenyl]-carbamoyl]-2-imidazol-4-yläthyl]carbamoyl]phenäthyl]carbamat als amorpher Festkörper, MS: 799 $(M+H)^+$ und
- aus t-Butyl [(S)-α-[[(S)-1-[[(1S,2S,4S,E)-1-(cyclohexylmethyl)-2-hydroxy-4-isopropyl-7-(2-pyridylcarbamoyl)-6-heptenyl]carbamoyl]-2-(3-t-butoxycarbonyl)imidazol-4-yläthyl]carbamoyl]phenätyl]carbamat das t-Butyl [-(S)-α-[[(S)-1-[[(1S,2S,4S, E)-1-(cyclohexylmethyl)-2-hydroxy-4-isopropyl-7-(2-pyridylcarbamoyl)-6-heptenyl]-carbamoyl]-2-imidazol-4-yläthyl]carbamoyl]phenyäthyl]carbamat als amorpher Festkörper, MS: 786 $(M+H)^+$.

Die als Ausgangsstoffe eingesetzten Carbamate wurden wie folgt hergestellt:

t-Butyl [(S)-[[(S)-1-[[(1S,2S,4S,E)-1-(cyclohexylmethyl)-2-hydroxy-4-isopropyl-7-(phenäthylcarbamoyl)-6-heptenyl]carbamoyl)-2-(3-t-butoxycarbonyl)imidazol-4-yläthyl]carbamoyl]phenätyl]carbamat

2.0 g (4.86 mMol) t-Butyl 4-(cyclohexylmethyl)-5-(4-hydroxy-2-isopropylbutyl)-2,2-dimethyl-3-oxazolidincarboxylat in 10 ml Methylenchlorid werden bei Raumtemperatur innerhalb von 10 Minuten zu einer Suspension von 2.74 g (7.3 mMol) Pyridiniumdichromat in 70 ml Methylenchlorid getropft, und das Reaktionsgemisch anschliessend 24 Stunden bei Raumtemperatur intensiv gerührt. Danach wird das Reaktionsgemisch unter vermindertem Druck eingedampft, und der Rückstand zweimal an 20 g Kieselgel mit einem Gemisch von Methylenchlorid und Isopropanol als Eluierungsmittel chromatographiert, wobei man 1.7 g t-Butyl (4S,5S)-4-(cyclohexymethyl)-5-[(R)-2-formylmethyl-3-methylbutyl]-2,2-dimethyl-3-oxazolidincarboxylat als gelbliches Oel erhält, MS: 394 $(M-CH_3)^+$.

0.39 g Natriumhydridsuspension (50% in Mineralöl) wird mit Hexan gewaschen und mit 30 ml Tetrahydrofuran überschichtet. Nach Abkühlen auf 0° wird eine Lösung von 2.4 ml (12 mMol) Phosphonoessigsäuretriäthylester in 5 ml Tetrahydrofuran bei 0° innerhalb von 5 Minuten zugetropft. Danach wird das Reaktionsgemisch 30 Minuten bei Raumtemperatur gerührt. Nach erneutem Abkühlen auf 0° werden 1.65 g (4.02 mMol) t-Butyl (4S,5S)-4-(cyclohexymethyl)-5-[(R)-2-formylmethyl-3-methylbutyl]-2,2-dimethyl-3-oxazolidincarboxylat in 10 ml Tetrahydrofuran innerhalb von fünf Minuten zugetropft. Nach beendeter Zugabe rührt man das Reaktionsgemisch 2 Stunden bei Raumtemperatur, giesst es anschliessend auf Eiswasser und extrahiert es mit Essigester. Die Essigesterextrakte werden über Magnesiumsulfat getrocknet und eingedampft. Chromatographie des Rückstands an 30 g Kieselgel mit einem Gemisch von Methylenchlorid und Isopropanol als Eluierungsmittel liefert 1.78 g t-Butyl (4S,5S)-4-(cyclohexylmethyl)-5-[(S,E)-5-(äthoxycarbonyl)-2-isopropyl-4-pentenyl]-2,2-dimethyl-3-oxazolidincarboxylat als Oel, MS: 464 $(M-CH_3)^+$.

1.7 g (3.5 mMol) t-Butyl (4S,5S)-4-(cyclohexylmethyl)-5-[(S,E)-5-(äthoxycarbonyl)-2-isopropyl-4-pentenyl]-2,2-dimethyl-3-oxazolidincarboxylat werden in 20 ml Dioxan gelöst und mit 7 ml (7.0 mMol) 1N Natronlauge versetzt. Danach wird das Reaktionsgemisch 2,5 Stunden bei 90° gerührt, abgekühlt und auf Eiswasser gegossen. Dann wird das Reaktionsgemisch mit einem pH 2-Puffer aus Kaliumhydrogensulfat und Kaliumsulfat auf pH 3 eingestellt und mit Methylenchlorid extrahiert. Die Methylenchloridextrakte werden über Magnesiumsulfat getrocknet und unter vermindertem Druck eingedampft. Chromatographie des Rückstands an 18 g Kieselgel mit einem Gemisch von Methylenchlorid und Isopropanol als Eluierungsmittel liefert 1.28 g (E,S)-5-[[(4S,5S)-3-(t-Butoxycarbonyl)-4-(cyclohexylmethyl)-2,2-dimethyl-5-oxazolidinyl]-methyl]-6-methyl-2-heptensäure als Oel, MS: 436 $(M-CH_3)^+$.

Ein Gemisch von 0.63 g (1.39 mMol) (E,S)-5-[[(4S,5S)-3-(t-Butoxycarbonyl)-4-(cyclohexylmethyl)-2,2-dimethyl-5-oxazolidinyl]methyl]- 6-methyl-2-heptensäure 0.32 g (1.7 mMol) EDC, 0.28 g (2 mMol) HOBT und 20 ml Methylenchlorid wird auf -10° abgekühlt, mit 0.35 ml (2.7 mMol) N-Aethylmorpholin und 0.26 ml (2 mMol) 2-Phenäthylamin in 10 ml Methylenchlorid versetzt und 1,5 Stunden bei -10° und 20 Stunden bei Raumtemperatur gerührt. Danach wird das Reaktionsgemisch auf Eiswasser gegossen, mit pH 2-Puffer aus Kaliumhydrogensulfat und Kaliumsulfat auf pH 3 eingestellt und mit Methylenchlorid extrahiert. Die Methylenchloridextrakte werden mit 2N Natriumcarbonatlösung gewaschen, über Magnesiumsulfat getrocknet und unter vermindertem Druck eingedampft. Chromatographie des Rückstands an 7.5 g Kieselgel liefert 0.66 g t-Butyl (4S,5S)-4-(cyclohexylmethyl)-5-[(S)-2-isopropyl-5-(phenäthylcarbamoyl)-4-pentenyl]-2,2-dimethyl-3-oxazolidincarboxylat als farbloses Harz, MS: 539 $(M-CH_3)^+$.

Abspalten der Boc-Schutzgruppe unter gleichzeitiger Oeffnung des Oxazolidinrings mit Salzsäure in Methanol und Umsetzen mit Boc-Phe-His(Boc)-OH liefert das t-Butyl [(S)-[[(S)-1-[[(1S,2S,4S,E)-1-(cyclohexylmethyl)-2-hydroxy-4-isopropyl-7-(phenäthylcarbamoyl)-6-heptenyl]carbamoyl)-2-(3-t-butoxycarbonyl)-imidazol-4-yläthyl]carbamoyl]phenäthyl]carbamat, welches ohne weitere Reinigung direkt in die nächste Stufe eingesetzt wird.

t-Butyl [(S)-α-[[(S)-1-[[(1S,2S,4S,E)-1-(cyclohexylmethyl)-2-hydroxy-4-isopropyl-7-(2-pyridylcarbamoyl)-6-heptenyl]carbamoyl]-2-(3-t-butoxycarbonyl)imidazol-4-yläthyl]carbamoyl]phenäthyl]carbamat

Diese Verbindung wird, in analoger Weise wie oben beschrieben, durch Umsetzen von (E,S)-5-[[-(4S,5S)-3-(t-Butoxycarbonyl)-4-(cyclohexylmethyl)-2,2-dimethyl-5-oxazolidinyl]methyl]-6-methyl-2-heptensäure mit 2-Pyridylmethylamin, Abspalten der Boc-Schutzgruppe unter gleichzeitiger Oeffnung des Oxazolidinrings mit Salzsäure in Methanol aus dem erhaltenen t-Butyl (4S,5S)-4-(cyclohexylmethyl)-5-[(E,S)-2-isopropyl-5-[(2-pyridylmethyl)carbamoyl]-4-pentenyl]-2,2-dimethyl-3-oxazolidincarboxylat [gelbliches Harz, MS: 541 $(M+H)^+$] und Umsetzen mit Boc-Phe-His(Boc)-OH erhalten, die ohne weitere Reinigung direkt in die nächste Stufe eingesetzt wird.

## Beispiel 25

80 mg (0.10 mMol) t-Butyl [(S)-[[(S)-1-[[(1S,2S,4S, E)-1-(cyclohexylmethyl)-2-hydroxy-4-isopropyl-7-phenäthylcarbamoyl)-6-heptenyl]carbamoyl]-2-imidazol-4-yläthyl)carbamoyl]phenäthyl]carbamat werden in 1 ml Methanol gelöst und nach Zugabe von 0.015 g Palladium auf Kohle (5%) 7 Stunden bei Raumtemperatur und Atmosphärendruck hydriert. Nach beendeter Wasserstoffaufnahme wird der Katalysator abfiltriert, und das Filtrat unter vermindertem Druck eingedampft, wobei man 64 mg t-Butyl [(S)-α-[[(S)-1-[[(1S,2S,4S)-1-(cyclohexylmethyl)-2-hydroxy-4-isopropyl-7-(phenäthylcarbamoyl)heptyl]carbamoyl]-2-imidazol-4-yläthyl]-carbamoyl]phenäthyl]carbamat als amorphen Festkörper erhält, MS: 801 $(M+H)^+$.

In analoger Weise wie oben beschrieben, wurde durch Hydrierung von t-Butyl [(S)-α-[[(S)-1-[[(1S,2S,4S, E)-1-(cyclohexylmethyl)-2-hydroxy-4-isopropyl-7-(2-pyridylcarbamoyl)-6-heptenyl]carbamoyl]-2-imidazol-4-yläthyl]carbamoyl]phenyäthyl]carbamat das t-Butyl [(S)-α-[[(S)-1-[[(1S,2S,4S)-1-(cyclohexylmethyl)-2-hydroxy-4-isopropyl-7-[(2-pyridylmethyl)carbamoyl]hetpyl]carbamoyl]-2-imidazol-4-yläthyl]carbamoyl]-phenäthyl]carbamat als beiges amorphes Pulver erhalten, MS: 788 $(M+H)^+$.

## Beispiel 26

In analoger Weise wie in Beispiel 6 beschrieben, wurde durch Abspalten der Boc-Schutzgruppe am Imidazolring mit Kaliumcarbonat in Methanol aus (S)-N-[(1S,2S,4R)-4-[(Butylcarbamoyl)methyl]-1-(cyclohexylmethyl)-2-hydroxy-5-methylhexyl]-α-[(R)-α-(3,3-dimethyl-2-oxobutyl)hydrocinnamamido]-3-t-butoxycarbonylimidazol-4-propionamid das (S)-N-[(1S,2S, 4R)-4-[(Butylcarbamoyl)methyl]-1-(cyclohexylmethyl)-2-hydroxy-5-methylhexyl]-α-[(R)-α-(3,3-dimethyl-2-oxobutyl)hydrocinnamamido]imidazol-4-propionamid als amorpher Festkörper erhalten, MS: 708 $(M+H)^+$.

Das als Ausgangsstoff eingesetzte (S)-N-[(1S,2S,4R)-4-[(Butylcarbamoyl)methyl]-1-(cyclohexylmethyl)-2-hydroxy-5-methylhexyl]-α-[(R)-α-(3,3-dimethyl-2-oxobutyl)hydrocinnamamido]-3-t-butoxycarbonylimidazol-4-propionamid wurde wie folgt hergestellt:

1.5 g (3.64 mMol) t-Butyl 4-(cyclohexylmethyl)-5-(4-hydroxy-2-isopropylbutyl)-2,2-dimethyl-3-oxazolidincarboxylat werden in 22 ml Benzol gelöst und mit 2.85 g (18 mMol) fein pulverisiertem Kaliumpermanganat und 12 ml Wasser versetzt. Das Reaktionsgemisch wird dann auf 10° abgekühlt und unter Rühren mit einer Lösung von 0.51 g Tricaprylmethylammoniumchlorid in 2 ml Benzol und anschliessend mit 2.4 ml (42 mMol) Eisessig versetzt. Danach wird das Reaktionsgemisch 18 Stunden bei Raumtemperatur intensiv gerührt. Zur Aufarbeitung wird das Reaktionsgemisch dann auf Eiswasser gegossen, mit Natriumpyrosulfit entfärbt und anschliessend mit Aether extrahiert. Die Aetherextrakte werden über Magnesiumsulfat getrocknet und unter vermindertem Druck eingedampft. Chromatographie des Rückstands an 25 g Kieselgel mit einem Gemisch von Methylenchlorid und Isopropanol als Eluierungsmittel liefert 1.3 g (αR,4S,5S)-3-(t-Butoxycarbonyl)-4- (cyclohexylmethyl)-β-isopropyl-2,2-dimethyl-5-oxazolidinbuttersäure als Oel, MS: 410 $(M-CH_3)^+$.

Ein Gemisch von 1.25 g (2.94 mMol) (αR,4S,5S)-3-(t-Butoxycarbonyl)-4-(cyclohexylmethyl)-β-isopropyl-2,2-dimethyl-5-oxazolidinbuttersäure 0.473 g (3.5 mMol) HOBT, 0.671 g (3.5 mMol) EDC, 0.72 ml (5.7 mMol) N-Aethylmorpholin und 60 ml Methylenchlorid wird auf -10° abgekühlt und mit 0.32 g (4.4 mMol) Butylamin in 10 ml Methylenchlorid versetzt. Danach wird das Reaktionsgemisch 18 Stunden ohne Kühlung gerührt, dann auf Eiswasser gegossen und mit Methylenchlorid extrahiert. Die Methylenchloridextrakte werden mit einem pH 2-Puffer aus Kaliumhydrogensulfat und Kaliumsulfat sowie 2N Natriumcarbonatlösung gewaschen, über Magnesiumsulfat getrocknet und unter vermindertem Druck eingedampft. Auf diese Weise werden 1.38 g t-Butyl (4S,5S)-5-[(R)-2- [(butylcarbamoyl]methyl]-3-methylbutyl]-4-(cyclohexylmethyl)-2,2-dimethyl-3-oxazolidincarboxylat als Oel erhalten, MS: 465 $(M-CH_3)^+$.

Durch Abspalten der Boc-Schutzgruppe unter gleichzeitiger Oeffnung des Oxazolidinrings und Umsetzen mit 1-(t-Butoxycarbonyl)-N-[(R)-α-(3,3-dimethyl-2-oxobutyl)hydrocinnamoyl]-L-histidin wird das (S)-N-[-(1S,2S,4R)-4-[(Butylcarbamoyl)methyl]-1-(cyclohexylmethyl)-2-hydroxy-5-methylhexyl]-α-[(R)-α-(3,3-dimethyl-2-oxobutyl)hydrocinnamamido]-3-t-butoxycarbonylimidazol-4-propionamid erhalten, welches ohne weitere Reinigung direkt in die nächste Stufe eingesetzt wird.

## Beispiel 27

In analoger Weise wie in Beispiel 9 beschrieben, wird durch Umsetzen von (3R,5S,6S)-6-Amino-7-cyclohexyl-5-hydroxy-3-isopropylheptansäurebutylamid mit Boc-Phe-His-OH das t-Butyl [(S)-α-[[(S)-1-[[-(1S,2S,4R)-4-[(butylcarbamoyl)methyl]-1-(cyclohexylmethyl)-2-hydroxy-5-methylhexyl]carbamoyl]-2-imidazol-4-yläthyl]carbamoyl]phenäthyl]carbamat als amorpher Festkörper erhalten, MS: 725 $(M+H)^+$.

Das als Ausgangsstoff eingesetzte (3R,5S,6S)-6-Amino-7-cyclohexyl-5-hydroxy-3-isopropylheptansäurebutylamid wurde wie folgt hergestellt:

Diese Verbindung wurde durch Abspalten der Boc-Schutzgruppe unter gleichzeitiger Oeffnung des Oxazolidinrings mit Salzsäure in Methanol aus t-Butyl (4S,5S)-5-[(R)-2-[(butylcarbamoyl]methyl]-3-methylbutyl]-4-(cyclohexylmethyl)-2,2-dimethyl-3-oxazolidincarboxylat erhalten und ohne weitere Reiningung direkt in die nächste Stufe eingesetzt.

## Beispiel A

Eine sterilfiltrierte, wässrige Lösung von (S)-N-[(1S,2S,4R)-4-(Butylcarbamoyl)methyl]-1-(cyclohexylmethyl)-2-hydroxy-5-methylhexyl]-α-[(R)-α-(3,3-dimethyl-2-oxobutyl)hydrocinnamamido]imidazol-4-propionamid wird unter Erwärmen mit einer sterilen Gelatinelösung, welche als Konservierungsmittel Phenol enthält, unter aseptischen Bedingungen so vermischt, dass 1,0 ml Lösung folgende Zusammensetzung hat:

| | |
|---|---|
| (S)-N-[(1S,2S,4R)-4-(Butylcarbamoyl)methyl]-1-(cyclohexylmethyl)-2-hydroxy-5-methylhexyl]-α-[(R)-α-(3,3-dimethyl-2-oxobutyl)hydrocinnamamido]imidazol-4-propionamid | 3,0 mg |
| Gelatine | 150,0 mg |
| Phenol | 4,7 mg |
| dest. Wasser bis zu | 1,0 ml |

Die Mischung wird unter aseptischen Bedingungen in Vialen zu 1,0 ml abgefüllt.

## Beispiel B

Man löst 5 mg (S)-N-[(1S,2S,4R)-4-(Butylcarbamoyl)methyl]-1-(cyclohexylmethyl)-2-hydroxy-5-methylhexyl]-α-[(R)-α-(3,3-dimethyl-2-oxobutyl)hydrocinnamamido]imidazol-4-propionamid in 1 ml einer wässrigen Lösung mit 20 mg Mannit. Die Lösung wird sterilfiltriert und unter aseptischen Bedingungen in eine 2 ml Ampulle gefüllt, tiefgekühlt und lyophilisiert. Vor Gebrauch wird das Lyophilisat in 1 ml destilliertem Wasser oder 1 ml physiologischer Salzlösung gelöst. Die Lösung wird intramuskulär oder

intravenös angewendet. Diese Formulierung kann auch in Doppelkammerspritzampullen abgefüllt werden.

Beispiel C

In einer Mischung von 3,5 ml Myglyol 812 und 0,08 g Benzylalkohol werden 500 mg fein gemahlenes (5,0 µm) (S)-N-[(1S,2S,4R)-4-(Butylcarbamoyl)methyl]-1-(cyclohexylmethyl)-2-hydroxy-5-methylhexyl]-α-[-(R)-α-(3,3-dimethyl-2-oxobutyl)hydrocinnamamido]imidazol-4-propionamid suspendiert. Diese Suspension wird in einen Behälter mit Dosierventil eingefüllt. Es werden 5,0 g Freon 12 unter Druck durch das Ventil in den Behälter abgefüllt. Durch Schütteln wird das Freon in der Myglyol-Benzylalkoholmischung gelöst. Dieser Spraybehälter enthält ca. 100 Einzeldosen, die einzeln appliziert werden können.

Beispiel D

Wenn man nach den in den Beispielen A-C beschriebenen Verfahren arbeitet, können aus den folgenden, ebenfalls bevorzugten Verbindungen entsprechende galenische Präparate hergestellt werden:
t-Butyl [(S)-α-[[(S)-1-[[(1S,2S,4R)-4-[(butylcarbamoyl)methyl]-1-(cyclohexylmethyl)-2-hydroxy-5-methylhexyl]carbamoyl]-2-imidazol-4-yläthyl]carbamoyl]phenäthyl]carbamat;
(S)-N-[1S,2S,4S)-1-(Cyclohexylmethyl)-2-hydroxy-4-isopropyl-5-oxodecyl]-α-[(R)-α-(3,3-dimethyl-2-oxobutyl]-hydrocinnamamido]imidazol-4-propionamid;
(S)-N-[(1S,2S,4S,E)-1-(Cyclohexylmethyl)-2-hydroxy-4-isopropyl-6-[(2-pyridylmethyl)carbamoyl]-5-hexenyl]-α-[(R)-α-(3,3-dimethyl-2-oxobutyl)hydrocinnamamido]imidazol-4-propionamid;
t-Butyl [(S)-α-[[(S)-1-[[(1S,2S,4S)-1-(cyclohexylmethyl)-2-hydroxy-4-isopropyl-6-(propionyloxy)hexyl]-carbamoyl]-2-imidazol-4-yläthyl]carbamoyl]phenäthyl]carbamat;
t-Butyl (R)-2-[[(S)-α-[[(R und S)-1-[[(1S,2S,4S)-1-(cyclohexylmethyl]-2-hydroxy-4-isopropyl-6-(pivaloyloxy)-hexyl]carbamoyl]-2-imidazol-4-yläthyl]carbamoyl]phenäthyl]carbamoyl]-1-pyrrolidincarboxylat;
(2S oder R,3S,5S,6S)-6-(Boc-D-Pro-Phe-His-NH)-7-cyclohexyl-3-isopropyl-1-(phenäthylamino)-2,5-heptandiol;
(2R oder S,3S,5S,6S)-6-(Boc-D-Pro-Phe-His-NH)-7-cyclohexyl-3-isopropyl-1-(phenäthylamino)-2,5-heptandiol und
(2RS,3S,5S,6S)-6-(Boc-D-Pro-Phe-His-NH)-7-cyclohexyl-3-isopropyl-1-[(2-pyridylmethyl)amino]-2,5-heptandiol.

**Ansprüche**

1. Aminosäurederivate der allgemeinen Formel

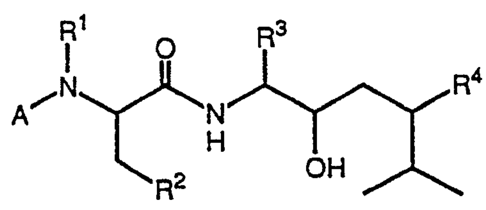

I

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, A eine der Gruppen

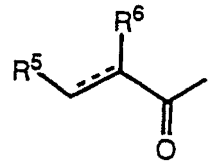

(a)

und -Y-Z (b)

und $R^4$ Alkanoyl, Arylcarbonyl, 2,2-Dialkylvinyl oder eine der Gruppen

$-CH=CH-R^7$

(c)

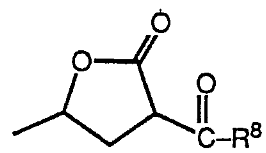

(d)

und

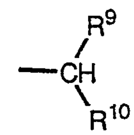

(e)

bedeuten, worin die gestrichelte Linie eine zusätzliche Bindung bedeuten kann, $R^5$ Phenyl, substituiertes Phenyl, Benzyl oder Naphthyl und $R^6$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Aminoalkylcarbonylalkyl, substituiertes Aminoalkylcarbonylalkyl, Aminoalkylsulfonylalkyl, substituiertes Aminoalkylsulfonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl, Alkylsulfonylalkyl, Arylthioalkyl, Arylsulfinylalkyl, Arylsulfonylalkyl, Arylalkylthioalkyl, Arylalkylsulfinylalkyl oder Arylalkylsulfonylalkyl bedeuten, mit der Massgabe, dass $R^6$ nicht Alkoxycarbonylamino oder Arylalkoxycarbonylamino bedeuten kann, wenn $R^5$ Phenyl, Benzyl oder α-Naphthyl bedeutet, Y den N-terminal mit Z verbundenen, bivalenten Rest von gegebenenfalls N- und/oder α-methyliertem Phenylglycin, Cyclohexylglycin, Phenylalanin, Cyclohexylalanin, 4-Fluorphenylalanin, 4-Chlorphenylalanin, Tyrosin, O-Methyltyrosin, α-Naphthylalanin oder Homophenylalanin, Z Acyl, $R^7$ Alkyl, Aryl, Heteroaryl, Alkylaminocarbonyl, Arylalkylaminocarbonyl oder Heteroarylalkylaminocarbonyl, $R^8$ Alkoxy, $R^9$ Wassersstoff oder Hydroxy und $R^{10}$ Azidomethyl, die Gruppe (c) oder eine der Gruppen

$-CH_2-OR^{11}$

(f)

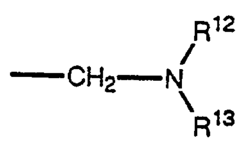

(g)

und

$-(CH_2)_n-R^7$

(h)

bedeuten, worin $R^{11}$ Wasserstoff, Alkyl, Arylalkyl, Aryl, Alkanoyl, Arylalkylcarbonyl, Heteroarylalkylcarbonyl oder Alkylcarbamoyl, $R^{12}$ und $R^{13}$ unabhängig voneinander je Wasserstoff, Alkyl, Arylalkyl, Heteroarylalkyl, Aryl, Alkanoyl, Alkoxycarbonyl, Arylalkylcarbonyl, Arylalkoxycarbonyl, Heteroarylalkylcarbonyl, Heterocycloalkylcarbonyl, Alkylcarbamoyl oder die Gruppe

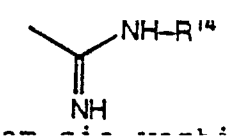

oder $R^{12}$ und $R^{13}$ zusammen mit dem sie verbindenden Stickstoffatom einen 5- oder 6-gliedrigen Heterocyclus oder gegebenenfalls substituiertes Benzimidazolonyl, n 0, 1 oder 2 und $R^{14}$ Wasserstoff oder Arylalkoxycarbonyl bedeuten,
in Form von optisch reinen Diastereomeren, Diastereomerengemischen, diastereomeren Racematen oder Gemischen von diastereomeren Racematen sowie pharmazeutisch verwendbare Salze dieser Verbindungen.

2. Verbindungen gemäss Anspruch 1, worin $R^1$ Wasserstoff bedeutet.

3. Verbindungen gemäss Anspruch 1 oder 2, worin $R^2$ Imidazol-2-yl, Imidazol-4-yl oder Thiazol-4-yl, insbesondere Imidazol-4-yl, bedeutet.

4. Verbindungen gemäss einem der Ansprüche 1-3, worin $R^3$ Cyclohexylmethyl bedeutet.

5. Verbindungen gemäss einem der Ansprüche 1-4, worin $R^4$ Alkanoyl oder die Gruppe (c) oder (e), insbesondere Alkanoyl oder die Gruppe (e), bedeutet.

6. Verbindungen gemäss Anspruch 5, worin $R^7$ Heteroarylalkylaminocarbonyl, insbesondere Pyridylalkylaminocarbonyl, bedeutet.

7. Verbindungen gemäss Anspruch 5, worin $R^{10}$ die Gruppe (f), (g) oder (h), insbesondere die Gruppe (g) oder die Gruppe (h), worin n 0 und $R^7$ Alkylaminocarbonyl bedeuten, bedeutet.

8. Verbindungen gemäss Anspruch 7, worin $R^{11}$ Alkanoyl bedeutet.

9. Verbindungen gemäss Anspruch 7, worin $R^{12}$ Wasserstoff bedeutet.

10. Verbindungen gemäss Anspruch 7 oder 9, worin $R^{13}$ Alkyl, Arylalkyl, Heteroarylalkyl oder Heteroarylalkylcarbonyl, insbesondere Arylalkyl oder Heteroarylalkyl, ganz besonders bevorzugt Phenylalkyl oder Pyridylalkyl, bedeutet.

11. Verbindungen gemäss einem der Ansprüche 1-10, worin A die Gruppe (b) bedeutet.

12. Verbindungen gemäss Anspruch 11, worin Y den N-terminal mit Z verbundenen, bivalenten Rest von Phenylalanin bedeutet.

13. Verbindungen gemäss Anspruch 11 oder 12, worin Z die Gruppe $R^a$-O-CO- oder den Rest einer durch diese Gruppe acylierten α-Aminosäure, inbesondere Prolin, bedeutet, worin $R^a$ einen gegebenenfalls substituierten, gesättigten aliphatischen Kohlenwasserstoffrest mit bis zu 10 Kohlenstoffatomen oder einen gegebenenfalls substituierten heteroaromatischen Kohlenwasserstoffrest mit bis zu 18 Kohlenstoffatomen, insbesondere einen gesättigten aliphatischen Kohlenwasserstoffrest mit bis zu 6 Kohlenstoffatomen, ganz besonders bevorzugt t-Butyl, bedeutet.

14. Verbindungen gemäss einem der Ansprüche 1-10, worin A die Gruppe (a) und $R^5$ Phenyl oder substituiertes Phenyl, insbesondere Phenyl, bedeuten.

15. Verbindungen gemäss einem der Ansprüche 1-10 und 14, worin $R^6$ Alkylcarbonylalkyl, Aminoalkylcarbonylalkyl, substituiertes Aminoalkylcarbonylalkyl, Aminoalkylsulfonylalkyl, substituiertes Aminoalkylsulfonylalkyl oder Alkylsulfonylalkyl, insbesondere Alkylcarbonylalkyl oder Alkylsulfonylalkyl, ganz besonders bevorzugt $C_1$-$C_4$-Alkylcarbonylmethyl oder $C_1$-$C_4$-Alkylsulfonylmethyl, bedeutet.

16. Verbindungen gemäss Anspruch 1, worin $R^1$ Wasserstoff, $R^2$ Imidazol-4-yl, $R^3$ Cyclohexylmethyl, $R^4$ Alkanoyl oder die Gruppe (e), $R^{10}$ die Gruppe (g) oder die Gruppe (h), worin n 0 und $R^7$ Alkylaminocarbonyl bedeuten, $R^{12}$ Wasserstoff, $R^{13}$ Arylalkyl oder Heteroarylalkyl, vorzugsweise Phenylalkyl oder Pyridylalkyl, Y den N-terminal mit Z verbundenen, bivalenten Rest von Phenylalanin und Z die Gruppe $R^a$-O-CO- oder den durch diese Gruppe acylierten Rest von Prolin bedeuten, worin $R^a$ einen gesättigten, aliphatischen Kohlenwasserstoffrest mit bis zu 6 Kohlenstoffatomen, vorzugsweise t-Butyl, bedeuten.

17. (S)-N-[(1S,2S,4R)-4-[(Butylcarbamoyl)methyl]-1-(cyclohexylmethyl)-2-hydroxy-5-methylhexyl]-α-[(R)-α-(3,3-dimethyl-2-oxobutyl)hydrocinnamamido]imidazol-4-propionamid, t-Butyl [(S)-α-[[(S)-1-[[(1S,2S,4R)-4-[(butylcarbamoyl)methyl]-1-(cyclohexylmethyl)-2-hydroxy-5-methylhexyl]carbamoyl]-2-imidazol-4-yläthyl]carbamoyl]phenäthyl]carbamat, (S)-N-[(1S,2S,4S)-1-(Cyclohexylmethyl)-2-hydroxy-4-isopropyl-5-oxodecyl]-α-[(R)-α-(3,3-dimethyl-2-oxobutyl]hydrocinnamamido]imidazol 4-propionamid, (S)-N-[(1S,2S,4S,E)-1-(Cyclohexylmethyl)-2-hydroxy-4-isopropyl-6-[(2-pyridylmethyl)carbamoyl]-5-hexenyl]-α-[(R)-α-(3,3-dimethyl-2-oxobutyl)hydrocinnamamido]imidazol-4-propionamid, t-Butyl [(S)-α-[[(S)-1-[[(1S,2S,4S)-1-(cyclohexylmethyl)-2-hydroxy-4-isopropyl-6-(propionyloxy)hexyl]carbamoyl]-2-imidazol-4-yläthyl]carbamoyl]-phenäthyl]carbamat, t-Butyl (R)-2-[[(S)-α-[[(R und S)-1-[[(1S,2S,4S)- 1-(cyclohexylmethyl]-2-hydroxy-4-isopropyl-6-(pivaloyloxy)hexyl]carbamoyl]-2-imidazol-4-yläthyl]carbamoyl]phenäthyl]carbamoyl]-1-pyrrolidincarboxylat, (2S oder R,3S,5S,6S)-6-(Boc-D-Pro-Phe-His-NH)-7-cyclohexyl-3-isopropyl-1-(phenäthylamino)-2,5-heptandiol, (2R oder S,3S,5S,6S)-6-(Boc-D-Pro-Phe-His-NH)-7-cyclohexyl-3-isopropyl-1-(phenäthylamino)-2,5-heptandiol oder (2RS,3S,5S,6S)-6-(Boc-D-Pro-Phe-His-NH)-7-cyclohexyl-3-isopropyl-1-[(2-pyridylmethyl)amino]-2,5-heptandiol.

18. Verbindungen der Formeln

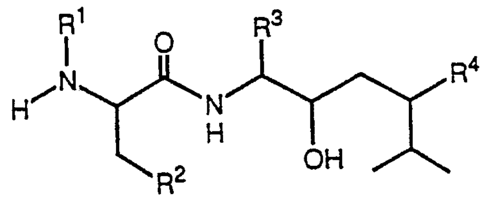

II

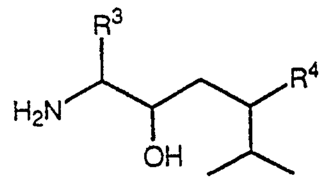

III

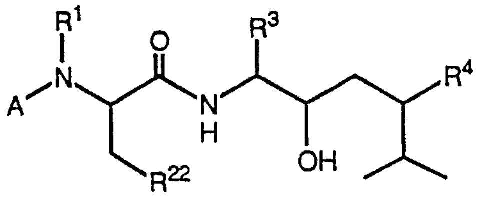

V'

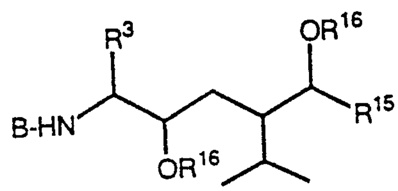

VI

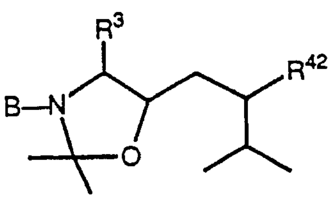

VII

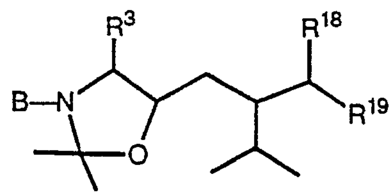

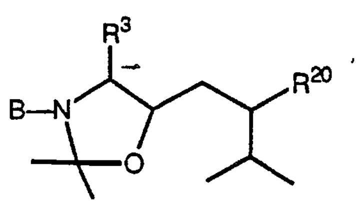

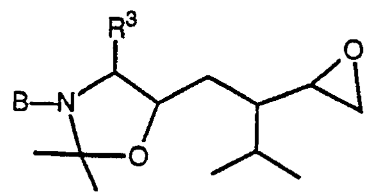

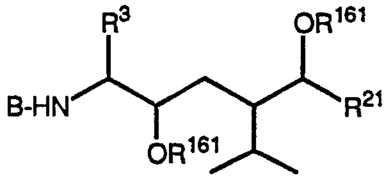

und

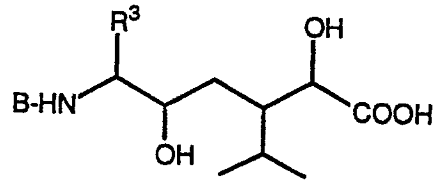

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, A eine der Gruppen

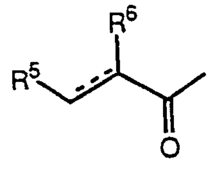

(a)

und -Y-Z (b)

und $R^4$ Alkanoyl, Arylcarbonyl, 2,2-Dialkylvinyl oder eine der Gruppen

$-CH=CH-R^7$

(c)

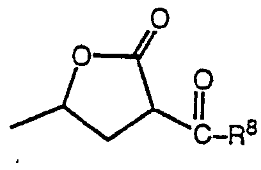

(d)

und

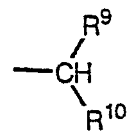

(e)

bedeuten, worin die gestrichelte Linie eine zusätzliche Bindung bedeuten kann, $R^5$ Phenyl, substituiertes Phenyl, Benzyl oder Naphthyl und $R^6$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Aminoalkylcarbonylalkyl, substituiertes Aminoalkylcarbonylalkyl, Aminoalkylsulfonylalkyl, substituiertes Aminoalkylsulfonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl, Alkylsulfonylalkyl, Arylthioalkyl, Arylsulfinylalkyl, Arylsulfonylalkyl, Arylalkylthioalkyl, Arylalkylsulfinylalkyl oder Arylalkylsulfonylalkyl bedeuten, mit der Massgabe, dass $R^6$ nicht Alkoxycarbonylamino oder Arylalkoxycarbonylamino bedeuten kann, wenn $R^5$ Phenyl, Benzyl oder $\alpha$-Naphthyl bedeutet, Y den N-terminal mit Z verbundenen, bivalenten Rest von gegebenenfalls N- und/oder $\alpha$-methyliertem Phenylglycin, Cyclohexylglycin, Phenylalanin, Cyclohexylalanin, 4-Fluorphenylalanin, 4-Chlorphenylalanin, Tyrosin, O-Methyltyrosin, $\alpha$-Naphthylalanin oder Homophenylalanin, Z Acyl, $R^7$ Alkyl, Aryl, Heteroaryl, Alkylaminocarbonyl, Arylalkylaminocarbonyl oder Heteroarylalkylaminocarbonyl, $R^8$ Alkoxy, $R^9$ Wassersstoff oder Hydroxy und $R^{10}$ Azidomethyl, die Gruppe (c) oder eine der Gruppen

$-CH_2-OR^{11}$

(f)

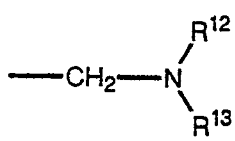

(g)

und $-(CH_2)_n-R^7$

(h)

bedeuten, worin $R^{11}$ Wasserstoff, Alkyl, Arylalkyl, Aryl, Alkanoyl, Arylalkylcarbonyl, Heteroarylalkylcarbonyl oder Alkylcarbamoyl, $R^{12}$ und $R^{13}$ unabhängig voneinander je Wasserstoff, Alkyl, Arylalkyl, Heteroarylalkyl, Aryl, Alkanoyl, Alkoxycarbonyl, Arylalkylcarbonyl, Arylalkoxycarbonyl, Heteroarylalkylcarbonyl, Heterocycloalkylcarbonyl, Alkylcarbamoyl oder die Gruppe

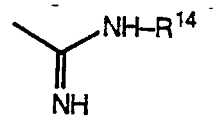

oder $R^{12}$ und $R^{13}$ zusammen mit dem sie verbindenden Stickstoffatom einen 5- oder 6-gliedrigen Heterocyclus oder gegebenenfalls substituiertes Benzimidazolonyl, n 0, 1 oder 2 und $R^{14}$ Wasserstoff oder Arylalkoxycarbonyl bedeuten, $R^{22}$ N-geschütztes Imidazol 2-yl, Imidazol-4-yl oder Pyrazol-3-yl, B eine Aminoschutzgruppe, $R^{15}$ die Gruppe (f) oder Alkylaminocarbonyläthyl, Arylalkylaminocarbonyläthyl oder Heteroarylalkylaminocarbonyläthyl und $R^{16}$ eine O-Schutzgruppe oder $R^{15}$ Alkylaminocarbonyl, Arylalkylaminocarbonyl oder Heteroarylalkylaminocarbonyl und $R^{16}$ Wasserstoff und $R^{42}$ Alkanoyl, Arylcarbonyl, 2,2-Dialkylvinyl oder eine der Gruppen (c), (d) und (e) bedeuten, mit der Massgabe, dass in der Gruppe (e) $R^9$ Wasserstoff bedeutet, wenn $R^{10}$ die Gruppe (f) oder die Gruppe (h) bedeutet, worin n 0 oder 2 und $R^7$ Alkylaminocarbonyl, Arylalkylaminocarbonyl oder Heteroarylalkylaminocarbonyl bedeuten, $R^{18}$ Wasserstoff und $R^{19}$ Hydroxymethyl, Carboxyl, Formyl, 2-Aethoxycarbonylvinyl, 2-Carboxyvinyl, Carboxymethyl oder die Gruppen -OX oder $-CH_2OX$ oder $R^{18}$ Hydroxy und $R^{19}$ Cyan oder Carboxymethyl, $R^{20}$ 2-Aethoxycarbonylvinyl oder 2-Carboxyvinyl, $R^{21}$ Hydroxymethyl, Carboxyl, Formyl oder die Gruppe $-CH=CH-R^{72}$, $R^{161}$ eine O-Schutzgruppe, X p-Tolylsulfonyl, Methylsulfonyl, Trifluormethylsulfonyl oder p-Brombenzolsulfonyl und $R^{72}$ Alkylaminocarbonyl, Arylalkylaminocarbonyl oder Heteroarylalkylaminocarbonyl bedeuten.

19. Aminosäurederivate gemäss einem der Ansprüche 1-17 zur Anwendung als therapeutische Wirkstoffe.

20. Aminosäurederivate gemäss einem der Ansprüche 1-17 zur Anwendung bei der Bekämpfung bzw. Verhütung von Bluthochdruck und Herzinsuffizienz.

21. Verfahren zur Herstellung einer Verbindung gemäss einem der Ansprüche 1-17, dadurch gekennzeichnet, dass man

a) eine Verbindung der allgemeinen Formel

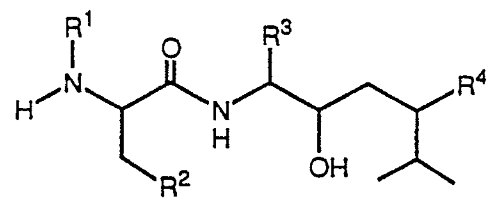

II

worin $R^1$, $R^2$, $R^3$ und $R^4$ die in Anspruch 1 angegebene Bedeutung besitzen,
mit einem die Gruppe

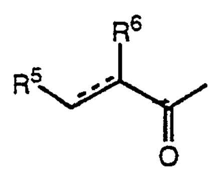

(a)

oder -Y-Z (b)
worin $R^5$, $R^6$, Y, Z und die gestrichelte Linie die in Anspruch 1 angegebene besitzen,
abgebenden Acylierungsmittel umsetzt, oder

b) eine Verbindung der allgemeinen Formel

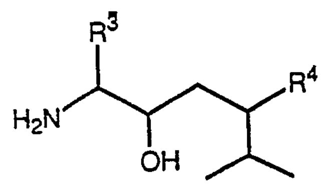

III

worin $R^3$ und $R^4$ die in Anspruch 1 angegebene Bedeutung besitzen, mit einer Verbindung der allgemeinen Formel

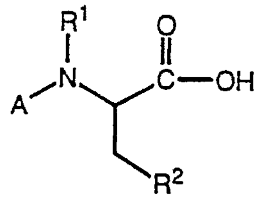

IV

worin $R^1$, $R^2$ und A die in Anspruch 1 angegebene Bedeutung besitzen, oder einem aktivierten Derivat davon umsetzt, oder

c) eine der Formel I entsprechende Verbindung, worin Z jedoch Wasserstoff bedeutet und die übrigen Symbole die in Anspruch 1 angegebene Bedeutung besitzen, mit einer acylierten Aminosäure oder einem acylierten Dipeptid umsetzt, oder

d) zur Herstellung einer Verbindung der Formel I, worin $R^2$ Imidazol-2-yl, Imidazol-4-yl oder Pyrazol-3-yl und/oder $R^{12}$ Wasserstoff bedeutet, aus einer Verbindung der allgemeinen Formel

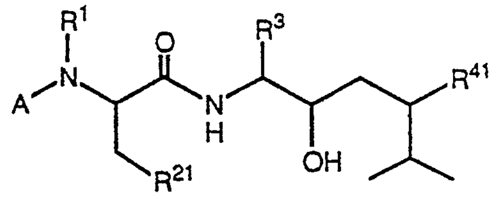

V

worin $R^{21}$ Aethyl, Propyl, Isopropyl, Thiazolyl-4-yl, Thien-2-yl, Aethoxycarbonyl, t-Butylcarbonylmethyl, Benzyloxycarbonylmethyl, t-Butoxy oder gegebenenfalls N-geschütztes Imidazol-2-yl, Imidazol-4-yl oder Pyrazol-3-yl und $R^{41}$ Alkanoyl, Arylcarbonyl, 2,2-Dialkylvinyl oder eine der Gruppen (c), (d) und (e) bedeuten und die übrigen Symbole die in Anspruch 1 angegebene Bedeutung besitzen, mit der Massgabe, dass $R^{21}$ N-geschütztes Imidazol-2-yl, Imidazol-4-yl oder Pyrazol-3-yl und/oder $R^{41}$ die Gruppe (e), worin $R^{10}$ die Gruppe (g) und $R^{12}$ leicht abspaltbares Arylalkyl, Alkoxycarbonyl oder Arylalkoxycarbonyl bedeuten, bedeutet, die N-Schutzgruppe und/oder den Substituenten $R^{12}$ abspaltet, und

e) erwünschtenfalls ein Gemisch diastereomerer Racemate in die diastereomeren Racemate oder optisch reinen Diastereomeren auftrennt, und/oder

f) erwünschtenfalls ein Diastereomerengemisch in die optisch reinen Diastereomeren auftrennt, und/oder

g) erwünschtenfalls eine erhaltene Verbindung in ein pharmazeutisch verwendbares Salz überführt.

22. Arzneimittel, enthaltend ein Aminosäurederivat gemäss einem der Ansprüche 1-17 und ein therapeutisch inertes Excipiens.

23. Mittel zur Bekämpfung bzw. Verhütung von Bluthochdruck und Herzinsuffizienz, enthaltend ein Aminosäurederivat gemäss einem der Ansprüche 1-17 und ein therapeutisch inertes Excipiens.

24. Verwendung eines Aminosäurederivates gemäss einem der Ansprüche 1-17 bei der Bekämpfung bzw. Verhütung von Krankheiten.

25. Verwendung eines Aminosäurederivates gemäss einem der Ansprüche 1-17 bei der Bekämpfung bzw. Verhütung von Bluthochdruck und Herzinsuffizienz.

26. Verwendung eines Aminosäurederivates gemäss einem der Ansprüche 1-17 zur Herstellung von Mitteln gegen Bluthochdruck und/oder Herzinsuffizienz.

Patentansprüche für folgenden Vertragsstaat: ES

1. Verfahren zur Herstellung von Aminosäurederivate der allgemeinen Formel

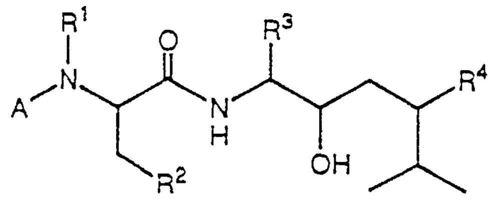

I

worin $R^1$ Wasserstoff oder Methyl, $R^2$ Aethyl, Propyl, Isopropyl, Imidazol 2-yl, Imidazol-4-yl, Pyrazol-3-yl, Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, A eine der Gruppen

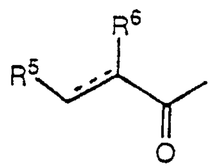

(a)

und -Y-Z (b)

und $R^4$ Alkanoyl, Arylcarbonyl, 2,2-Dialkylvinyl oder eine der Gruppen

$-CH=CH-R^7$

(c)

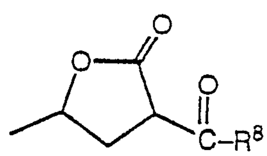

(d)

und

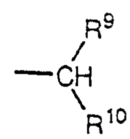

(e)

bedeuten, worin die gestrichelte Linie eine zusätzliche Bindung bedeuten kann, $R^5$ Phenyl, substituiertes Phenyl, Benzyl oder Naphthyl und $R^6$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Aminoalkylcarbonylalkyl, substituiertes Aminoalkylcarbonylalkyl, Aminoalkylsulfonylalkyl, substituiertes Aminoalkylsulfonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl, Alkylsulfonylalkyl, Arylthioalkyl, Arylsulfinylalkyl, Arylsulfonylalkyl, Arylalkylthioalkyl, Arylalkylsulfinylalkyl oder Arylalkylsulfonylalkyl bedeuten, mit der Massgabe, dass $R^6$ nicht Alkoxycarbonylamino oder Arylalkoxycarbonylamino bedeuten kann, wenn $R^5$ Phenyl, Benzyl oder $\alpha$-Naphthyl bedeutet, Y den N-terminal mit Z verbundenen, bivalenten Rest von gegebenenfalls N- und/oder $\alpha$-methyliertem Phenylglycin, Cyclohexylglycin, Phenylalanin, Cyclohexylalanin, 4-Fluorphenylalanin, 4-Chlorphenylalanin, Tyrosin, O-Methyltyrosin, $\alpha$-Naphthylalanin oder Homophenylalanin, Z Acyl, $R^7$ Alkyl, Aryl, Heteroaryl, Alkylaminocarbonyl, Arylalkylaminocarbonyl oder Heteroarylalkylaminocarbonyl, $R^8$ Alkoxy, $R^9$ Wassersstoff oder Hydroxy und $R^{10}$ Azidomethyl, die Gruppe (c) oder eine der Gruppen

$-CH_2-OR^{11}$ (f)

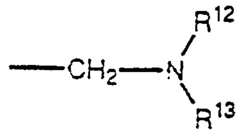

(g) und

$-(CH_2)_n-R^7$ (h)

bedeuten, worin $R^{11}$ Wasserstoff, Alkyl, Arylalkyl, Aryl, Alkanoyl, Arylalkylcarbonyl, Heteroarylalkylcarbonyl oder Alkylcarbamoyl, $R^{12}$ und $R^{13}$ unabhängig voneinander je Wasserstoff, Alkyl, Arylalkyl, Heteroarylalkyl, Aryl, Alkanoyl, Alkoxycarbonyl, Arylalkylcarbonyl, Arylalkoxycarbonyl, Heteroarylalkylcarbonyl, Heterocycloalkylcarbonyl, Alkylcarbamoyl oder die Gruppe

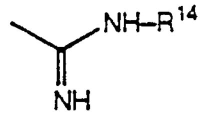

oder $R^{12}$ und $R^{13}$ zusammen mit dem sie verbindenden Stickstoffatom einen 5- oder 6-gliedrigen Heterocyclus oder gegebenenfalls substituiertes Benzimidazolonyl, n 0, 1 oder 2 und $R^{14}$ Wasserstoff oder Arylalkoxycarbonyl bedeuten,
in Form von optisch reinen Diastereomeren, Diastereomerengemischen, diastereomeren Racematen oder Gemischen von diastereomeren Racematen sowie von pharmazeutisch verwendbaren Salzen dieser Verbindungen, dadurch gekennzeichnet, dass man

a) eine Verbindung der allgemeinen Formel

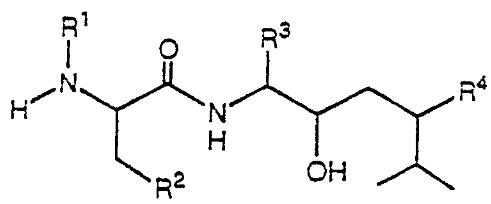

II

worin $R^1$, $R^2$, $R^3$ und $R^4$ die oben angegebene Bedeutung besitzen,
mit einem die Gruppe

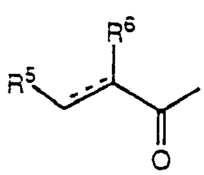

(a)

oder -Y-Z (b)
worin $R^5$, $R^6$, Y, Z und die gestrichelte Linie die oben angegebene besitzen,
abgebenden Acylierungsmittel umsetzt, oder

b) eine Verbindung der allgemeinen Formel

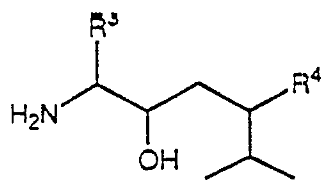

III

worin $R^3$ und $R^4$ die oben angegebene Bedeutung besitzen, mit einer Verbindung der allgemeinen Formel

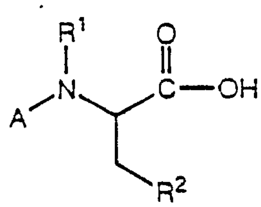

IV

worin $R^1$, $R^2$ und A die oben angegebene Bedeutung besitzen, oder einem aktivierten Derivat davon umsetzt, oder

c) eine der Formel I entsprechende Verbindung, worin Z jedoch Wasserstoff bedeutet und die übrigen Symbole die oben angegebene Bedeutung besitzen, mit einer acylierten Aminosäure oder einem acylierten Dipeptid umsetzt, oder

d) zur Herstellung einer Verbindung der Formel I, worin $R^2$ Imidazol-2-yl, Imidazol-4-yl oder Pyrazol-3-yl und/oder $R^{12}$ Wasserstoff bedeutet, aus einer Verbindung der allgemeinen Formel

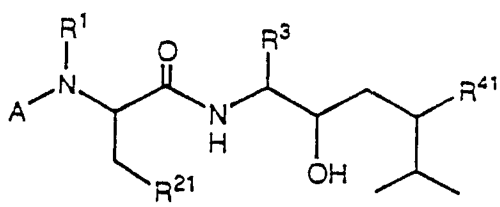

V

worin $R^{21}$ Aethyl, Propyl, Isopropyl, Thiazolyl-4-yl, Thien-2-yl, Aethoxycarbonyl, t-Butylcarbonylmethyl, Benzyloxycarbonylmethyl, t-Butoxy oder gegebenenfalls N-geschütztes Imidazol-2-yl, Imidazol-4-yl oder Pyrazol-3-yl und $R^{41}$ Alkanoyl, Arylcarbonyl, 2,2-Dialkylvinyl oder eine der Gruppen (c), (d) und (e) bedeuten und die übrigen Symbole die oben angegebene Bedeutung besitzen, mit der Massgabe, dass $R^{21}$ N-geschütztes Imidazol-2-yl, Imidazol-4-yl oder Pyrazol-3-yl und/oder $R^{41}$ die Gruppe (e), worin $R^{10}$ die Gruppe (g) und $R^{12}$ leicht abspaltbares Arylalkyl, Alkoxycarbonyl oder Arylalkoxycarbonyl bedeuten, bedeutet, die N-Schutzgruppe und/oder den Substituenten $R^{12}$ abspaltet, und

e) erwünschtenfalls ein Gemisch diastereomerer Racemate in die diastereomeren Racemate oder optisch reinen Diastereomeren auftrennt, und/oder

f) erwünschtenfalls ein Diastereomerengemisch in die optisch reinen Diastereomeren auftrennt, und/oder

g) erwünschtenfalls eine erhaltene Verbindung in ein pharmazeutisch verwendbares Salz überführt.

2. Verfahren gemäss Anspruch 1, worin $R^1$ Wasserstoff bedeutet.

3. Verfahren gemäss Anspruch 1 oder 2, worin $R^2$ Imidazol-2-yl, Imidazol-4-yl oder Thiazol-4-yl, insbesondere Imidazol-4-yl, bedeutet.

4. Verfahren gemäss einem der Ansprüche 1-3, worin $R^3$ Cyclohexylmethyl bedeutet.

5. Verfahren gemäss einem der Ansprüche 1-4, worin $R^4$ Alkanoyl oder die Gruppe (c) oder (e), insbesondere Alkanoyl oder die Gruppe (e), bedeutet.

6. Verfahren gemäss Anspruch 5, worin $R^7$ Heteroarylalkylaminocarbonyl, insbesondere Pyridylalkylaminocarbonyl, bedeutet.

7. Verfahren gemäss Anspruch 5, worin $R^{10}$ die Gruppe (f), (g) oder (h), insbesondere die Gruppe (g) oder die Gruppe (h), worin n 0 und $R^7$ Alkylaminocarbonyl bedeuten, bedeutet.

8. Verfahren gemäss Anspruch 7, worin $R^{11}$ Alkanoyl bedeutet.

9. Verfahren gemäss Anspruch 7, worin $R^{12}$ Wasserstoff bedeutet.

10. Verfahren gemäss Anspruch 7 oder 9, worin $R^{13}$ Alkyl, Arylalkyl, Heteroarylalkyl oder Heteroarylalkylcarbonyl, insbesondere Arylalkyl oder Heteroarylalkyl, ganz besonders bevorzugt Phenylalkyl oder Pyridylalkyl, bedeutet.

11. Verfahren gemäss einem der Ansprüche 1-10, worin A die Gruppe (b) bedeutet.

12. Verfahren gemäss Anspruch 11, worin Y den N-terminal mit Z verbundenen, bivalenten Rest von Phenylalanin bedeutet.

13. Verfahren gemäss Anspruch 11 oder 12, worin Z die Gruppe $R^a$-O-CO- oder den Rest einer durch diese Gruppe acylierten α-Aminosäure, inbesondere Prolin, bedeutet, worin $R^a$ einen gebenenfalls substituierten, gesättigten aliphatischen Kohlenwasserstoffrest mit bis zu 10 Kohlenstoffatomen oder einen gegebenenfalls substituierten heteroaromatischen Kohlenwasserstoffrest mit bis zu 18 Kohlenstoffatomen, insbesondere einen gesättigten aliphatischen Kohlenwasserstoffrest mit bis zu 6 Kohlenstoffatomen, ganz besonders bevorzugt t-Butyl, bedeutet.

14. Verfahren gemäss einem der Ansprüche 1-10, worin A die Gruppe (a) und $R^5$ Phenyl oder substituiertes Phenyl, insbesondere Phenyl, bedeuten.

15. Verfahren gemäss einem der Ansprüche 1-10 und 14, worin $R^6$ Alkylcarbonylalkyl, Aminoalkylcarbonylalkyl, substituiertes Aminoalkylcarbonylalkyl, Aminoalkylsulfonylalkyl, substituiertes Aminoalkylsulfonylalkyl oder Alkylsulfonylalkyl, insbesondere Alkylcarbonylalkyl oder Alkylsulfonylalkyl, ganz besonders bevorzugt $C_1$-$C_4$-Alkylcarbonylmethyl oder $C_1$-$C_4$-Alkylsulfonylmethyl, bedeutet.

16. Verfahren gemäss Anspruch 1, worin $R^1$ Wasserstoff, $R^2$ Imidazol-4-yl, $R^3$ Cyclohexylmethyl, $R^4$ Alkanoyl oder die Gruppe (e), $R^{10}$ die Gruppe (g) oder die Gruppe (h), worin n 0 und $R^7$ Alkylaminocarbonyl bedeuten, $R^{12}$ Wasserstoff, $R^{13}$ Arylalkyl oder Heteroarylalkyl, vorzugsweise Phenylalkyl oder Pyridylalkyl, Y den N-terminal mit Z verbundenen, bivalenten Rest von Phenylalanin und Z die Gruppe $R^a$-O-CO- oder den durch diese Gruppe acylierten Rest von Prolin bedeuten, worin $R^a$ einen gesättigten, aliphatischen Kohlenwasserstoffrest mit bis zu 6 Kohlenstoffatomen, vorzugsweise t-Butyl, bedeuten.

17. Verfahren gemäss Anspruch 1, dadurch gekennzeichnet, dass man (S)-N-[(1S,2S,4R)-4-[-(Butylcarbamoyl)methyl]-1-(cyclohexylmethyl)-2-hydroxy-5-methylhexyl]-α-[(R)-α-(3,3-dimethyl-2-oxobutyl)-hydrocinnamamido]imidazol-4-propionamid herstellt.

18. Verfahren gemäss Anspruch 1, dadurch gekennzeichnet, dass man t-Butyl [(S)-α-[[(S)-1-[[-(1S,2S,4R)-4- [(butylcarbamoyl)methyl]-1-(cyclohexylmethyl)-2-hydroxy-5-methylhexyl]carbamoyl]-2-imidazol-4-yläthyl]carbamoyl]phenäthyl]carbamat herstellt.

19. Verfahren gemäss Anspruch 1, dadurch gekennzeichnet, dass man (S)-N-[(1S,2S,4S)-1-(Cyclohexylmethyl)-2-hydroxy-4-isopropyl-5-oxodecyl]-α-[(R)-α-(3,3-dimethyl-2-oxobutyl]-hydrocinnamamido]imidazol-4-propionamid herstellt.

20. Verfahren gemäss Anspruch 1, dadurch gekennzeichnet, dass man (S)-N-[(1S,2S,4S,E)-1-(Cyclohexylmethyl)-2-hydroxy-4-isopropyl-6-[(2-pyridylmethyl)carbamoyl]-5-hexenyl]-α-[(R)-α-(3,3-dimethyl-2-oxobutyl]hydrocinnamamido]imidazol-4-propionamid herstellt.

21. Verfahren gemäss Anspruch 1, dadurch gekennzeichnet, dass man t-Butyl [(S)-α-[[(S)-1-[[-(1S,2S,4S)-1-(cyclohexylmethyl)-2-hydroxy-4-isopropyl-6-(propionyloxy)hexyl]carbamoyl]-2-imidazol-4-yläthyl]carbamoyl]phenäthyl]carbamat herstellt.

22. Verfahren gemäss Anspruch 1, dadurch gekennzeichnet, dass man t-Butyl (R)-2-[[(S)-α-[[(R und S)-1-[[(1S,2S,4S)-1-(cyclohexylmethyl)-2-hydroxy-4-isopropyl-6-(pivaloyloxy)hexyl]carbamoyl]-2-imidazol-4-yläthyl]carbamoyl]phenäthyl]carbamoyl]-1-pyrrolidincarboxylat herstellt.

23. Verfahren gemäss Anspruch 1, dadurch gekennzeichnet, dass man (2S oder R,3S,5S,6S)-6-(Boc-D-Pro-Phe-His-NH)-7-cyclohexyl-3-isopropyl-1-(phenäthylamino)-2,5-heptandiol herstellt.

24. Verfahren gemäss Anspruch 1, dadurch gekennzeichnet, dass man (2R oder S,3S,5S,6S)-6-(Boc-D-Pro-Phe-His-NH)-7-cyclohexyl-3-isopropyl-1-(phenäthylamino)-2,5-heptandiol herstellt.

25. Verfahren gemäss Anspruch 1, dadurch gekennzeichnet, dass man (2RS,3S,5S,6S)-6-(Boc-D-Pro-Phe-His-NH)-7-cyclohexyl-3-isopropyl-1-[(2-Pyridylmethyl)amino]-2,5-heptandiol herstellt.

26. Verfahren zur Herstellung von Arzneimitteln, insbesondere zur Verwendung bei der Bekämpfung bzw. Verhütung von Bluthochdruck und Herzinsuffizienz, dadurch gekennzeichnet, dass man ein Aminosäurederivat der Formel I in Anspruch 1, in Form eines optisch reinen Diastereomeren, eines Diastereomerengemisches, eines diastereomeren Racemates oder eines Gemisches von diastereomeren Racematen, oder ein pharmazeutisch verwendbares Salz einer solchen Verbindung in eine galenische Darreichungsform bringt.

27. Verwendung eines Aminosäurederivates der Formel I in Anspruch 1, in Form eines optisch reinen Diastereomeren. eines Diastereomerengemisches, eines diastereomeren Racemates oder eines Gemisches von diastereomeren Racematen, oder eines pharmazeutisch verwendbaren Salzes einer solchen Verbindung zur Herstellung von Mitteln gegen Bluthochdruck und/oder Herzinsuffizienz.

Patentansprüche für folgenden Vertragsstaat: GR

1. Verfahren zur Herstellung von Aminosäurederivate der allgemeinen Formel

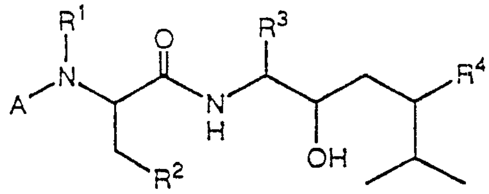

I

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, A eine der Gruppen

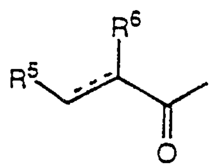

(a)

und -Y-Z (b)
und $R^4$ Alkanoyl, Arylcarbonyl, 2,2-Dialkylvinyl oder eine der Gruppen

$-CH = CH-R^7$

(c)

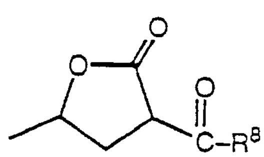

(d)

und

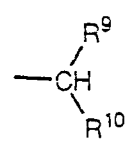

(e)

bedeuten, worin die gestrichelte Linie eine zusätzliche Bindung bedeuten kann, $R^5$ Phenyl, substituiertes Phenyl, Benzyl oder Naphthyl und $R^6$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Aminoalkylcarbonylalkyl, substituiertes Aminoalkylcarbonylalkyl, Aminoalkylsulfonylalkyl, substituiertes Aminoalkylsulfonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl, Alkylsulfonylalkyl, Arylthioalkyl, Arylsulfinylalkyl, Arylsulfonylalkyl, Arylalkylthioalkyl, Arylalkylsulfinylalkyl oder Arylalkylsulfonylalkyl bedeuten, mit der Massgabe, dass $R^6$ nicht Alkoxycarbonylamino oder Arylalkoxycarbonylamino bedeuten

kann, wenn $R^5$ Phenyl, Benzyl oder α-Naphthyl bedeutet, Y den N-terminal mit Z verbundenen, bivalenten Rest von gegebenenfalls N- und/oder α-methyliertem Phenylglycin, Cyclohexylglycin, Phenylalanin, Cyclohexylalanin, 4-Fluorphenylalanin, 4-Chlorphenylalanin, Tyrosin, O-Methyltyrosin, α-Naphthylalanin oder Homophenylalanin, Z Acyl, $R^7$ Alkyl, Aryl, Heteroaryl, Alkylaminocarbonyl, Arylalkylaminocarbonyl oder Heteroarylalkylaminocarbonyl, $R^8$ Alkoxy, $R^9$ Wassersstoff oder Hydroxy und $R^{10}$ Azidomethyl, die Gruppe (c) oder eine der Gruppen

$-CH_2-OR^{11}$

(f)

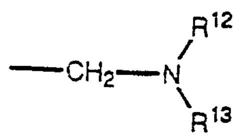

(g)

und

$-(CH_2)_n-R^7$

(h)

bedeuten, worin $R^{11}$ Wasserstoff, Alkyl, Arylalkyl, Aryl, Alkanoyl, Arylalkylcarbonyl, Heteroarylalkylcarbonyl oder Alkylcarbamoyl, $R^{12}$ und $R^{13}$ unabhängig voneinander je Wasserstoff, Alkyl, Arylalkyl, Heteroarylalkyl, Aryl, Alkanoyl, Alkoxycarbonyl, Arylalkylcarbonyl, Arylalkoxycarbonyl, Heteroarylalkylcarbonyl, Heterocycloalkylcarbonyl, Alkylcarbamoyl oder die Gruppe

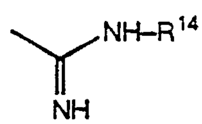

oder $R^{12}$ und $R^{13}$ zusammen mit dem sie verbindenden Stickstoffatom einen 5- oder 6-gliedrigen Heterocyclus oder gegebenenfalls substituiertes Benzimidazolonyl, n 0, 1 oder 2 und $R^{14}$ Wasserstoff oder Arylalkoxycarbonyl bedeuten,
in Form von optisch reinen Diastereomeren, Diastereomerengemischen, diastereomeren Racematen oder Gemischen von diastereomeren Racematen sowie von pharmazeutisch verwendbaren Salzen dieser Verbindungen, dadurch gekennzeichnet, dass man

a) eine Verbindung der allgemeinen Formel

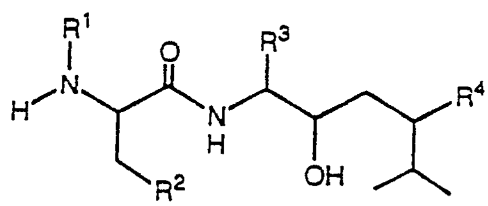

II

worin $R^1$, $R^2$, $R^3$ und $R^4$ die oben angegebene Bedeutung besitzen,
mit einem die Gruppe

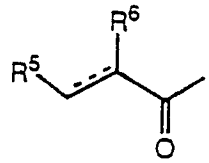

(a)

oder -Y-Z (b)

worin $R^5$, $R^6$, Y, Z und die gestrichelte Linie die oben angegebene besitzen, abgebenden Acylierungsmittel umsetzt, oder

b) eine Verbindung der allgemeinen Formel

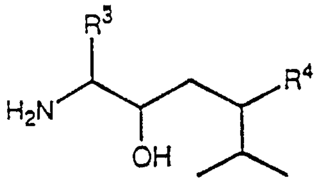

III

worin $R^3$ und $R^4$ die oben angegebene Bedeutung besitzen, mit einer Verbindung der allgemeinen Formel

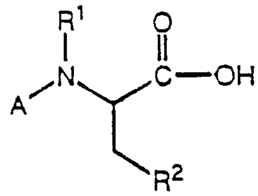

IV

worin $R^1$, $R^2$ und A die oben angegebene Bedeutung besitzen, oder einem aktivierten Derivat davon umsetzt, oder

c) eine der Formel I entsprechende Verbindung, worin Z jedoch Wasserstoff bedeutet und die übrigen Symbole die oben angegebene Bedeutung besitzen, mit einer acylierten Aminosäure oder einem acylierten Dipeptid umsetzt, oder

d) zur Herstellung einer Verbindung der Formel I, worin $R^2$ Imidazol-2-yl, Imidazol-4-yl oder Pyrazol-3-yl und/oder $R^{12}$ Wasserstoff bedeutet, aus einer Verbindung der allgemeinen Formel

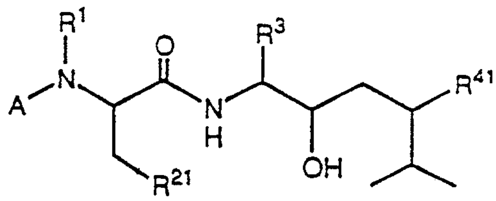

V

worin $R^{21}$ Aethyl, Propyl, Isopropyl, Thiazolyl-4-yl, Thien-2-yl, Aethoxycarbonyl, t-Butylcarbonylmethyl, Benzyloxycarbonylmethyl, t-Butoxy oder gegebenenfalls N-geschütztes Imidazol-2-yl, Imidazol-4-yl oder Pyrazol-3-yl und $R^{41}$ Alkanoyl, Arylcarbonyl, 2,2-Dialkylvinyl oder eine der Gruppen (c), (d) und (e) bedeuten und die übrigen Symbole die oben angegebene Bedeutung besitzen, mit der Massgabe, dass $R^{21}$ N-geschütztes Imidazol-2-yl, Imidazol-4-yl oder Pyrazol-3-yl und/oder $R^{41}$ die Gruppe (e), worin $R^{10}$ die Gruppe (g) und $R^{12}$ leicht abspaltbares Arylalkyl, Alkoxycarbonyl oder Arylalkoxycarbonyl bedeuten, bedeutet, die N-Schutzgruppe und/oder den Substituenten $R^{12}$ abspaltet, und

e) erwünschtenfalls ein Gemisch diastereomerer Racemate in die diastereomeren Racemate oder optisch reinen Diastereomeren auftrennt, und/oder

f) erwünschtenfalls ein Diastereomerengemisch in die optisch reinen Diastereomeren auftrennt, und/oder

g) erwünschtenfalls eine erhaltene Verbindung in ein pharmazeutisch verwendbares Salz überführt.

2. Verfahren gemäss Anspruch 1, worin $R^1$ Wasserstoff bedeutet.

3. Verfahren gemäss Anspruch 1 oder 2, worin $R^2$ Imidazol-2-yl, Imidazol 4-yl oder Thiazol-4-yl, insbesondere Imidazol-4-yl, bedeutet.

4. Verfahren gemäss einem der Ansprüche 1-3, worin $R^3$ Cyclohexylmethyl bedeutet.

5. Verfahren gemäss einem der Ansprüche 1-4, worin $R^4$ Alkanoyl oder die Gruppe (c) oder (e), insbesondere Alkanoyl oder die Gruppe (e), bedeutet.

6. Verfahren gemäss Anspruch 5, worin $R^7$ Heteroarylalkylaminocarbonyl, insbesondere Pyridylalkylaminocarbonyl, bedeutet.

7. Verfahren gemäss Anspruch 5, worin $R^{10}$ die Gruppe (f), (g) oder (h), insbesondere die Gruppe (g) oder die Gruppe (h), worin n 0 und $R^7$ Alkylaminocarbonyl bedeuten, bedeutet.

8. Verfahren gemäss Anspruch 7, worin $R^{11}$ Alkanoyl bedeutet.

9. Verfahren gemäss Anspruch 7, worin $R^{12}$ Wasserstoff bedeutet.

10. Verfahren gemäss Anspruch 7 oder 9, worin $R^{13}$ Alkyl, Arylalkyl, Heteroarylalkyl oder Heteroarylalkylcarbonyl, insbesondere Arylalkyl oder Heteroarylalkyl, ganz besonders bevorzugt Phenylalkyl oder Pyridylalkyl, bedeutet.

11. Verfahren gemäss einem der Ansprüche 1-10, worin A die Gruppe (b) bedeutet.

12. Verfahren gemäss Anspruch 11, worin Y den N-terminal mit Z verbundenen, bivalenten Rest von Phenylalanin bedeutet.

13. Verfahren gemäss Anspruch 11 oder 12, worin Z die Gruppe $R^a$-O-CO- oder den Rest einer durch diese Gruppe acylierten $\alpha$-Aminosäure, inbesondere Prolin, bedeutet, worin $R^a$ einen gegebenenfalls substituierten, gesättigten aliphatischen Kohlenwasserstoffrest mit bis zu 10 Kohlenstoffatomen oder einen gegebenenfalls substituierten heteroaromatischen Kohlenwasserstoffrest mit bis zu 18 Kohlenstoffatomen, insbesondere einen gesättigten aliphatischen Kohlenwasserstoffrest mit bis zu 6 Kohlenstoffatomen, ganz besonders bevorzugt t-Butyl, bedeutet.

14. Verfahren gemäss einem der Ansprüche 1-10, worin A die Gruppe (a) und $R^5$ Phenyl oder substituiertes Phenyl, insbesondere Phenyl, bedeuten.

15. Verfahren gemäss einem der Ansprüche 1-10 und 14, worin $R^6$ Alkylcarbonylalkyl, Aminoalkylcarbonylalkyl, substituiertes Aminoalkylcarbonylalkyl, Aminoalkylsulfonylalkyl, substituiertes Aminoalkylsulfonylalkyl oder Alkylsulfonylalkyl, insbesondere Alkylcarbonylalkyl oder Alkylsulfonylalkyl, ganz besonders bevorzugt $C_1$-$C_4$-Alkylcarbonylmethyl oder $C_1$-$C_4$-Alkylsulfonylmethyl, bedeutet.

16. Verfahren gemäss Anspruch 1, worin $R^1$ Wasserstoff, $R^2$ Imidazol-4-yl, $R^3$ Cyclohexylmethyl, $R^4$ Alkanoyl oder die Gruppe (e), $R^{10}$ die Gruppe (g) oder die Gruppe (h), worin n 0 und $R^7$ Alkylaminocarbonyl bedeuten, $R^{12}$ Wasserstoff, $R^{13}$ Arylalkyl oder Heteroarylalkyl, vorzugsweise Phenylalkyl oder Pyridylalkyl, Y den N-terminal mit Z verbundenen, bivalenten Rest von Phenylalanin und Z die Gruppe $R^a$-O-CO- oder den durch diese Gruppe acylierten Rest von Prolin bedeuten, worin $R^a$ einen gesättigten, aliphatischen Kohlenwasserstoffrest mit bis zu 6 Kohlenstoffatomen, vorzugsweise t-Butyl, bedeuten.

17. Verfahren gemäss Anspruch 1, dadurch gekennzeichnet, dass man (S)-N-[(1S,2S,4R)-4-[(Butylcarbamoyl)methyl]-1-(cyclohexylmethyl)-2-hydroxy-5-methylhexyl]-$\alpha$-[(R)-$\alpha$-(3,3-dimethyl-2-oxobutyl)-hydrocinnamamido]imidazol-4-propionamid herstellt.

18. Verfahren gemäss Anspruch 1, dadurch gekennzeichnet, dass man t-Butyl [(S)-$\alpha$-[[(S)-1-[[(1S,2S,4R)-4- [(butylcarbamoyl)methyl]-1-(cyclohexylmethyl)-2-hydroxy-5-methylhexyl]carbamoyl]-2-imidazol-4-yläthyl]carbamoyl]phenäthyl]carbamat herstellt.

19. Verfahren gemäss Anspruch 1, dadurch gekennzeichnet, dass man (S)-N-[(1S,2S,4S)-1-(Cyclohexylmethyl)-2-hydroxy-4-isopropyl-5-oxodecyl]-$\alpha$-[(R)-$\alpha$-(3,3-dimethyl-2-oxobutyl]-hydrocinnamamido]imidazol-4-propionamid herstellt.

20. Verfahren gemäss Anspruch 1, dadurch gekennzeichnet, dass man (S)-N-[(1S,2S,4S,E)-1-(Cyclohexylmethyl)-2-hydroxy-4-isopropyl-6-[(2-pyridylmethyl)carbamoyl]-5-hexenyl]-$\alpha$-[(R)-$\alpha$-(3,3-dimethyl-2-oxobutyl)hydrocinnamamido]imidazol-4-propionamid herstellt.

21. Verfahren gemäss Anspruch 1, dadurch gekennzeichnet, dass man t-Butyl [(S)-$\alpha$-[[(S)-1-[[(1S,2S,4S)-1-(cyclohexylmethyl)-2-hydroxy-4-isopropyl-6-(propionyloxy)hexyl]carbamoyl]-2-imidazol-4-yläthyl]carbamoyl]phenäthyl]carbamat herstellt.

22. Verfahren gemäss Anspruch 1, dadurch gekennzeichnet, dass man t-Butyl (R)-2-[[(S)-$\alpha$-[[(R und S)-1-[[(1S,2S,4S)-1-(cyclohexylmethyl]-2-hydroxy-4-isopropyl-6-(pivaloyloxy)hexyl]carbamoyl]-2-imidazol-4-yläthyl]carbamoyl]phenäthyl]carbamoyl]-1-pyrrolidincarboxylat herstellt.

23. Verfahren gemäss Anspruch 1, dadurch gekennzeichnet, dass man (2S oder R,3S,5S,6S)-6-(Boc-D-Pro-Phe-His-NH)-7-cyclohexyl-3-isopropyl-1-(phenäthylamino)-2,5-heptandiol herstellt.

24. Verfahren gemäss Anspruch 1, dadurch gekennzeichnet, dass man (2R oder S,3S,5S,6S)-6-(Boc-D-Pro-Phe-His-NH)-7-cyclohexyl-3-isopropyl-1-(phenäthylamino)-2,5-heptandiol herstellt.

25. Verfahren gemäss Anspruch 1, dadurch gekennzeichnet, dass man (2RS,3S,5S,6S)-6-(Boc-D-Pro-Phe-His-NH)-7-cyclohexyl-3-isopropyl-1-[(2-pyridylmethyl)amino]-2,5-heptandiol herstellt.

26. Verfahren zur Herstellung von Arzneimitteln, insbesondere zur Verwendung bei der Bekämpfung bzw. Verhütung von Bluthochdruck und Herzinsuffizienz, dadurch gekennzeichnet, dass man ein Aminosäurederivat der Formel I in Anspruch 1, in Form eines optisch reinen Diastereomeren, eines Diastereomerengemisches, eines diastereomeren Racemates oder eines Gemisches von diastereomeren Racematen, oder ein pharmazeutisch verwendbares Salz einer solchen Verbindung in eine galenische Darreichungsform bringt.

27. Verwendung eines Aminosäurederivates der Formel I in Anspruch 1, in Form eines optisch reinen Diastereomeren, eines Diastereomerengemisches, eines diastereomeren Racemates oder eines Gemisches von diastereomeren Racematen, oder eines pharmazeutisch verwendbaren Salzes einer solchen Verbindung zur Herstellung von Mitteln gegen Bluthochdruck und/oder Herzinsuffizienz.

28. Verbindungen der Formeln

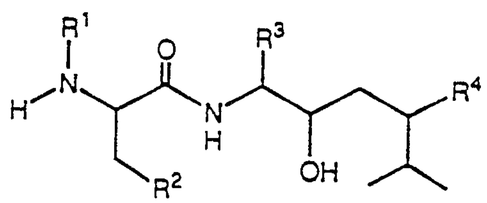

II

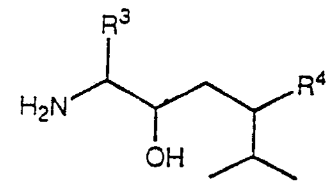

III

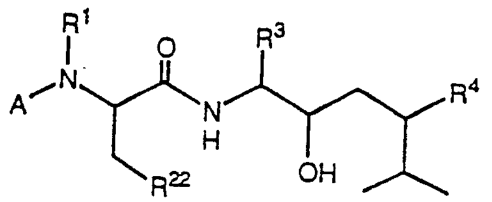

V'

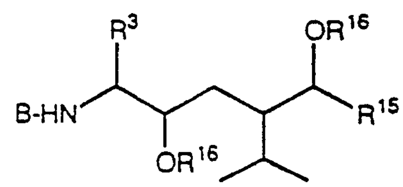

VI

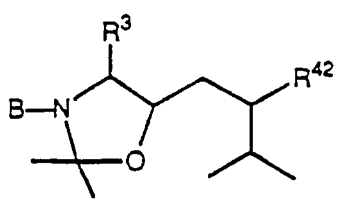

VII

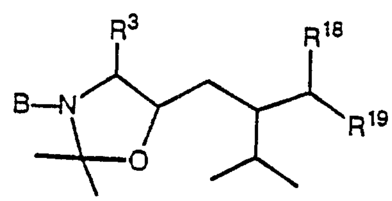

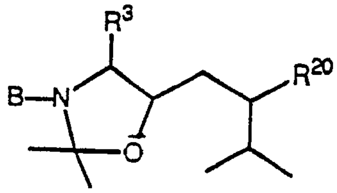

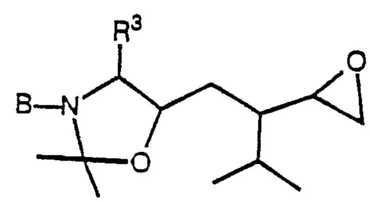

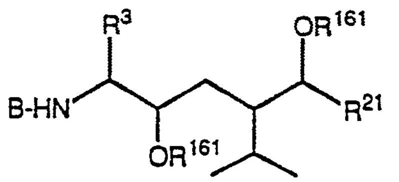

und

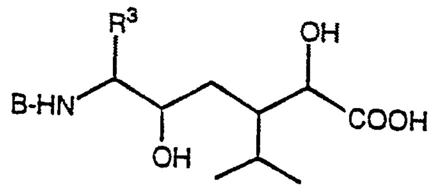

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, A eine der Gruppen

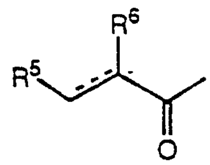

(a)

und -Y-Z (b)

und $R^4$ Alkanoyl, Arylcarbonyl, 2,2-Dialkylvinyl oder eine der Gruppen

$-CH=CH-R^7$

(c)

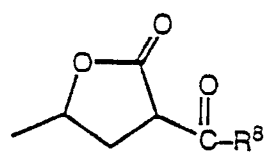

(d)

und

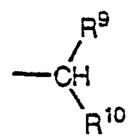

(e)

bedeuten, worin die gestrichelte Linie eine zusätzliche Bindung bedeuten kann, $R^5$ Phenyl, substituiertes Phenyl, Benzyl oder Naphthyl und $R^6$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Aminoalkylcarbonylalkyl, substitu iertes Aminoalkylcarbonylalkyl, Aminoalkylsulfonylalkyl, substituiertes Aminoalkylsulfonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl, Alkylsulfonylalkyl, Arylthioalkyl, Arylsulfinylalkyl, Arylsulfonylalkyl, Arylalkylthioalkyl, Arylalkylsulfinylalkyl oder Arylalkylsulfonylalkyl bedeuten, mit der Massgabe, dass $R^6$ nicht Alkoxycarbonylamino oder Arylalkoxycarbonylamino bedeuten kann, wenn $R^5$ Phenyl, Benzyl oder $\alpha$-Naphthyl bedeutet, Y den N-terminal mit Z verbundenen, bivalenten Rest von gegebenenfalls N- und/oder $\alpha$-methyliertem Phenylglycin, Cyclohexylglycin, Phenylalanin, Cyclohexylalanin, 4-Fluorphenylalanin, 4-Chlorphenylalanin, Tyrosin, O-Methyltyrosin, $\alpha$-Naphthylalanin oder Homophenylalanin, Z Acyl, $R^7$ Alkyl, Aryl, Heteroaryl, Alkylaminocarbonyl, Arylalkylaminocarbonyl oder Heteroarylalkylaminocarbonyl, $R^8$ Alkoxy, $R^9$ Wassersstoff oder Hydroxy und $R^{10}$ Azidomethyl, die Gruppe (c) oder eine der Gruppen

$-CH_2-OR^{11}$

(f)

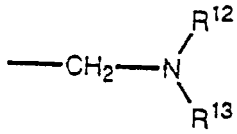

(g)

und $-(CH_2)_n-R^7$

(h)

bedeuten, worin $R^{11}$ Wasserstoff, Alkyl, Arylalkyl, Aryl, Alkanoyl, Arylalkylcarbonyl, Heteroarylalkylcarbonyl oder Alkylcarbamoyl, $R^{12}$ und $R^{13}$ unabhängig voneinander je Wasserstoff, Alkyl, Arylalkyl, Heteroarylalkyl, Aryl, Alkanoyl, Alkoxycarbonyl, Arylalkylcarbonyl, Arylalkoxycarbonyl, Hetero arylalkylcarbonyl, Heterocycloalkylcarbonyl, Alkylcarbamoyl oder die Gruppe

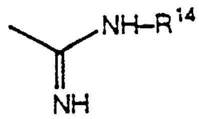

oder $R^{12}$ und $R^{13}$ zusammen mit dem sie verbindenden Stickstoffatom einen 5- oder 6-gliedrigen Heterocyclus oder gegebenenfalls substituiertes Benzimidazolonyl, n 0, 1 oder 2 und $R^{14}$ Wasserstoff oder Arylalkoxycarbonyl bedeuten, $R^{22}$ N-geschütztes Imidazol-2-yl, Imidazol-4-yl oder Pyrazol-3-yl, B eine Aminoschutzgruppe, $R^{15}$ die Gruppe (f) oder Alkylaminocarbonyläthyl, Arylalkylaminocarbonyläthyl oder Heteroarylalkylaminocarbonyläthyl und $R^{16}$ eine O-Schutzgruppe oder $R^{15}$ Alkylaminocarbonyl, Arylalkylaminocarbonyl oder Heteroarylalkylaminocarbonyl und $R^{16}$ Wasserstoff und $R^{42}$ Alkanoyl, Arylcarbonyl, 2,2-Dialkylvinyl oder eine der Gruppen (c), (d) und (e) bedeuten, mit der Massgabe, dass in der Gruppe (e) $R^9$ Wasserstoff bedeutet, wenn $R^{10}$ die Gruppe (f) oder die Gruppe (h) bedeutet, worin n 0 oder 2 und $R^7$ Alkylaminocarbonyl, Arylalkylaminocarbonyl oder Heteroarylalkylaminocarbonyl bedeuten, $R^{18}$ Wasserstoff und $R^{19}$ Hydroxymethyl, Carboxyl, Formyl, 2-Aethoxycarbonylvinyl, 2-Carboxyvinyl, Carboxymethyl oder die Gruppen -OX oder $-CH_2OX$ oder $R^{18}$ Hydroxy und $R^{19}$ Cyan oder Carboxymethyl, $R^{20}$ 2-Aethoxycarbonylvinyl oder 2-Carboxyvinyl, $R^{21}$ Hydroxymethyl, Carboxyl, Formyl oder die Gruppe $-CH=CH-R^{72}$, $R^{161}$ eine O-Schutzgruppe, X p-Tolylsulfonyl, Methylsulfonyl, Trifluormethylsulfonyl oder p-Brombenzolsulfonyl und $R^{72}$ Alkylaminocarbonyl, Arylalkylaminocarbonyl oder Heteroarylalkylaminocarbonyl bedeuten.